# EUROPEAN PATENT SPECIFICATION

(11) **EP 3 057 618 B1**
(45) Date of publication and mention of the grant of the patent: **14.12.2022**
(21) Application number: 14790776.0
(22) Date of filing: 14.10.2014
(51) Int. Cl.: A61K 47/68, A61P 35/00

(54) **GLYCOENGINEERED ANTIBODY, ANTIBODY-CONJUGATE AND METHODS FOR THEIR PREPARATION**
DURCH GLYCO-ENGINEERING HERGESTELLTER ANTIKÖRPER, ANTIKÖRPERKONJUGAT UND VERFAHREN ZU DEREN HERSTELLUNG
ANTICORPS GLYCOSYNTHÉTISÉE, ANTICORPS/CONJUGUÉ ET PROCÉDÉS POUR LEUR PRÉPARATION

(30) Priority: 14.10.2013 EP 13188592; 23.04.2014 EP 14165575
(43) Date of publication of application: 24.08.2016
(73) Proprietor: SynAffix B.V., 5342 CC Oss (NL)
(72) Inventor: VAN DELFT, Floris Louis, NL-6524 KZ Nijmegen (NL); VAN GEEL, Remon, NL-5396 PM Lith-Oijen (NL); WIJDEVEN, Maria Antonia, NL-6663 HR Lent (NL); VERKADE, Jorge Merijn Mathieu, NL-5642 NC Nijmegen (NL); HEESBEEN, Ryan, NL-6532 CM Nijmegen (NL)
(74) Representative: Nederlandsch Octrooibureau
(86) International application number: PCT/NL2014/050716
(87) International publication number: WO 2015/057065

(56) References cited:
- WO-A1-2007/095506
- WO-A1-2016/022027
- WO-A2-03/031464
- BRIAN M. ZEGLIS ET AL: "Enzyme-Mediated Methodology for the Site-Specific Radiolabeling of Antibodies Based on Catalyst-Free Click Chemistry", BIOCONJUGATE CHEMISTRY, vol. 24, no. 6, 19 June 2013 (2013-06-19), pages 1057-1067, XP055101895, ISSN: 1043-1802, DOI: 10.1021/bc400122c cited in the application
- DANIEL AYOUB ET AL: "Correct primary structure assessment and extensive glyco-profiling of cetuximab by a combination of intact, middle-up, middle-down and bottom-up ESI and MALDI mass spectrometry techniques", MABS, vol. 5, no. 5, 1 September 2013 (2013-09-01), pages 699-710, XP055102410, ISSN: 1942-0862, DOI: 10.4161/mabs.25423
- WEI HUANG ET AL: "Chemoenzymatic Glycoengineering of Intact IgG Antibodies for Gain of Functions", JOURNAL OF THE AMERICAN CHEMICAL SOCIETY, vol. 134, no. 29, 25 July 2012 (2012-07-25), pages 12308-12318, XP055035929, ISSN: 0002-7863, DOI: 10.1021/ja3051266 cited in the application

## Description

### Technical field of the invention

The present invention relates to antibodies, modified antibodies and antibody-conjugates, in particular to glycoengineered antibodies, modified antibodies and antibody-conjugates. The invention also relates to a method for preparation of the modified antibodies and antibody-conjugates of the invention. The antibodies may be conjugated to an active substance. The invention therefore also relates to antibody-drug conjugates (ADCs) and a method for the preparation thereof.

### Background of the invention

Antibody-conjugates, *i.e.* antibodies conjugated to a molecule of interest via a linker, are known in the art. There is great interest in antibody-conjugates wherein the molecule of interest is a drug, for example a cytotoxic chemical. Antibody-drug-conjugates are known in the art, and consist of a recombinant antibody covalently bound to a cytotoxic chemical via a synthetic linker (S.C. Alley et al, Curr. Opin. Chem. Biol. 2010, 14, 529-537). The main objective of an antibody-drug-conjugate (ADC), also called immunotoxin, is to combine the high specificity of a monoclonal antibody for a tumor-associated antigen with the pharmacological potency of a "small" cytotoxic drug (typically 300 to 1,000 Da). Examples of ADCs include gemtuzumab ozogamicin (Mylotarg; anti-CD33 mAb conjugated to calicheamycin, Pfizer/Wyeth); brentuximab vedotin (SGN-35, Adcetris, a CD30-targeting ADC consisting of brentuximab, covalently linked to MMAE (monomethylauristatin), Seattle Genetics); trastuzumab-DM1 conjugate (T-DM1, Kadcyla).

One advance in the field includes the emergence of extremely potent toxins, in particular taxanes, calicheamycins, maytansins, pyrrolobenzodiazepines, duocarmycins and auristatins. The low nanomolar to picomolar toxicity of these substances is a principal driver improvement over the earlier applied toxins. Another important technological advance involves the use of optimized linkers that are hydrolysable in the cytoplasm, resistant or susceptible to proteases, or resistant to multi-drug resistance efflux pumps that are associated with highly cytotoxic drugs.

ADCs known from the prior art are commonly prepared by conjugation of the linker-toxin to the side chain of amino acid lysine or cysteine, by acylation or alkylation, respectively.

For lysines, conjugation takes place preferentially at lysine side chains with highest steric accessibility, the lowest pKa, or a combination thereof. Disadvantage of this method is that site-control of conjugation is low.

Better control of site-specificity is obtained by alkylation of cysteines, based on the fact that typically no free cysteines are present in an antibody, thereby offering the option of alkylating only those cysteines that are selectively liberated by a reductive step or specifically engineered into the antibody as free cysteines (as in so-called THIOmabs). Selective cysteine liberation by reduction is typically performed by treatment of whole antibody with a reducing agent (*e.g.* TCEP or DTT), leading to conversion of a disulfide bond into two free thiols (mostly in the antibody's hinge region). The liberated thiols are then alkylated with an electrophilic reagent, typically based on a maleimide attached to a linker-toxin, which generally proceeds fast and with high selectivity. With respect to engineering of an additional (free) cysteine into an antibody, enhanced site-control is attained with respect to the location of the added cysteine(s). Also in this strategy alkylation of free cysteines is effected with maleimide chemistry, but full homogeneity is not attained. One most recent report (N.M. Okeley et al., Bioconj. Chem. 2013, 24, 1650) describes the metabolic incorporation of 6-thiofucose into a monoclonal antibody. However, efficiency of incorporation of 6-thiofucose was found to be only 70%, thus a DAR of 1.3 is attained after maleimide conjugation.

At the same time, a disadvantage of ADCs obtained via alkylation with maleimides is that in general the resulting conjugates are unstable due to the reverse of alkylation, *i.e.* a retro-Michael reaction, thereby leading to release of linker-toxin from the antibody. It has been described that the stability of the cysteine-maleimide conjugate is highly dependent on the position of the cysteine in the monoclonal antibody. For example, a highly solvent-accessible site typically rapidly loses conjugation in plasma owing to maleimide exchange with reactive thiols in albumin, free cysteine or glutathione. In contrast, this undesired exchange reaction is prevented in a partially accessible site with a positively charged environment, while the site with partial solvent-accessibility and neutral charge displayed both properties. Similarly, it was found that the 6-thiofucose maleimide conjugate described above was found to display somewhat enhanced stability with respect to cysteine maleimide conjugates, but an explanation was not provided. In view of the above, conjugation based on cysteine-maleimide alkylation is not an ideal technology for development of ADCs that preferably should not show premature release of toxin. Less popular but also regularly applied for protein conjugation involves halogenated acetamides that may also react with high selectivity with free thiols although chemoselectivity is compromised with respect to maleimide conjugation. A particular advantage of conjugation with halogenated acetamides is the irreversible formation of a thioether, which compares favorably to maleimide conjugates with respect to stability. Other alternatives are also known, for example vinylsulfone conjugation, but less frequently applied. Finally, light-induced thiol-ene reaction has also been shown to be suitable for protein conjugation, see for example Kunz et al. Angew. Chem. Int. Ed. 2007, 46, 5226-5230), also in this case leading to highly stable thioethers.

An alternative strategy to prepare antibody-drug conjugates involves the generation of one or more aldehyde functions on the antibody's glycan structure. All recombinant antibodies, generated in mammalian host systems, contain the conserved N-glycosylation site at asparagine-297, which is modified by a glycan of the complex type. The latter glycan features 0, 1 or 2 terminal galactose units at the non-reducing termini of each N-glycan, which can be applied for the generation of an aldehyde function, either by chemical means (sodium periodate) or by enzymatic means (galactose oxidase). The latter aldehyde function can subsequently be employed for a selective conjugation process, for example by condensation with a functionalized hydroxylamine or hydrazine molecule, thereby generating an oxime-linked or hydrazone-linked antibody conjugate, respectively. However, it is known that oximes and hydrazones, in particular derived from aliphatic aldehydes, show limited stability over time in water or at lower pH. For example, gemtuzumab ozogamicin is an oxime-linked antibody-drug conjugate and is known to suffer from premature deconjugation in vivo.

Antibody-conjugates known in the art generally suffer from several disadvantages. For antibody drug-conjugates, a measure for the loading of the antibody with a toxin is given by the drug-antibody ratio (DAR), which gives the average number of active substance molecules per antibody. However, the DAR does not give any indication regarding the homogeneity of such ADC.

Processes for the preparation of an antibody-conjugate known from the prior art generally result in a product with a DAR between 1.5 and 4, but in fact such a product comprises a mixture of antibody-conjugates with a number of molecules of interest varying from 0 to 8 or higher. In other words, antibody-conjugates known from the prior art generally are formed with a DAR with high standard deviation.

For example, gemtuzumab ozogamicin is a heterogeneous mixture of 50% conjugates (0 to 8 calicheamycin moieties per IgG molecules with an average of 2 or 3, randomly linked to solvent exposed lysine residues of the antibody) and 50% unconjugated antibody (Bross et al., Clin. Cancer Res. 2001, 7, 1490; Labrijn et al., Nat. Biotechnol. 2009 27, 767). But also for brentuximab vedotin, T-DM1, and other ADCs in the clinic, it is still uncontrollable exactly how many drugs are attaching to any given antibody (drug-antibody ratio, DAR) and the ADC is obtained as a statistical distribution of conjugates. Whether the optimal number of drugs per antibody is for example two, four or more, attaching them in a predictable number and in predictable locations through site-specific conjugation with a narrow standard deviation is still problematic.

A versatile strategy that may be generally applicable to all monoclonal antibodies involves the site-specific conjugation to the Fc-attached glycan, which is naturally present in all antibodies expressed in mammalian (or yeast) cell cultures. Several strategies based on this concept are known in the art, such as via oxidation of the terminal galactose or via enzymatic transfer of (unnatural) sialic acid to the same galactose moiety. However, for ADC purpose such a strategy is suboptimal because glycans are always formed as a complex mixture of isoforms, which may contain different levels of galactosylation (G0, G1, G2) and therefore would afford ADCs with poor control of drug-antibody ratio (DAR, see below).

Qasba et al. disclose in WO 2004/063344 and in J. Biol. Chem. 2002, 277, 20833 that mutant galactosyltransferases GalT(Y289L), GalT(Y289I) and GalT(Y289N) can enzymatically attach GalNAc to a non-reducing GlcNAc sugar. For example, GlcNAc is the terminal component of some of the complex N-glycans such as those on monoclonal antibodies (*e.g.* Rituxan, Remicade, Herceptin).

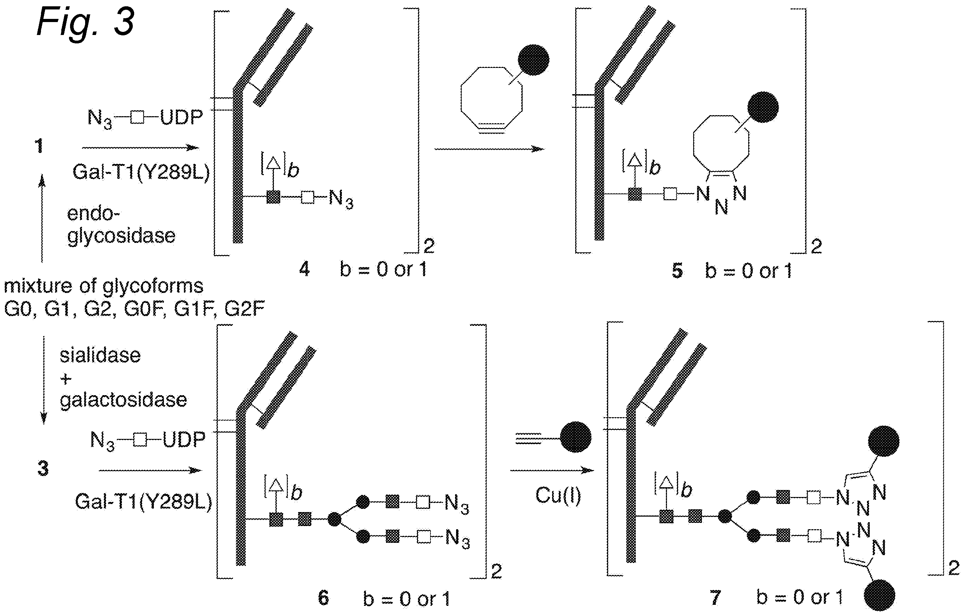

Qasba et al. disclose in Bioconjugate Chem. 2009, 20, 1228 that the process disclosed in WO 2004/063344 also proceeds for non-natural GalNAc-UDP variants substituted on the N-acetyl group. β-Galactosidase treated monoclonal antibodies having a G0 glycoform are fully galactosylated to the G2 glycoform after transfer of a galactose moiety comprising a C2-substituted azidoacetamido moiety (GalNAz) to the terminal GlcNAc residues of the glycan, leading to tetraazido-substituted antibodies, *i.e.* two GalNAz moieties per heavy chain (see Figure 3, conversion of 3 to 6). The conjugation of said tetraazido-substituted antibodies to a molecule of interest, for example by Staudinger ligation or click reaction with an alkyne (see Figure 3, conversion of 6 to 7) is of potential interest to prepare antibody-drug conjugates with a DAR of 4, for example, but was not disclosed. The transfer of a galactose moiety comprising a C2-substituted keto group (C2-keto-Gal) to the terminal GlcNAc residues of a G0 glycoform glycan, as well as the linking of C2-keto-Gal to aminooxy biotin, is also disclosed. However, as mentioned above, the resulting oxime conjugates may display limited stability due to aqueous hydrolysis.

WO 2007/095506 and WO 2008/029281 (Invitrogen Corporation) disclose that the combination of GalT(Y289L) mutant with C2-substituted azidoacetamido-galactose UDP-derivative (UDP-GalNAz) leads to the incorporation of GalNAz at a terminal non-reducing GlcNAc of a glycan. Subsequent conjugation by Staudinger ligation or with copper-catalyzed click chemistry then provides the respective antibody conjugates wherein a fluorescent alkyne probe is conjugated to an antibody. WO 2007/095506 and WO 2008/029281 further disclose that trimming of the glycan can take place with endo H, thereby hydrolyzing a GlcNAc-GlcNAc glycosidic bond and liberating a GlcNAc for enzymatic introduction of GalNAz.

A disadvantage of the method disclosed in WO 2004/063344 and Bioconjugate Chem. 2009, 20, 1228 is that conjugation of the tetraazido-substituted antibodies to a molecule of interest would lead to an antibody-conjugate with typically two molecules of interest per glycan (provided that said conjugation would proceed with complete conversion). In some cases, for example when the molecule of interest is a lipophilic toxin, the presence of too many molecules of interest per antibody is undesired since this may lead to aggregate formation (BioProcess International 2006, 4, 42-43), in particular when the lipophilic moieties are in proximity. More advantageously, the lipophilic moieties would be positioned more remote from each other, however a robust and controlled method for such constellation is currently lacking.

In WO 2007/133855 (University of Maryland Biotechnology Institute), a chemoenzymatic method for the preparation of a homogeneous glycoprotein or glycopeptide is disclosed, involving a two-stage strategy entailing first trimming of the near-complete glycan tree (under the action of endo A, endo H or endo S) leaving only the core N-acetylglucosamine (GlcNAc) moiety (the so-called GlcNAc-protein), followed by a reglycosylation event wherein, in the presence of a catalyst comprising endoglycosidase (ENGase), an oligosaccharide moiety is transferred to the GlcNAc-protein to yield a homogeneous glycoprotein or glycopeptide. A strategy for azide-functionalized glycoproteins is disclosed, wherein a GlcNAc-protein is reacted in the presence of ENGase with a tetrasaccharide oxazoline containing two 6-azidomannose moieties, thereby introducing two azides simultaneously in the glycan. The tetraazide-functionalized glycoprotein may then be utilized to attach four equivalents of a bioactive moiety, *e.g.* a toxin for the preparation of an ADC, by a catalytical "click chemistry" cycloaddition reaction, in the presence of a catalyst (e.g. a Cu(I) catalyst) with a terminal alkyne bearing a functional moiety X of interest or with a cyclic alkyne by means of strain-promoted cycloadditon. No actual examples of said click chemistry are disclosed.

In J. Am. Chem. Soc. 2012, 134, 8030, Davis *et al.* disclose the transfer of oligosaccharide oxazolines on a core-fucosylated as well as nonfucosylated core-GlcNAc-Fc domain of intact antibodies, in the presence of glycosynthase EndoS.

In J. Am. Chem. Soc. 2012, 134, 12308, Wang *et al.* disclose the transfer of a tetrasaccharide oxazoline containing two 6-azidomannose moieties on core-fucosylated as well as nonfucosylated core-GlcNAc-Fc domain of intact antibodies (Rituximab) in the presence of glycosynthase mutants EndoS-D233A and EndoS-D233Q.

However, a disadvantage of the glycosynthase strategies disclosed in WO 2007/133855, J. Am. Chem. Soc. 2012, 134, 8030 and J. Am. Chem. Soc. 2012, 134, 12308 is the lengthy and complex synthesis of the required azido-containing oligosaccharide oxazolines.

In any of the strategies that enable the introduction of multiple azides into an antibody, a subsequent conjugation with a molecule of interest may be effectuated via Staudinger ligation or an azide-based click reaction. For example, Zeglis et al. (Bioconj. Chem. 2013, 24, 1057) disclose the radiolabeling of an antibody by means of sialidase/galactosidase trimming, followed by Gal-T mediated GalNAz introduction and copper-free click conjugation. However, as it appears quantitative labeling of the antibody is not achieved (efficiency of labeling is ±2.8, not 4), potentially due to the proximal nature of the azide groups that hamper dual conjugation.

One limitation of the current technologies for the preparation of antibody conjugates via the N-glycan is the inherent dependence of such an approach to (a) naturally existing N-glycosylation site(s). All monoclonal antibodies of IgG-type have at least one N-glycosylation site at (or around) asparagine-297 of the heavy chain. The glycan at N297 is essential to induce effector function (antibody-dependent cellular cytotoxicity, ADCC) by binding to Fc-gamma receptors. The N297 glycan, however is not essential for retaining a long circulation time in blood, which is regulated by the C_{H}2-C_{H}3 domain interface of the antibody, with the FcRn receptor. Apart from the N297 glycosylation site, approximately 20% of monoclonal antibodies harbour a second glycosylation site, typically in the Fab domain. The second glycosylation site, however, is not known to be essential for antibody activity of any kind and may as such be engineered out of the antibody if desirable (as for example applied in the development of trastuzumab).

Wright et al. (EMBO J. 1991, 10, 2717) describe a *de novo* engineering of a glycosylation site at specific position 54 or 60 in the V_{H} of two different antibodies TST2 and TSU7, respectively, and determine the influence of such sites on glycosylation processing, on antigen affinity and binding of the (Fab')₂-fragment derived from the antibody.

In US6254868 and EP97916787, it is described how 10 new glycosylation sites are designed and engineered into a humanized anti-CD22 monoclonal antibody, hLL2. Two CH₁ domain glycosylation sites, HCN1 and HCN5, were identified that were positioned favorably for glycosylation. The new glycosylation sites were applied for site-specific conjugation of chelates and drugs to the (Fab')₂-fragment derived from the antibody. Conjugation was effected by means of periodate oxidation, then reductive amination, with negligible influence on immunoreactivity. Both the CH₁-appended CHOs conjugated equally efficiently with small chelates, it was concluded that the HCN5-CHOs, due to the structural and positional superiority, appears to be a better conjugation site for large drug complexes then conjugation in the variable domain of the antibody.

Another disadvantage of current technologies for the preparation of antibody conjugates is that differential labeling of one antibody with e.g. two different labels is not achievable.

### Summary of the invention

The present invention relates to a process for the preparation of an antibody-conjugate, comprising the steps of:
(1) providing an IgG antibody comprising at least two N-linked glycosylation sites on the combination of a single heavy chain and single light chain; and
(2) trimming an oligosaccharide that is attached to a glycosylation site, by the action of an endoglycosidase, in order to obtain a proximal N-linked GlcNAc-residue at said glycosylation site; and
(3) optionally repeating step (2) in order to trim an oligosaccharide that is attached to a different glycosylation site; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(5) optionally:
   (5a) repeating step (2), in order to trim an oligosaccharide that is attached to a different glycosylation site; and
   (5b) repeating step (4); and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
(7) optionally:
   (7a) repeating step (2) in order to trim an oligosaccharide that is attached to a different glycosylation site; and
   (7b) repeating step (4); and
   (7c) repeating step (6); and
wherein the proximal N-linked GlcNAc-residue in steps (2), (4) and (6) is optionally fucosylated; wherein the process comprises one of steps (3), (5) and (7).

The invention also relates to an antibody-conjugate obtainable by the process according to the invention, wherein an antibody conjugate is defined as an antibody that is conjugated to a molecule of interest D via a linker L.

As was described above, the processes known from the prior art for conjugation of a linker-toxin to antibodies still need to be improved, in terms of control of both site-specificity and stoichiometry. Despite the ability of ADCs to home in on their targets, the amount of drug estimated to get inside tumor cells is typically <2% of an administered dose. This problem is amplified by the unpredictable conjugation results of ADCs known in the art. It is important to avoid underconjugated antibodies, which decrease the potency, as well as highly conjugated species, which may have markedly decreased circulating half-lives, impaired binding to the target protein, and increased toxicity.

For antibody-drug conjugates, a measure for the loading of molecules of interest (e.g. drugs, active substances) onto the antibody is the so-called Drug to Antibody Ratio (DAR), which gives the average number of active substance molecules per antibody, calculated from a statistical distribution. The theoretical maximum value of DAR for a certain type of ADC is equal to the number of anchoring sites. As was described above, processes for the preparation of ADCs known from the prior art generally result in a product comprising a mixture of antibody-conjugates with a varying number of molecules of interest present in each antibody-conjugate, and in a DAR with a high standard deviation.

One of the advantages of the modified antibodies, the antibody-conjugates and the process for their preparation according to the invention is that these antibodies and antibody-conjugates are homogeneous, both in site-specificity and stoichiometry. The modified antibodies and antibody-conjugates according to the invention are obtained with a DAR very near to the theoretical value, and with a very low standard deviation. This also means that the antibody-conjugates according to the invention result in a more consistent product for preclinical testing.

Another advantage of the processes and antibodies according to the invention involves the reduction of waste in manufacturing, thereby enhancing companies' cost-of-goods.

Furthermore, when an azide-modified antibody according to the invention is coupled to a linker-conjugate comprising an alkynyl group, or when an alkyne-modified antibody according to the invention is coupled to a linker-conjugate comprising an azide moiety, via a cycloaddition reaction, the resulting triazoles are not susceptible to hydrolysis or other degradation pathways. When a ketone-modified antibody according to the invention is coupled to a linker-conjugate comprising a hydroxylamine or a hydrazine, the resulting oximes or hydrazones are also relatively inert at neutral conditions. When a thiol-modified antibody according to the invention is coupled to a linker-conjugate comprising a maleimide, the process is well-known in the art, highly robust and validated. Many maleimide-functionalized toxins have been described, because currently the preferred methodology for antibody-drug conjugation involves the combination of a cysteine mutant of a mAb (THIOmAb) and a maleimide derivative of a toxin. It is well known that such thiol-maleimide conjugates can be prepared with a highly beneficial stoichiometry of reagents (small excess of maleimide component). When a thiol-modified antibody according to the invention is coupled to a linker-conjugate comprising a halogenated acetamide derivative of a toxin, the desired antibody conjugate is an irreversibly formed (highly stable) thio-ether conjugate, although in some cases the efficiency of the process may be somewhat compromized with respect to maleimide conjugation and slightly more undesired alternative conjugation may take place (*e.g.* on lysine side chains). When a halogen-modified antibody according to the invention is coupled to a linker-conjugate comprising a derivative of a toxin containing a nucleophilic group (e.g. a thiolgroup, an alcohol group, an amine group), the resulting conjugate is a thio-ether, a regular ether or an amino-ether, all of which are formed irreversibly. In contrast to the use of halogenated acetemides for conjugation to proteins containing free thiols (as in THIOmAbs or in a thiofucose-containing mAb), the enzymatic incorporation of a halogenated sugar substrate is not compromised by competitive aspecific reaction with nucleophilic side chains of other amino acids (*e.g.* lysine). The lack of aspecific reactions also pertains to the subsequent conjugation step where in this case excess of a nucleophlic derivative of a functional group is applied to the halogenated mAb.

Additional advantages are thus the stability of antibody-conjugates according to the invention, as well as the straightforward and generally applicable process for the introduction of an azido group, a keto group, an alkynyl group, a thiol group, a halogen, a sulfonyloxygroup, a halogenated acetamido group, a mercaptoacetamido group and a sulfonylated hydroxyacetamido group into an antibody.

The use of monoclonal antibodies that naturally contain a second glycosylation site, or the engineering of one (or more) *de novo* glycosylation sites into a monoclonal antibody, has a number of advantages. Firstly, the presence of two glycosylation sites in an antibody, natural or engineered, allows the straightforward conversion into an antibody conjugate with increased ratio of functional label:antibody which may increase *e.g.* level of detection for imaging application or delivery of drug substances to specific antigen-presenting cells. Secondly, N-glycosylation may be specifically engineered at any site of the Fab fragment of an antibody (V_{H}, V_{L}, C_{H}1 or C_{V}1 domain), based on the consensus sequence Asn-X-Ser/Thr (X = any amino acid except Pro) to enable the use of smaller mAb fragments for directed binding. In such event, after expression of the antibody in a suitable host organism, the Fc fragment may be selectively separated from the Fab fragment with *e.g.* pepsin or SpeB, followed by conjugation to the Fab fragment through the novel N-glycan. One important advantage of *de novo* engineering of a new glycosylation site is the flexibility to design the site remote from the antibody binding region to avoid negative interference on antigen affinity. Another advantage involves the potential to include multiple glycosylation sites, even with a single antibody domain if desired, to enhance the loading efficiency of the antibody (or a fragment thereof). For example, it has been well established that antibody-drug conjugates with increased drug-antibody ratio show enhanced cytotoxicity with respect to ADCs with lower DAR. Similarly, in the field of diagnostics or molecular imaging, detection of binding of antibodies labelled with *e.g.* a fluorophore or a radionuclide, will be enhanced in case a higher labeling of the antibody is secured (without compromising binding affinity). Finally, there is a correlation between location of drug and the *in vivo* efficacy of ADC. Hence, designing, expressing, processing into ADCs and activity determination of monoclonal antibodies with different glycosylation profiles is a versatile and powerful tool to modulate the properties of an antibody conjugate. Also, it is known to someone skilled in the art that antibody-drug conjugates with a higher DAR (4 and up) are particularly useful for antigens that have relatively low copy numbers. Engineering one or more additional glycosylation sites and converting the mAb into an ADC with DAR = 4 (or 6 or more in case more glycosylation sites are engineered in) will be particularly advantageous in such cases.

Another advantage of the invention of having an ADC with a DAR = 4 (or higher) with the hydrophobic toxins at remote sites on the mAb, is that the tendency to aggregate for such ADCs may be smaller than in case payloads are in close proximity, which may lead to enhanced lipophilic interaction of the toxins. Hence the stability of an ADC with payloads at remote sites will be higher.

### Description of the Figures

Figure 1 shows examples of possible glycosylation profiles of monoclonal antibodies expressed in a mammalian host organism.
Figure 2 shows the different glycoforms of a monoclonal antibody that can be obtained by regular expression followed by trimming with an endoglycosidase (**1**). Glycoform **2** can be obtained by expression of a mAb in a mammalian system in the presence of swainsonine or by expression in an engineered host organism, *e.g.* Pichia. Finally, glycoform **3** can be obtained by trimming of the regular mixture of glycoforms (G0, G1, G2, G0F, GIF and G2F) upon combined action of sialidase and galactosidase.
Figure 3 shows the enzymatic conversion of the mixture of glycoforms of a mAb into GlcNAc-terminated mAb **1** or **3** upon treatment with an endoglycosidase or a mixture of sialidase and galactosidase, respectively. Upon treatment of UDP-GalNAz in the presence of Gal-T1(Y289L), one or two GalNAz moieties per glycosylation site are introduced, respectively. The azide moieties in **4** and **6** serve as attachment point for functional group introduction by *e.g.* strain-promoted cycloaddition (**4→5**) or copper-catalyzed click reaction (**6→7**).
Figure 4 shows the structures of azido-modified galactose derivatives (**9-11**) for transfer onto a GlcNAc-terminated sugar under the action of a galactosyl transferase (or a mutant thereof).
Figure 5 shows the structures of other galactose derivatives (**12-27**) for transfer onto a GlcNAc-terminated sugar under the action of a galactosyl transferase (or a mutant thereof).
Figure 6A shows the possible structures of IgGs with two glycosylation sites of which one the native glycosylation site at N297.
Figure 6B shows the possible structures of IgGs with two glycosylation sites but not the native glycosylation site at N297.
Figure 7 shows the enzymatic cleavage sites for endoglycosidases of an IgG with two glycosylation sites, one of which is the native glycosylation site at N297.
Figure 8 shows the chemoenzymatic conversion of an IgG with two glycosylation sites (one at N297 and one at another site) into an IgG with two functional groups D upon trimming of both glycans (**28 → 29**), then galactosyl transfer of a modified galactose Su(A) (**29 → 30**), then conjugation with excess B-D, leading to **31.**
Figure 9 shows two step-wise approaches for the same overall transformation of **28** into **31.** Both routes commence by selective trimming of the native glycosylation site with endo S, followed by introduction of modified sugar Su(A) (**28 → 32**). Next, one route involves endo F trimming, followed by introduction of the second Su(A), then global conjugation as in Figure 8 ((**29 → 30**). The second route comprises a single conjugation with B-D (**32 → 33**), prior to endo F trimming and Su(A) introduction (**33 → 34**) and again conjugation with B-D to give the same product **31.**
Figure 10 shows two options for the preparation of an IgG with two functionalities of different nature (D and D²), optionally from **34** based on the same conjugation chemistry but with differently functionalized B-D². Alternatively, **33** can be modified with a different Su(A²) after endo F treatment (**33 → 36**), and then conjugated to the appropriate complementary B²-D² (**36 → 37**). A final option (not depicted) to make the same **37** involves consecutive treatment of **32** with endo F, then introduction of Su(A²), then simultaneous conjugation to B-D and B²-D².
Figure 11 shows the enzymatic cleavage sites for endo F of an IgG with two glycosylation sites (**38**), neither of which is the native glycosylation site at N297.
Figure 12 shows the enzymatic cleavage sites for endoglycosidases of an IgG with three glycosylation sites (**39**), one of which is the native glycosylation site at N297.
Figure 13 shows the reaction scheme for the synthesis of BCN-Val-Cit-PABA-MMAF conjugate (**42**).

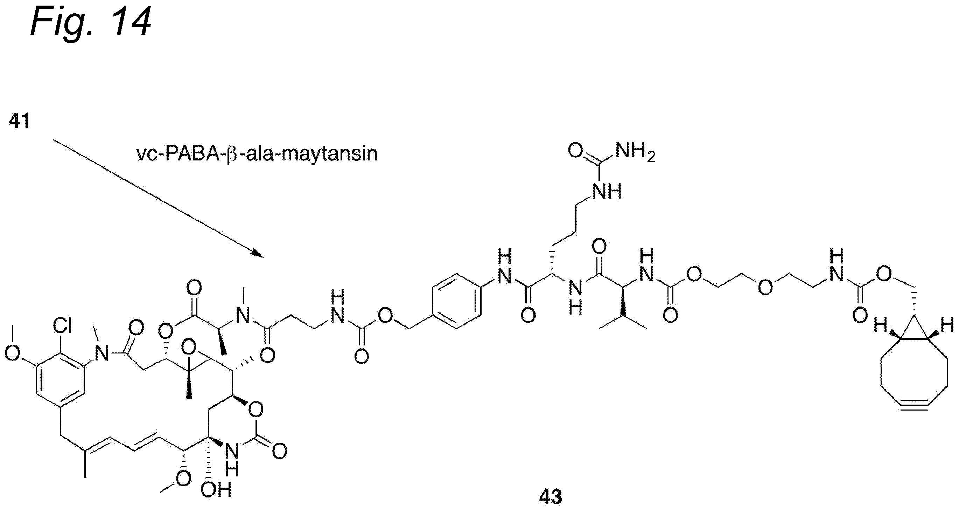

Figure 14 shows the reaction scheme for the synthesis of BCN-Val-Cit-PABA-β-Ala-maytansin conjugate (**43**).
Figure 15 shows the schematic process for the conversion of a trastuzumab mutant (L196N, **44**) with two glycosylation sites into an ADC with DAR = 4 upon consecutive endoglycosidase cleavage, GalNAz-transfer and four-fold copper-free click conjugation with **42,** respectively, leading to an ADC **45** with MMAF at N297 and MMAF at N196.
Figure 16 shows the schematic process for the conversion of a trastuzumab mutant (L196N, **46**) with two glycosylation sites into an ADC with DAR = 4 with two different toxins (**48**) upon consecutive endoglycosidase cleavage, GalNAz-transfer and copper-free click conjugation with **42** and **43,** respectively, leading to an ADC with maytansin at N297 and MMAF at N196.
Figures 17-22 show the MS analysis of intermediates and final products for the conversion of trastuzumab(L196N) into an ADC with toxins maytansin and MMAF.

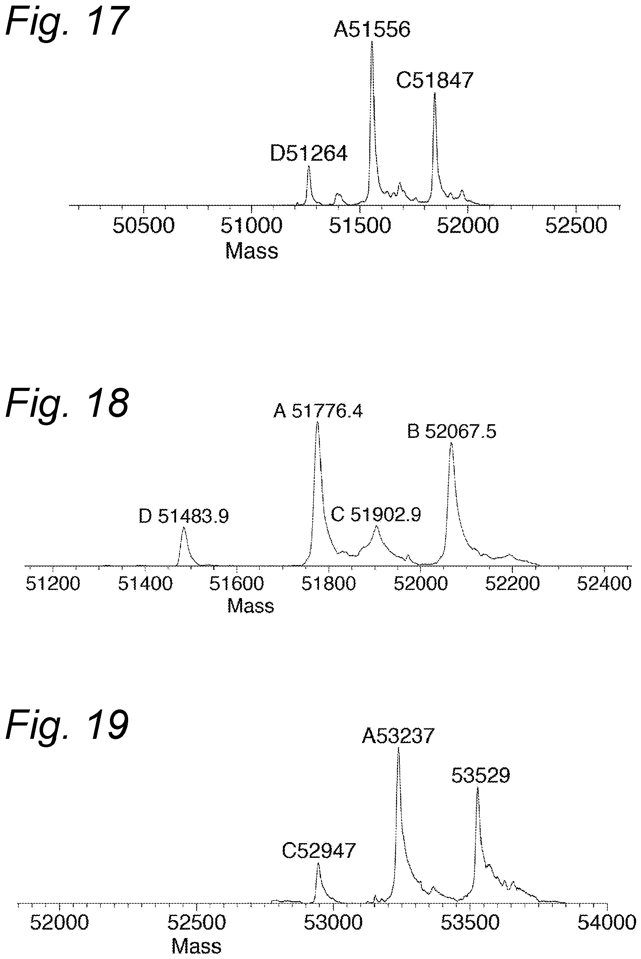

Figure 17 shows the MS spectrum of endo S-treated **46.**
Figure 18 shows the MS spectrum of endo S-treated **46,** followed by UDP-GalNAz in the presence of Gal-T1(Y289L).
Figure 19 shows the MS spectrum of **47.**

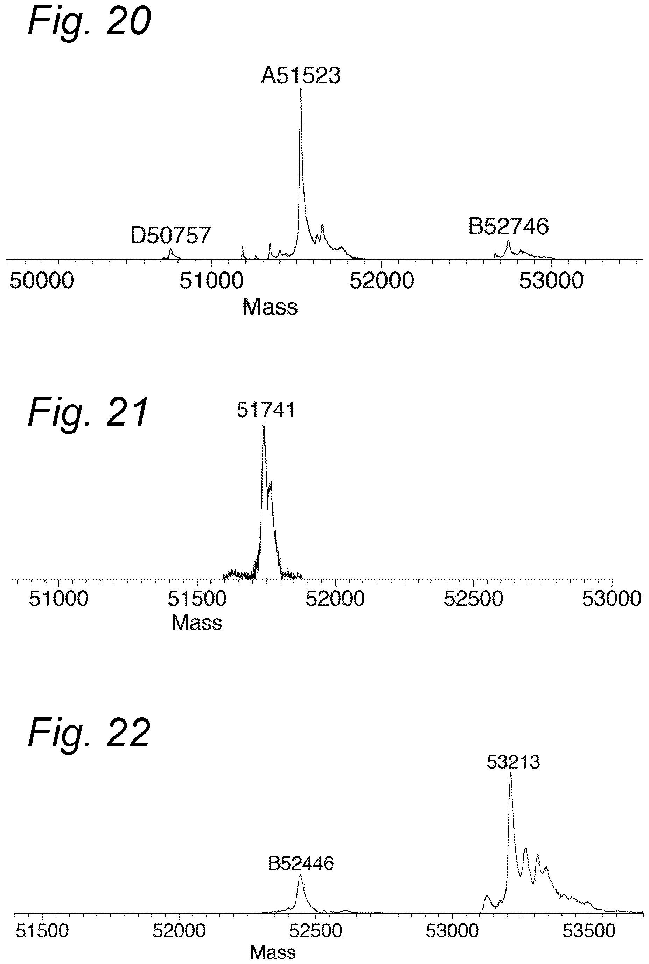

Figure 20 shows the MS spectrum of endo F3-treated **47.**
Figure 21 shows the MS spectrum of endo F3-treated **47,** followed by UDP-GalNAz in the presence of Gal-T1(Y289L).
Figure 22 shows the MS spectrum of **48.**

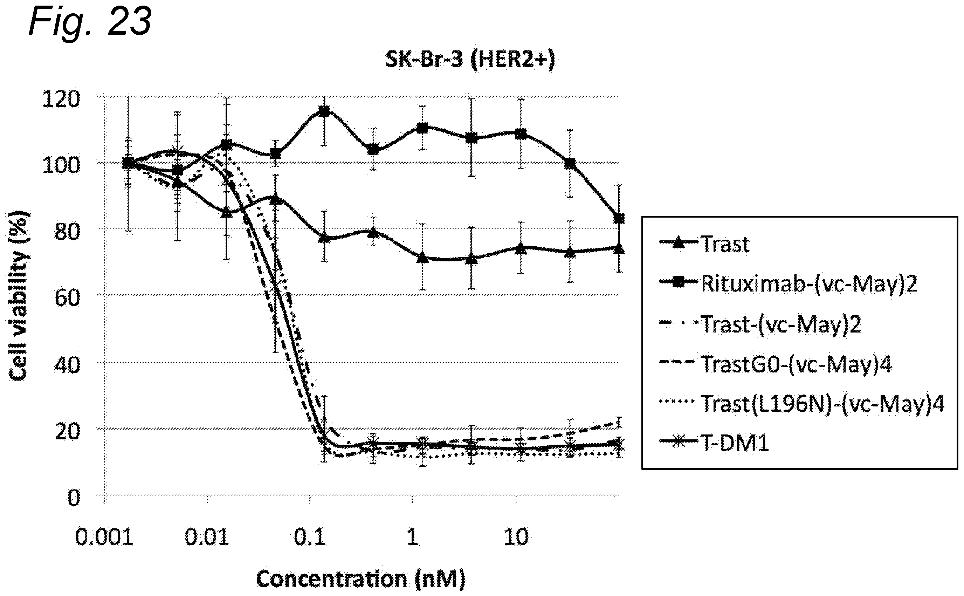

Figure 23 shows the in vitro cytotoxicity of a range of ADCs against SK-Br-3 cell line.

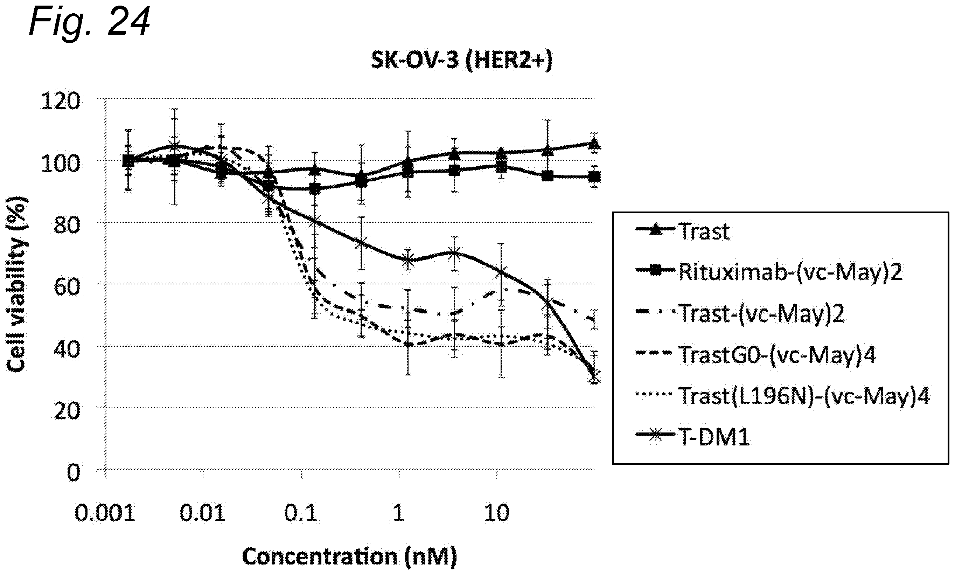

Figure 24 shows the in vitro cytotoxicity of a range of ADCs against SK-OV-3 cell line.

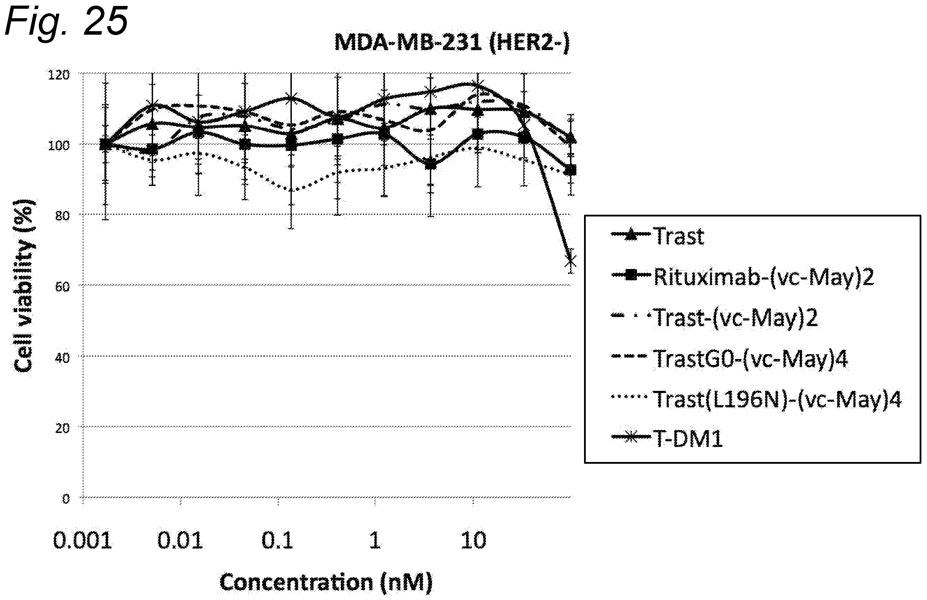

Figure 25 shows the in vitro cytotoxicity of a range of ADCs against MDA-MB-231 cell line (negative control).

### Detailed description of the invention

### Definitions

The verb "to comprise" as is used in this description and in the claims and its conjugations is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer.

Unsubstituted alkyl groups have the general formula CₙH₂ₙ₊₁ and may be linear or branched. Unsubstituted alkyl groups may also contain a cyclic moiety, and thus have the concomitant general formula CₙH₂ₙ₋₁. Optionally, the alkyl groups are substituted by one or more substituents further specified in this document. Examples of alkyl groups include methyl, ethyl, propyl, 2-propyl, t-butyl, 1-hexyl, 1-dodecyl, etc.

An aryl group comprises six to twelve carbon atoms and may include monocyclic and bicyclic structures. Optionally, the aryl group may be substituted by one or more substituents further specified in this document. Examples of aryl groups are phenyl and naphthyl.

Arylalkyl groups and alkylaryl groups comprise at least seven carbon atoms and may include monocyclic and bicyclic structures. Optionally, the arylalkyl groups and alkylaryl may be substituted by one or more substituents further specified in this document. An arylalkyl group is for example benzyl. An alkylaryl group is for example 4-*t*-butylphenyl.

Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and a alkylheteroaryl group, and (hetero)arylalkyl is meant to include an arylalkyl group and a heteroarylalkyl group. A C₂ - C₂₄ (hetero)aryl group is thus to be interpreted as including a C₂ - C₂₄ heteroaryl group and a C₆ - C₂₄ aryl group. Similarly, a C₃ - C₂₄ alkyl(hetero)aryl group is meant to include a C₇ - C₂₄ alkylaryl group and a C₃ - C₂₄ alkylheteroaryl group, and a C₃ - C₂₄ (hetero)arylalkyl is meant to include a C₇ - C₂₄ arylalkyl group and a C₃ - C₂₄ heteroarylalkyl group.

Unless stated otherwise, alkyl groups, alkenyl groups, alkenes, alkynes, (hetero)aryl groups, (hetero)arylalkyl groups and alkyl(hetero)aryl groups may be substituted with one or more substituents selected from the group consisting of C1- C₁₂ alkyl groups, C₂ - C₁₂ alkenyl groups, C₂ - C₁₂ alkynyl groups, C₃ - C₁₂ cycloalkyl groups, C₅ - C₁₂ cycloalkenyl groups, C₈ - C₁₂ cycloalkynyl groups, C₁- C₁₂ alkoxy groups, C₂ - C₁₂ alkenyloxy groups, C₂ - C₁₂ alkynyloxy groups, C₃ - C₁₂ cycloalkyloxy groups, halogens, amino groups, oxo and silyl groups, wherein the silyl groups can be represented by the formula (R¹⁰)₃Si-, wherein R¹⁰ is independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups and C₃ - C₁₂ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S.

An alkynyl group comprises a carbon-carbon triple bond. An unsubstituted alkynyl group comprising one triple bond has the general formula CₙH₂ₙ₋₃. A terminal alkynyl is an alkynyl group wherein the triple bond is located at a terminal position of a carbon chain. Optionally, the alkynyl group is substituted by one or more substituents further specified in this document, and/or interrupted by heteroatoms selected from the group of oxygen, nitrogen and sulphur. Examples of alkynyl groups include ethynyl, propynyl, butynyl, octynyl, etc.

A cycloalkynyl group is a cyclic alkynyl group. An unsubstituted cycloalkynyl group comprising one triple bond has the general formula CₙH₂ₙ₋₅. Optionally, a cycloalkynyl group is substituted by one or more substituents further specified in this document. An example of a cycloalkynyl group is cyclooctynyl.

A heterocycloalkynyl group is a cycloalkynyl group interrupted by heteroatoms selected from the group of oxygen, nitrogen and sulphur. Optionally, a heterocycloalkynyl group is substituted by one or more substituents further specified in this document. An example of a heterocycloalkynyl group is azacyclooctynyl.

A (hetero)aryl group comprises an aryl group and a heteroaryl group. An alkyl(hetero)aryl group comprises an alkylaryl group and an alkylheteroaryl group. A (hetero)arylalkyl group comprises a arylalkyl group and a heteroarylalkyl groups. A (hetero)alkynyl group comprises an alkynyl group and a heteroalkynyl group. A (hetero)cycloalkynyl group comprises an cycloalkynyl group and a heterocycloalkynyl group.

A (hetero)cycloalkyne compound is herein defined as a compound comprising a (hetero)cycloalkynyl group.

Several of the compounds disclosed in this description and in the claims may be described as fused (hetero)cycloalkyne compounds, *i.e.* (hetero)cycloalkyne compounds wherein a second ring structure is fused, i.e. annelated, to the (hetero)cycloalkynyl group. For example in a fused (hetero)cyclooctyne compound, a cycloalkyl (e.g. a cyclopropyl) or an arene (e.g. benzene) may be annelated to the (hetero)cyclooctynyl group. The triple bond of the (hetero)cyclooctynyl group in a fused (hetero)cyclooctyne compound may be located on either one of the three possible locations, *i.e.* on the 2, 3 or 4 position of the cyclooctyne moiety (numbering according to "IUPAC Nomenclature of Organic Chemistry", Rule A31.2). The description of any fused (hetero)cyclooctyne compound in this description and in the claims is meant to include all three individual regioisomers of the cyclooctyne moiety.

When an alkyl group, a (hetero)aryl group, alkyl(hetero)aryl group, a (hetero)arylalkyl group, a (hetero)cycloalkynyl group is optionally substituted, said groups are independently optionally substituted with one or more substituents independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups, C₃ - C₁₂ cycloalkyloxy groups, halogens, amino groups, oxo groups and silyl groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S, wherein the silyl groups are represented by the formula (R⁶)₃Si-, wherein R⁶ is independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups and C₃ - C₁₂ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S.

The general term "sugar" is herein used to indicate a monosaccharide, for example glucose (Glc), galactose (Gal), mannose (Man) and fucose (Fuc). The term "sugar derivative" is herein used to indicate a derivative of a monosaccharide sugar, i.e. a monosaccharide sugar comprising substituents and/or functional groups. Examples of a sugar derivative include amino sugars and sugar acids, e.g. glucosamine (GlcNH₂), galactosamine (GalNH₂) N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (IdoA). Examples of a sugar derivative also include compounds herein denoted Su(A)ₓ, wherein Su is a sugar or a sugar derivative, and wherein Su comprises x functional groups A.

The term "nucleotide" herein refers to a molecule that is composed of a nucleobase, a five-carbon sugar (either ribose or deoxyribose) and one, two or three phosphate groups. Without the phosphate group, the nucleobase and sugar compose a nucleoside. A nucleotide can thus also be called a nucleoside monophosphate, a nucleoside diphosphate or a nucleoside triphosphate. The nucleobase may be adenine, guanine, cytosine, uracil or thymine. Examples of a nucleotide include uridine diphosphate (UDP), guanosine diphosphate (GDP), thymidine diphosphate (TDP), cytidine diphosphate (CDP) and cytidine monophosphate (CMP).

The term "protein" is herein used in its normal scientific meaning. Herein, polypeptides comprising about 10 or more amino acids are considered proteins. A protein may comprise natural, but also unnatural amino acids.

The term "glycoprotein" herein refers to a protein comprising one or more monosaccharide or oligosaccharide chains ("glycans") covalently bonded to the protein. A glycan may be attached to a hydroxyl group on the protein (O-linked-glycan), e.g. to the hydroxyl group of serine, threonine, tyrosine, hydroxylysine or hydroxyproline, or to an amide function on the protein (*N-*glycoprotein), *e.g.* asparagine or arginine, or to a carbon on the protein (*C*-glycoprotein), *e.g.* tryptophan. A glycoprotein may comprise more than one glycan, may comprise a combination of one or more monosaccharide and one or more oligosaccharide glycans, and may comprise a combination of N-linked, O-linked and C-linked glycans. It is estimated that more than 50% of all proteins have some form of glycosylation and therefore qualify as glycoprotein. Examples of glycoproteins include PSMA (prostate-specific membrane antigen), CAL (candida antartica lipase), gp41, gp120, EPO (erythropoietin), antifreeze protein and antibodies.

The term "glycan" herein refers to a monosaccharide or oligosaccharide chain that is linked to a protein. The term glycan thus refers to the carbohydrate-part of a glycoprotein. The glycan is attached to a protein via the C-1 carbon of one sugar, which may be without further substitution (monosaccharide) or may be further substituted at one or more of its hydroxyl groups (oligosaccharide). A naturally occurring glycan typically comprises 1 to about 10 saccharide moieties. However, when a longer saccharide chain is linked to a protein, said saccharide chain is herein also considered a glycan.

A glycan of a glycoprotein may be a monosaccharide. Typically, a monosaccharide glycan of a glycoprotein consists of a single N-acetylglucosamine (GlcNAc), glucose (Glc), mannose (Man) or fucose (Fuc) covalently attached to the protein.

A glycan may also be an oligosaccharide. An oligosaccharide chain of a glycoprotein may be linear or branched. In an oligosaccharide, the sugar that is directly attached to the protein is called the core sugar. In an oligosaccharide, a sugar that is not directly attached to the protein and is attached to at least two other sugars is called an internal sugar. In an oligosaccharide, a sugar that is not directly attached to the protein but to a single other sugar, *i.e.* carrying no further sugar substituents at one or more of its other hydroxyl groups, is called the terminal sugar. For the avoidance of doubt, there may exist multiple terminal sugars in an oligosaccharide of a glycoprotein, but only one core sugar.

A glycan may be an O-linked glycan, an N-linked glycan or a C-linked glycan. In an O-linked glycan a monosaccharide or oligosaccharide glycan is bonded to an O-atom in an amino acid of the protein, typically via a hydroxyl group of serine (Ser) or threonine (Thr). In an N-linked glycan a monosaccharide or oligosaccharide glycan is bonded to the protein via an N-atom in an amino acid of the protein, typically via an amide nitrogen in the side chain of asparagine (Asn) or arginine (Arg). In a C-linked glycan a monosaccharide or oligosaccharide glycan is bonded to a C-atom in an amino acid of the protein, typically to a C-atom of tryptophan (Trp).

The end of an oligosaccharide that is directly attached to the protein is called the reducing end of a glycan. The other end of the oligosaccharide is called the non-reducing end of a glycan.

For O-linked glycans, a wide diversity of chains exist. Naturally occurring O-linked glycans typically feature a serine or threonine-linked α-O-GalNAc moiety, further substituted with galactose, sialic acid and/or fucose. The hydroxylated amino acid that carries the glycan substitution may be part of any amino acid sequence in the protein.

For N-linked glycans, a wide diversity of chains exist. Naturally occurring N-linked glycans typically feature an asparagine-linked β-N-GlcNAc moiety, in turn further substituted at its 4-OH with β-GlcNAc, in turn further substituted at its 4-OH with β-Man, in turn further substituted at its 3-OH and 6-OH with α-Man, leading to the glycan pentasaccharide Man₃GlcNAc₂. The core GlcNAc moiety may be further substituted at its 6-OH by α-Fuc. The pentasaccharide Man₃GlcNAc₂ is the common oligosaccharide scaffold of nearly all *N*-linked glycoproteins and may carry a wide variety of other substituents, including but not limited to Man, GlcNAc, Gal and sialic acid. The asparagine that is substituted with the glycan on its side-chain is typically part of the sequence Asn-X-Ser/Thr, with X being any amino acid but proline and Ser/Thr being either serine or threonine.

The term "antibody" is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multispecific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole antibodies, but also fragments of an antibody, for example an antibody Fab fragment, (Fab')₂, Fv fragment or Fc fragment from a cleaved antibody, a scFv-Fc fragment, a minibody, a diabody or a scFv. Furthermore, the term includes genetically engineered antibodies and derivatives of an antibody. Antibodies, fragments of antibodies and genetically engineered antibodies may be obtained by methods that are known in the art. Suitable marketed antibodies include, amongst others, abciximab, rituximab, basiliximab, palivizumab, infliximab, trastuzumab, alemtuzumab, adalimumab, tositumomab-¹³¹I, cetuximab, ibrituximab tiuxetan, omalizumab, bevacizumab, natalizumab, ranibizumab, panitumumab, eculizumab, certolizumab pegol, golimumab, canakinumab, catumaxomab, ustekinumab, tocilizumab, ofatumumab, denosumab, belimumab, ipilimumab and brentuximab.

The term "a thiol group precursor" as used herein refers to a derivative of a thiol-containing compound, wherein the thiol group is present in a protected form in order to mask the natural reactivity of the thiol group until a later stage. A protected form of a thiol typically involves the use of a protective group for the thiol, well-known in the art.

The terms "treatment," "treating," and the like refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment," as used herein, covers any treatment of a disease in a mammal, particularly in a human, and includes preventing the disease from occurring in a subject which may be predisposed to the disease but has not yet been diagnosed as having it; inhibiting the disease, i.e., arresting its development; relieving the disease, i.e., causing regression of the disease.

### Process for the preparation of an antibody-conjugate

The present invention relates to a process for the preparation of a glycoengineered antibody. The glycoengineered antibodies according to the invention comprise specifically designed properties.

The present invention relates to a process for the preparation of an antibody-conjugate. An antibody-conjugate is generally defined as an antibody (Ab) that is linked to one or more molecules of interest D via a linker L. The antibody may be linked to more than one linker, and/or a linker may be linked to more than one molecule of interest. Said molecule of interest D and linker L are described in more detail below.

In the antibody-conjugate according to the invention however, the antibody is linked to four or more molecules of interest D, via four or more linkers. The antibody-conjugate according the invention is described in more detail below.

The present invention relates to a process for the preparation of an antibody-conjugate, the process comprising the steps of:
(1) providing an IgG antibody comprising at least two N-linked glycosylation sites on the combination of a single heavy chain and single light chain; and
(2) trimming an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate; and
(3) optionally repeating step (2) in order to trim an oligosaccharide that is attached to a different glycosylation site; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(5) optionally:
   (5a) repeating step (2), in order to trim an oligosaccharide that is attached to a different glycosylation site; and
   (5b) repeating step (4); and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
(7) optionally:
   (7a) repeating step (2) in order to trim an oligosaccharide that is attached to a different glycosylation site; and
   (7b) repeating step (4); and
   (7c) repeating step (6); and
wherein the proximal N-linked GlcNAc-residue in steps (2), (4) and (6) is optionally fucosylated; wherein the process comprises one of steps (3), (5) and (7).

The enzyme in step (2) is preferably an endoglycosidase, more preferably an endo-β-N-acetylglucosaminidase.

The process comprises one of steps (3), (5) and (7). In other words, the process according to the invention preferably comprises step (3), or step (5), or step (7).

### Step (1)

In step (1) of the process according to the invention, an IgG antibody comprising at least two N-linked glycosylation sites on the combination of a single heavy chain and single light chain is provided.

When an IgG antibody is a whole antibody, the antibody is typically composed of two immunoglobulin (Ig) heavy chains and two Ig light chains. The Ig heavy chains comprise a constant region, composed of the constant domains C_{H}1, C_{H}2 and C_{H}3, and a variable region, V_{H}. The Ig light chains are composed of a variable region V_{L} and a constant region C_{L}.

Herein, the term "the combination of a single heavy chain and single light chain" refers to the combination of one heavy and one light Ig chain. Since a whole antibody comprises two heavy and two light chains, a whole antibody is a symmetrical dimer of two of said combinations of a single heavy chain and single light chain. The term "combination of a single heavy chain and single light chain" is herein merely used as a means to define the parts of an IgG antibody where said N-glycosylation sites may be present, and herein does not refer to e.g. a reduced IgG antibody, unless otherwise stated.

The term "an IgG antibody comprising at least two N-linked glycosylation sites on the combination of a single heavy chain and single light chain" herein thus defines that the two or more N-linked glycosylation sites may be present in the C_{H}1, C_{H}2, C_{H}3 and/or V_{H} domains of the heavy chain, but also in the C_{L} and/or V_{L} domain of the light chain. When an antibody comprises e.g. two or more N-linked glycosylation sites in the heavy chain C_{H}2 and C_{H}3 domains, and no N-linked glycosylation sites on the light chain, such an antibody is also deemed an IgG antibody comprising at least two N-linked glycosylation sites on the combination of a single heavy chain and single light chain.

It is to be understood that, when the IgG antibody is a whole antibody, and the combination of a single heavy chain and single light chain comprises e.g. two N-linked glycosylation sites, the whole antibody thus comprises four N-linked glycosylation sites.

As defined above, the term "antibody" herein not only refers to whole antibodies, but also to antibody fragments. When said IgG antibody comprising at least two N-linked glycosylation sites on the combination of a single heavy chain and single light chain is e.g. a Fab fragment comprising two or more N-linked glycosylation sites on the the C_{H}1, V_{H}, C_{L} and/or V_{L} domain, this Fab fragment is herein deemed to be an IgG antibody comprising at least two N-linked glycosylation sites on the combination of a single heavy chain and single light chain. Also e.g. an IgG antibody Fc fragment comprising two or more N-linked glycosylation sites on the C_{H}2 and/or C_{H}3 domain is deemed an IgG antibody comprising at least two N-linked glycosylation sites on the combination of a single heavy chain and single light chain, even though said fragment does not comprise a light chain domain. Examples of antibody fragments that herein may be deemed an IgG antibody comprising at least two N-linked glycosylation sites on the combination of a single heavy chain and single light chain include Fc fragments, Fab fragments, (Fab')₂ fragments, Fab' fragments, scFv fragments, reduced antibodies, diabodies, triabodies, tetrabodies, etc., provided that said fragments comprise two or more N-linked glycosylation sites.

In a preferred embodiment, the IgG antibody comprising at least two N-linked glycosylation sites on the combination of a single heavy chain and single light chain, is a whole antibody. When an IgG antibody comprising two or more N-linked glycosylation sites on the combination of a single heavy chain and single light chain is a whole antibody, i.e. an antibody comprising two heavy chains and two light chains, then the whole antibody comprises four N-linked glycosylation sites. Similarly, if there are three N-linked glycosylation sites on the combination of a single heavy chain and single light chain then the whole antibody comprises six N-linked glycosylation sites.

According to the invention, a combination of a single heavy chain and single light chain comprises two or more N-linked glycosylation sites. Preferably said combination comprises 2, 3, 4, 5, 6, 7, 8, 9 or 10 N-linked glycosylation sites, more preferably 2, 3, 4, 5, 6, 7 or 8, even more preferably 2, 3, 4, 5 or 6, even more preferably 2, 3 or 4 and most preferably 2 or 3 N-linked glycosylation sites. As a consequence, when the antibody is a whole antibody, the whole antibody comprises 4, 6, 8, 10, 12, 14, 16 18 or 20 N-linked glycosylation sites, preferably 4, 6, 8, 10, 12, 14, 16 or 18, even more preferably 4, 6, 8, 10, 12, even more preferably 4, 6 or 8 and most preferably 4 or 6 N-linked glycosylation sites.

The term "N-linked glycosylation site" herein refers to a site on an antibody where a mono- or oligosaccharide is attached to the antibody via an N-glycosidic bond. A mono- or oligosaccharide that is attached to an antibody is herein also referred to as a glycan. An N-linked glycosylation site may be a native N-linked glycosylation site of an antibody, but also an N-linked glycosylation site that is mutated into an antibody, e.g. by glycoengineering techniques.

In one embodiment, the IgG antibody comprising at least two N-linked glycosylation sites on the combination of a single heavy chain and single light chain comprises at least one native N-linked glycosylation site. A native N-linked glycosylation site may also be referred to as a conserved N-linked glycosylation site.

The Fc regions of IgG antibodies bear a highly conserved N-glycosylation site. The N-glycans attached to this site are predominantly core-fucosylated diantennary structures of the complex type. In addition, small amounts of these N-glycans also bear bisecting GlcNAc and α-2,6-linked sialic acid residues. The native glycosylation site in IgG is present at (or around) asparagine 297, also referred to as Asn297 or N297. In a further preferred embodiment, a native N-glycosylation site is present at N297.

In another embodiment, the IgG antibody comprising at least two N-linked glycosylation sites on the combination of a single heavy chain and single light chain comprises at least one mutant N-linked glycosylation site as compared to its wild type counterpart.

In yet another embodiment, the IgG antibody comprising at least two N-linked glycosylation sites on the combination of a single heavy chain and single light chain comprises at least one native and at least one mutant N-linked glycosylation site as compared to its wild type counterpart.

In yet another preferred embodiment, the amino acid sequence of the IgG heavy chain is altered in order to remove the native N-glycosylation site present at (or around) position 297. More preferably, said amino acid sequence is altered by mutating the asparagine on position 297 to glutamine, i.e. the IgG heavy chain preferably comprises an N297Q mutation. When the native N-glycosylation site around N297 is removed, said IgG antibody comprising at least two N-linked glycosylation sites on the combination of a single heavy chain and single light chain comprises two or more mutant N-linked glycosylation sites as compared to its wild type counterpart.

In another embodiment, said IgG antibody comprises two or more native N-linked N-glycosylation sites. In this embodiment, said IgG antibody may be a native antibody comprising two N-glycosylation sites.

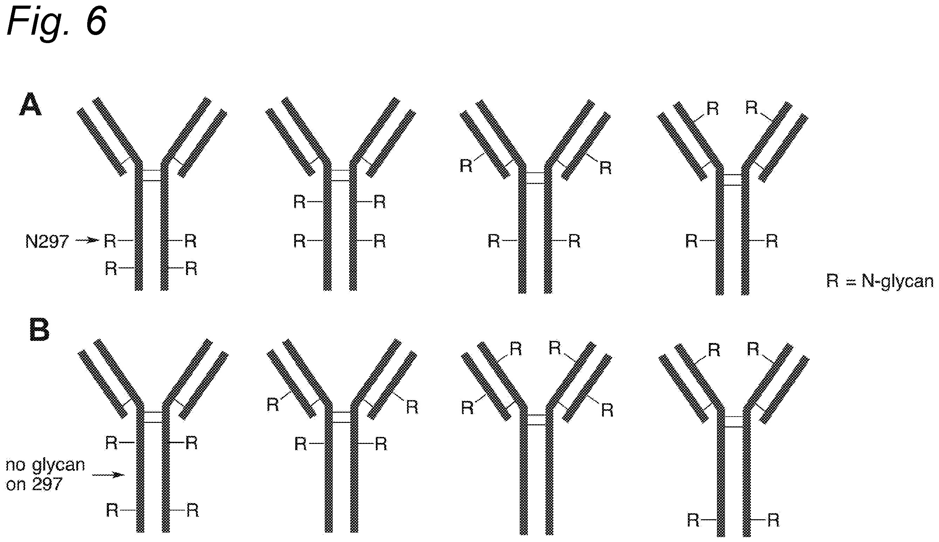

Figure 6A shows several examples of structures of IgGs with two glycosylation sites of which one is the native glycosylation site at N297. Figure 6B shows the possible structures of IgGs with two glycosylation sites but not the native glycosylation site at N297.

In the process for the preparation of an antibody-conjugate according to the invention, an antibody mixture may be used as the starting antibody, said mixture comprising antibodies comprising one or more non-fucosylated glycans and/or one or more fucosylated glycans. Advantageously, removal of fucose prior to the process according to the invention is therefore not necessary, since an antibody mixture comprising both fucosylated and non-fucosylated glycans may be used in the process.

In a specific embodiment of the process for the preparation of a modified antibody according to the invention, in step (1) the IgG antibody comprising at least two N-linked glycosylation sites on the combination of a single heavy chain and single light chain is provided by a site-specific mutagenesis process involving first the design of a potential N-glycosylation site based on the N-X-S/T sequence. However, most naturally occurring consensus sequences in secreted proteins are not glycosylated. Consensus sequences are necessary but not sufficient for N-linked carbohydrate addition and therefore expression of newly glycosylated proteins requires an extensive trial-and-error process. Possible secondary structures required for carbohydrate addition within functional glycosylation sites are β or Asn-X turns. Because carbohydrate addition precedes protein folding, sites introduced into normally buried positions of the molecule can be glycosylated however, the resultant proteins may have altered protein structures and/or or stabilities due to inhibition of correct protein folding.

### Step (2)

Step (2) of the process for the preparation of a modified antibody according to the invention comprises the trimming of an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate. The enzyme in step (2) is preferably an endoglycosidase, more preferably an endo-β-N-acetylglucosaminidase.

In the IgG antibody comprising at least two N-linked glycosylation sites on the combination of a single heavy chain and single light chain, at each N-glycosylation site an oligosaccharide is attached to the amide side chain of an antibody amino acid. The oligosaccharide is attached via an N-glycosidic bond, in most cases to an asparagine (Asn) or arginine (Arg) amino acid side chain. The oligosaccharide attached to the antibody is also referred to as a glycan. A glycan may for example be attached to an antibody via C1 of a GlcNAc-residue, which is bonded to the amide side chain of an asparagine amino acid that is part of the antibody.

Numerous different types of glycans exist. As described above, the Fc regions of IgG antibodies bear a highly conserved N-glycosylation site. The N-glycans attached to this site are predominantly core-fucosylated diantennary structures of the complex type, sometimes triantennary. Another type of glycan is the class of high mannose glycans. High-mannose typically comprises two N-acetylglucosamines and a varying number of mannose residues. Another type of glycan is the hybrid type, which has at least five mannose residues in the chain but also sugars of the complex type.

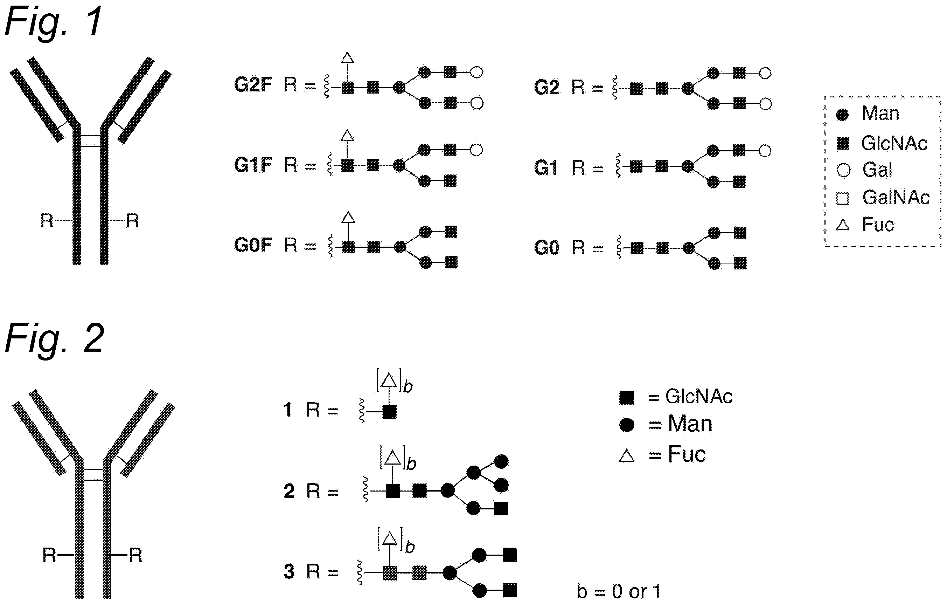

Figure 1 shows diantennary glycan of the complex type, and the different glycoforms with respect of galactosylation (G0, G1 and G3) and fucosylation (G0F, G1F and G2F).

Figure 2 shows examples of different glycosylation profiles of a monoclonal antibody that may be obtained by regular expression followed by trimming with endoglycosidase (**1**), or by trimming with α- and β-mannosidases (**2**). Glycoform **3** can be obtained by trimming of the regular mixture of glycoforms (G0, G1, G2, G0F, GIF and G2F) upon combined action of sialidase and galactosidase.

In a preferred embodiment, in an IgG antibody comprising at least two N-linked glycosylation sites on the combination of a single heavy chain and single light chain, the oligosaccharide attached at an N-linked glycosylation site is a diantennary glycan of the complex type, and glycoforms thereof. Typical glycoforms of an antibody obtained from a mammalian expression system are depicted in Figure 1.

As described above, a glycan may be bonded to the antibody via a GlcNAc-residue, and this GlcNAc residue may be fucosylated. In Figure 2, this is denoted by b: when b is 0, said GlcNAc-residue is non-fucosylated and when b is 1, said GlcNAc is fucosylated.

In a large number of glycans, a second GlcNAc-residue is bonded to the GlcNac-residue that is directly bonded to the antibody, as is also seen in Figure 2, (**2**) and (**3**). Trimming of an oligosaccharide (glycan) in step (2) of the process according to the invention occurs in between these two GlcNAc-residues. Trimming of a glycan according to step (2) of the process according to the invention provides a GlcNAc-residue that is covalently bonded to an N-glycosylation site on an antibody. This is also shown in Figure 2 (**1**). Such a GlcNAc-residue that is covalently bonded to an N-glycosylation site is herein referred to as a "proximal N-linked GlcNAc-residue", and also "core-GlcNAc-substituent". Said proximal N-linked GlcNAc-residue or core-GlcNAc-substituent is optionally fucosylated.

In step (2) of the process according to invention, the trimming of an oligosaccharide that is attached to an N-glycosylation site occurs by the action of a suitable enzyme, and after trimming the oligosaccharide (also referred to as glycan), a fucosylated (b in Figure 2(1) is 1) or a non-fucosylated (b is 0) proximal N-linked GlcNAc-residue (also referred to as core-GlcNAc-substituent) is obtained.

A "suitable enzyme" is defined as an enzyme wherefore the oligosaccharide that is to be trimmed is a substrate. The preferred type of enzyme that is to be used in step (2) depends on the specific oligosaccharide or oligosaccharides that is or are trimmed.

In a preferred embodiment of step (1) of the process according to the invention, the enzyme in step (2) is selected from the group of endoglycosidases.

Endoglycosidases are capable of cleaving internal glycosidic linkages in glycan structures, which provides another benefit to remodeling and synthetic endeavors. For example, endoglycosidases can be employed for facile homogenization of heterogeneous glycan populations, when they cleave at predictable sites within conserved glycan regions. One of the most significant classes of endoglycosidases in this respect comprises the endo-β-N-acetylglucosaminidases (EC 3.2.1.96, commonly known as Endos and ENGases;), a class of hydrolytic enzymes that remove N-glycans from glycoproteins by hydrolyzing the β-1,4-glycosidic bond in the N,N'-diacetylchitobiose core (reviewed by Wong et al. Chem. Rev. 2011, 111, 4259), leaving a single protein proximal N-linked GlcNAc residue. Endo-β-N-acetylglucosaminidases are found widely distributed through nature with common chemoenzymatic variants including Endo D, which is specific for pauci mannose; Endo A and Endo H, which are specific for high mannose; Endo F subtypes, which range from high mannose to biantennary complex; and Endo M, which can cleave most N-glycan structures (high mannose/complex-type/hybrid-type), except fucosylated glycans, and the hydrolytic activity for the high-mannose type oligosaccharides is significantly higher than that for the complex-and hybrid-type oligosaccharides. These ENGases show specificity toward the distal N-glycan structure and not the protein displaying it, making them useful for cleaving most N-linked glycans from glycoproteins under native conditions.

Endoglycosidases F1, F2, and F3 are most suitable for deglycosylation of native proteins. The linkage specificities of endo F1, F2, and F3 suggest a general strategy for deglycosylation of proteins that may remove all classes of N-linked oligosaccharides without denaturing the protein. Biantennary and triantennary structures can be immediately removed by endoglycosidases F2 and F3, respectively. Oligo- mannose and hybrid structures can be removed by Endo F1.

Endo F3 is unique in that its cleavage is sensitive to the state of peptide linkage of the oligosaccharide, as well as the state of core fucosylation. Endoglycosidase F3 cleaves asparagine-linked biantennary and triantennary complex oligosaccharides. It will cleave non-fucosylated biantennary and triantennary structures at a slow rate, but only if peptide-linked. Core fucosylated biantennary structures are efficient substrates for Endo F3, which activity up to 400-fold. There is no activity on oligomannose and hybrid molecules. See for example Tarentino et al. Glycobiology 1995, 5, 599.

Endo S is a secreted endoglycosidase from *Streptococcus pyogenes,* and also belongs to the glycoside hydrolase family 18, as disclosed by Collin et al. (EMBO J. 2001, 20, 3046). In contrast to the ENGases mentioned above, endo S has a more defined specificity and is specific for cleaving only the conserved N-glycan in the Fc domain of human IgGs (no other substrate has been identified to date), suggesting that a protein-protein interaction between the enzyme and IgG provides this specificity.

Endo S49 is described in WO 2013/037824 (Genovis AB). Endo S49 is isolated from Streptococcus poyogenes NZ131 and is a homologue of Endo S. Endo S49 has a specific endoglycosidase activity on native IgG and cleaves a larger variety of Fc glycans than Endo S.

In a further preferred embodiment, the enzyme in step (2) is an endo-β-N-acetylglucosaminidase. In a further preferred embodiment, the endo-β-N-acetylglucosaminidase is selected from the group consisting of Endo S, Endo S49, Endo F1, Endo F2, Endo F3, Endo H, Endo M, Endo A, and any combination thereof.

When the oligosaccharide to be trimmed is a diantennary structure of the complex type, the endo-β-N-acetylglucosaminidase is preferably selected from the group consisting of Endo S, Endo S49, Endo F1, Endo F2, Endo F3, and a combination thereof.

When the oligosaccharide to be trimmed is a diantennary structure of the complex type (i.e. according to Figure 2(**3**)), and it is present at the IgG conserved N-glycosylation site at N297, the endo-β-N-acetylglucosaminidase is preferably selected from the group consisting of Endo S, Endo S49, Endo F2, Endo F3, and a combination thereof, more preferably from the group consisting of Endo S, Endo S49, and a combination thereof.

When the oligosaccharide to be trimmed is a diantennary structure of the complex type, and it is not present at the IgG conserved N-glycosylation site at N297, the endo-β-N-acetylglucosaminidase is preferably selected from the group consisting of Endo F2 and Endo F3, and a combination thereof.

When the oligosaccharide to be trimmed is a high mannose, the endo-β-N-acetylglucosaminidase is preferably selected from the group consisting of Endo H, Endo M, Endo A and Endo F1.

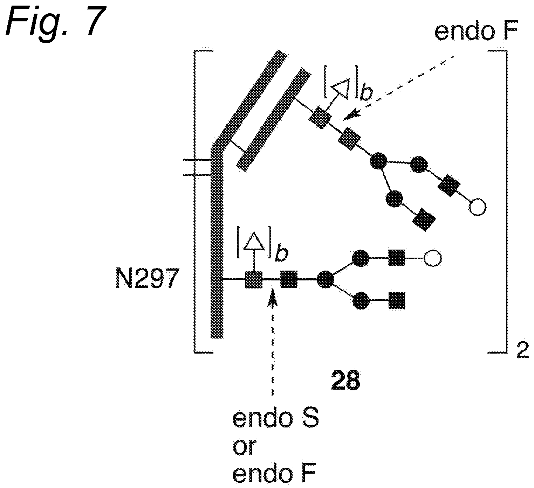

Figure 7 shows the enzymatic cleavage sites of an IgG antibody comprising two N-linked glycosylation sites on the combination of a single heavy chain and a single light chain (i.e. the total number of N-glycosylation sites in a whole antibody is four), one of which is the native glycosylation site at N297.

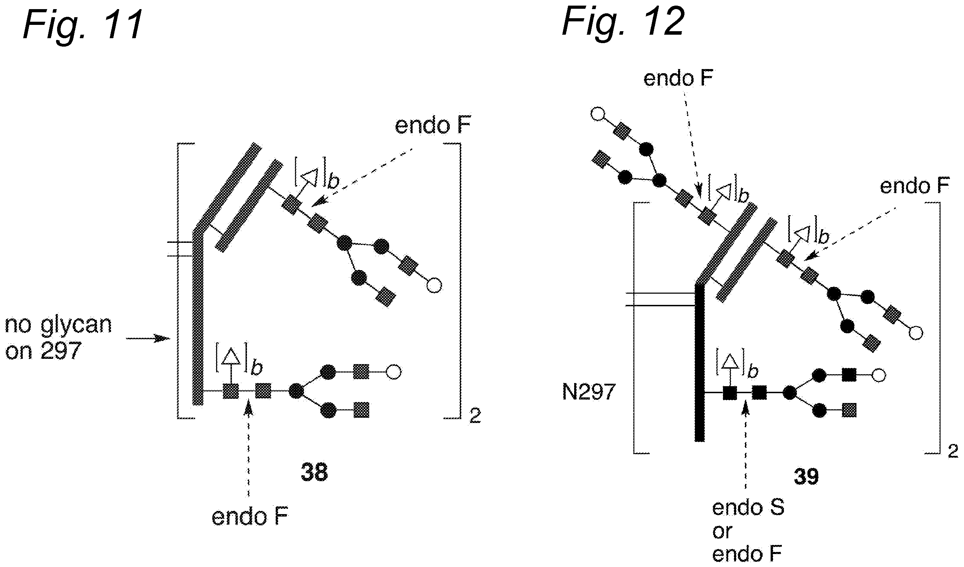

Figure 11 shows the enzymatic cleavage sites of an IgG antibody comprising two N-linked glycosylation sites on the combination of a single heavy chain and a single light chain (i.e. the total number of N-glycosylation sites in a whole antibody is four), neither of which is the native glycosylation site at N297.

Figure 12 shows the enzymatic cleavage sites of an IgG antibody comprising three N-linked glycosylation sites on the combination of a single heavy chain and a single light chain (i.e. the total number of N-glycosylation sites in a whole antibody is four), one of which is the native glycosylation site at N297, and both others are mutant N-linked glycosylation sites.

The trimming step (2) of the process according to the invention is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer.

The process is preferably performed at a temperature in the range of about 4 to about 50°C, more preferably in the range of about 10 to about 45°C, even more preferably in the range of about 20 to about 40°C, and most preferably in the range of about 30 to about 37°C.

The process is preferably performed a pH in the range of about 5 to about 9, preferably in the range of about 5.5 to about 8.5, more preferably in the range of about 6 to about 8. Most preferably, the process is performed at a pH in the range of about 7 to about 8.

In a preferred embodiment, different types of oligosaccharides may be trimmed simultaneously in a one-pot procedure by choosing the right combination of enzyme or the right combination of enzymes.

### Step 3

Step (3) of the process according to the invention is an optional step. As was described above, the process comprises one of steps (3), (5) and (7).

When step (3) is included in the process, this means that the trimming step (2) of the process is repeated, in order to trim an oligosaccharide that is attached to a different glycosylation site than the oligosaccharide that was already trimmed during the first time that step (2) was performed.

Although different oligosaccharides may be trimmed simultaneous in step (2), in some instances it is preferred to trim a different oligosaccharide in a separate step.

When the IgG antibody comprising at least two N-linked glycosylation sites comprises a conserved glycosylation site at N297, it is preferred that the oligosaccharide attached to that conserved site is trimmed during the first execution of step (2). Endo S and Endo S29 do have a very high efficiency for the trimming of an oligosaccharide at the N297 conserved site, however efficiency for other glycosilation sites is low. If an oligosaccharide at N297 needs to be trimmed, it is preferred that this native site is trimmed during the first execution of step (2). In other words, if step (3) is present in the process according to the invention, it is preferred that a glycosylation site other than N297 is trimmed in this step. As a consequence it is preferred in step (3) that the endo-β-N-acetylglucosaminidase is selected from the group consisting of Endo F1, Endo F2, Endo F3, Endo H, Endo M, Endo M, and any combination thereof.

The description of the details of step (2), and the preferred embodiments thereof, also hold for step 3. A preferred embodiment of a process for the preparation of an antibody-conjugate comprising step (3) is described in more detail below.

### Step (4)

In step (4) of the process for the preparation of an antibody-conjugate, a monosaccharide derivative Su(A)ₓ is attached to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site.

In a preferred embodiment, this step of said process comprises contacting an IgG antibody comprising a proximal N-linked GlcNAc-residue with Su(A)ₓ-P in the presence of a suitable catalyst; wherein the proximal N-linked GlcNAc-residue of said antibody is optionally fucosylated; wherein a suitable catalyst is defined as a galactosyltransferase or a galactosyltransferase comprising a mutant catalytic domain, wherefore Su(A)ₓ-P is a substrate; wherein Su(A)ₓ is a sugar derivative comprising x functional groups A wherein x is 1, 2, 3 or 4 and A is independently selected from the group consisting of an azido group, a keto group, an alkynyl group, a thiol group or a precursor thereof, a halogen, a sulfonate group, a halogenated acetamido group, a mercaptoacetamido group and a sulfonated acetamido group; wherein P is a nucleotide.

Step (4) of the process for the preparation of an antibody-conjugate according to the invention is performed in the presence of a suitable catalyst. A suitable catalyst is defined as an enzyme, wherefore Su(A)ₓ-P is a substrate.

When the catalyst is a galactosyltransferase, i.e. without a mutant domain, said galactosyltransferase preferably is a wild-type galactosyltransferase. When the catalyst is a galactosyltransferase comprising a mutant catalytic domain, said mutant GalT domain may be present within a full-length GalT enzyme, but it may also be present in a recombinant molecule comprising a catalytic domain.

In one embodiment, the catalyst is a wild-type galactosyltransferase, more preferably a wild-type *β*(1,4)-galactosyltransferase or a wild-type *β*(1,3)-N-galactosyltransferase, and even more preferably a wild-type *β*(1,4)-galactosyltransferase. *β*(1,4)-Galactosyltransferase is herein further referred to as GalT. Even more preferably, the *β*(1,4)-galactosyltransferase is selected from the group consisting of a bovine *β*(1,4)-Gal-T1, a human *β*(1,4)-Gal-T1, a human *β*(1,4)-Gal-T2, a human *β*(1,4)-Gal-T3 and a human *β*(1,4)-Gal-T4. Even more preferably, the *β*(1,4)-galactosyltransferase is a *β*(1,4)-Gal-T1. When the catalyst is a wild-type *β*(1,3)-*N*-galactosyltransferase, a human *β*(1,3)-Gal-T5 is preferred.

This embodiment wherein the catalyst is a wild-type galactosyltransferase is particularly preferred when a functional group A in sugar derivative Su(A)ₓ is present on C2 or C6, preferably C6, of said sugar derivative. In this embodiment, it is further preferred that Su(A)ₓ comprises one functional group A, i.e. preferably x is 1. P, Su(A)ₓ and Su(A)ₓ-P are described in more detail below.

In a specific embodiment of step (4) of the process according to the invention, step (4) comprises contacting an IgG antibody comprising a proximal N-linked GlcNAc-residue with Su(A)ₓ-P in the presence of a suitable catalyst; wherein the proximal N-linked GlcNAc residue of said antibody is optionally fucosylated; wherein a suitable catalyst is defined as a galactosyltransferase or a galactosyltransferase comprising a mutant catalytic domain, wherefore Su(A)ₓ-P is a substrate; wherein Su(A)ₓ is a sugar derivative comprising x functional groups A wherein x is 1, 2, 3 or 4 and A is independently selected from the group consisting of an azido group, a keto group, an alkynyl group, a thiol group or a precursor thereof, a halogen, a sulfonyloxy group, a halogenated acetamido group, a mercaptoacetamido group and a sulfonyloxy acetamido group; wherein P is a nucleotide; with the proviso that when the catalyst is a wild-type galactosyltransferase, then Su(A)ₓ-P comprises one functional group A (i.e. x is 1), and said functional group A is present on C2 or C6, preferably C6, of Su(A)ₓ.

Accordingly, in a specific embodiment, step (4) of the process for the preparation of an antibody-conjugate comprises contacting a an IgG antibody comprising a proximal N-linked GlcNAc-residue with Su(A)ₓ-P in the presence of a suitable catalyst; wherein the proximal N-linked GlcNAc-residue is optionally fucosylated; wherein a suitable catalyst is defined as a wild-type galactosyltransferase wherefore Su(A)ₓ-P is a substrate; wherein Su(A)ₓ is a sugar derivative comprising x functional groups A wherein x is 1, A is present on C2 or C6, preferably C6, of sugar derivative Su and A is independently selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group; wherein P is a nucleotide.

Preferably, the wild-type galactosyltransferase in this specific embodiment is a *β*(1,4)-galactosyltransferase or a *β*(1,3)-N-galactosyltransferase, more preferably a *β*(1,4)-galactosyltransferase. Even more preferably, the wild-type a *β*(1,4)-galactosyltransferase is a wild-type human GalT, more preferably a wild-type human GalT selected from the group consisting of a wild-type human *β*4-Gal-T1, a wild-type human *β*(1,4)-Gal-T2, a wild-type human *β*(1,4)-Gal-T3 and a wild-type human *β*(1,4)-Gal-T4.

In another embodiment of the process for the preparation of an antibody-conjugate according to the invention, the catalyst is a galactosyltransferase comprising a mutant catalytic domain, preferably a *β*(1,4)-galactosyltransferase comprising a mutant catalytic domain or a *β*(1,3)-N-galactosyltransferase comprising a mutant catalytic domain, more preferably a *β*(1,4)-galactosyltransferase comprising a mutant catalytic domain. *β*(1,4)-Galactosyltransferase I is herein further referred to as GalT.

In a preferred embodiment the catalyst is a *β*(1,3)-N-galactosyltransferase comprising a mutant catalytic domain, and preferably said *β*(1,3)-N-galactosyltransferase is a human *β*(1-3)-Gal-T5.

More preferably, the catalyst is a *β*(1,4)-N-galactosyltransferase comprising a mutant catalytic domain, more preferably, a *β*(1,4)-galactosyltransferase I comprising a mutant catalytic domain, and even more preferably selected from the group consisting of a bovine *β*(1,4)-Gal-T1, a human *β*4-Gal-T1, a human *β*(1,4)-Gal-T2, a human *β*(1,4)-Gal-T3 and a human *β*(1,4)-Gal-T4, all comprising a mutant catalytic domain.

Most preferably the catalyst is a bovine *β*(1,4)-Gal-T1 comprising a mutant catalytic domain.

Several suitable catalysts for step (4) of the process for the preparation of an antibody-conjugate according to the invention are known in the art. A suitable catalyst is for example a catalyst that comprises a mutant catalytic domain from a *β*(1,4)-galactosyltransferase I. A catalytic domain herein refers to an amino acid segment that folds into a domain that is able to catalyze the linkage of the specific sugar derivative nucleotide Su(A)ₓ-P to the terminal non-reducing GlcNAc-glycan in a specific process according to the invention. *β*(1,4)-galactosyltransferase I is herein further referred to as GalT. Such mutant GalT catalytic domains are for example disclosed in J. Biol. Chem. 2002, 277, 20833 and WO 2004/063344 (National Institutes of Health). J. Biol. Chem. 2002, 277, 20833 and WO 2004/063344 disclose Tyr-289 mutants of bovine *β*(1,4)-Gal-T1, which are referred to as Y289L, Y289N and Y289I. The method of preparation of said mutant catalytic domains Y289L, Y289N and Y289I is disclosed in detail in WO 2004/063344, p. 34, l. 6 - p. 36, l. 2.

Mutant GalT domains that catalyze the formation of a glucose-*β*(1,4)-N-acetylglucosamine bond are disclosed in WO 2004/063344 on p. 10, l, 25 - p. 12, 1. 4. Mutant GalT domains that catalyze the formation of an N-acetylgalactosamine-*β*(1,4)-N-acetylglucosamine bond are disclosed in WO 2004/063344 on p. 12, 1, 6 - p. 13, 1. 2. Mutant GalT domains that catalyze the formation of a N-acetylglucosamine-*β*(1,4)-N-acetylglucosamine bond and a mannose-*β*(1,4)-N-acetylglucosamine bond are disclosed in WO 2004/063344 on p. 12, l, 19 - p. 14, l. 6.

The disclosed mutant GalT domains may be included within full-length GalT enzymes, or in recombinant molecules containing the catalytic domains, as is disclosed in WO 2004/063344 on p. 14, l, 31 - p. 16, l. 28.

Another mutant GalT domain is for example Y284L, disclosed by Bojarová et al., Glycobiology 2009, 19, 509, wherein Tyr284 is replaced by leucine.

Another mutant GalT domain is for example R228K, disclosed by Qasba et al., Glycobiology 2002, 12, 691, wherein Arg228 is replaced by lysine.

The catalyst may also comprise a mutant catalytic domain from a bovine *β*(1,4)-galactosyltransferase, selected from the group consisting of GalT Y289N, GalT Y289F, GalT Y289M, GalT Y289V, GalT Y289G, GalT Y289I and GalT Y289A, preferably selected from the group consisting of GalT Y289F and GalT Y289M. GalT Y289N, GalT Y289F, GalT Y289M, GalT Y289V, GalT Y289G, GalT Y289I and GalT Y289A may be provided via site-directed mutagenesis processes, in a similar manner as disclosed in WO 2004/063344, in Qasba et al., Prot. Expr. Pur. 2003, 30, 219 and in Qasba et al., J. Biol. Chem. 2002, 277, 20833 for Y289L, Y289N and Y289I. In GalT Y289N the tyrosine amino acid (Y) at position 289 is replaced by an asparagine (N) amino acid, in GalT Y289F the tyrosine amino acid (Y) at position 289 is replaced by a phenyl alanine (F) amino acid, in GalT Y289M said tyrosine is replaced by a methionine (M) amino acid, in GalT Y289V by a valine (V) amino acid, in GalT Y289G by a glycine (G) amino acid, in GalT Y289I by an isoleucine (I) amino acid and in Y289A by an analine (A) amino acid.

In a preferred embodiment of the process for the preparation of a modified antibody according to the invention, said catalyst is a catalyst comprising a mutant catalytic domain from a *β*(1,4)-galactosyltransferase, preferably from a bovine β(1,4)-Gal-T1.

Preferably, the catalyst is a catalyst comprising a mutant catalytic domain from a *β*(1,4)-galactosyltransferase, preferably selected from the group consisting of bovine *β*(1,4)-Gal-T1 GalT Y289L, GalT Y289N, GalT Y289I, GalT Y289F, GalT Y289M, GalT Y289V, GalT Y289G and GalT Y289A, more preferably selected from the group consisting of bovine *β*(1,4)-Gal-T1 GalT Y289L, GalT Y289N and GalT Y289I.

In a further preferred embodiment, said catalyst is a catalyst comprising a GalT mutant catalytic domain selected from the group consisting of Y289L, Y289N, Y289I, Y284L, R228K, Y289F, Y289M, Y289V, Y289G and Y289A, preferably selected from the group consisting of Y289L, Y289N, Y289I, Y284L and R228K. In another preferred embodiment, said catalyst is a catalyst comprising a bovine *β*(1,4)-Gal-T1 mutant catalytic domain selected from the group consisting of Y289F, Y289M, Y289V, Y289G and Y289A. More preferably said catalyst is a catalyst comprising a GalT mutant catalytic domain selected from the group consisting of Y289L, and Y289I, and most preferably said catalyst is a catalyst comprising a GalT mutant catalytic domain selected from the group consisting of Y289L.

Another type of suitable catalysts is a catalyst based on *α*(1,3)-N-galactosyltransferase (further referred to as α3Gal-T), preferably *α*(1,3)-N-acetylgalactosaminyltransferase (further referred to as α3GalNAc-T), as disclosed in WO 2009/025646. Mutation of α3Gal-T can broaden donor specificity of the enzyme, and make it an α3GalNAc-T. Mutation of α3GalNAc-T can broaden donor specificity of the enzyme. Polypeptide fragments and catalytic domains of *α*(1,3)-N-acetylgalactosaminyltransferases are disclosed in WO 2009/025646 on p. 26, l. 18 - p. 47, l. 15 and p. 77,1. 21 - p. 82,1. 4.

Step (4) of the process for the preparation of an antibody-conjugate according to the invention is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer.

The process is preferably performed at a temperature in the range of about 4 to about 50°C, more preferably in the range of about 10 to about 45°C, even more preferably in the range of about 20 to about 40°C, and most preferably in the range of about 30 to about 37°C.

The process is preferably performed a pH in the range of about 5 to about 9, preferably in the range of about 5.5 to about 8.5, more preferably in the range of about 6 to about 8. Most preferably, the process is performed at a pH in the range of about 7 to about 8.

Su(A)ₓ is defined as a monosaccharide derivative (which may also be referred to as a sugar derivative) comprising x functional groups A, wherein x is 1, 2, 3 or 4 and wherein A is independently selected from the group consisting of an azido group, a keto group an alkynyl group, a thiol group or a precursor thereof, a halogen, a sulfonyloxy group, a halogenated acetamido group, a mercaptoacetamido group and a sulfonylated hydroxyacetamido group.

A Su(A)ₓ-moiety may also be referred to as a "modified sugar". A modified sugar is herein defined as a sugar or a sugar derivative, said sugar or sugar derivative comprising 1, 2, 3 or 4 functional groups A, wherein A is selected from the group consisting of an azido group, a keto group an alkynyl group, a thiol group or a precursor thereof, a halogen, a sulfonyloxy group, a halogenated acetamido group, a mercaptoacetamido group and a sulfonylated hydroxyacetamido group.

When a modified sugar or sugar derivative comprises e.g. an azido group, said sugar or sugar derivative may be referred to as an azido-modified sugar or sugar derivative. When a modified sugar or sugar derivative comprises e.g. a keto group, said sugar or sugar derivative may be referred to as a keto-modified sugar or sugar derivative. When a modified sugar or sugar derivative comprises e.g. an alkynyl group, said sugar or sugar derivative may be referred to as an alkynyl-modified sugar or sugar derivative. When a modified sugar or sugar derivative comprises e.g. a thiol group, said sugar or sugar derivative may be referred to as a thiol-modified sugar or sugar derivative. When a modified sugar or sugar derivative comprises e.g. a thiol-precursor group, said sugar or sugar derivative may be referred to as a thiol-precursor-modified sugar or sugar derivative. When a modified sugar or sugar derivative comprises e.g. a halogen, said sugar or sugar derivative may be referred to as a halogen-modified sugar or sugar derivative. When a modified sugar or sugar derivative comprises e.g. a sulfonyloxy group, said sugar or sugar derivative may be referred to as a sulfonyloxy-modified sugar or sugar derivative.

An azido group is herein defined as a -[C(R⁷)₂]ₒN₃ group, wherein R⁷ is independently selected from the group consisting of hydrogen, halogen and an (optionally substituted) C₁- C₂₄ alkyl group, and o is 0 - 24. Preferably R⁷ is hydrogen or a C₁, C₂, C₃ or C₄ alkyl group, more preferably R⁷ is hydrogen or -CH₃. Preferably o is 0 - 10, more preferably 0, 1, 2, 3, 4, 5 or 6. More preferably, R⁷ is hydrogen, -CH₃ or a C₂ alkyl group and/or o is 0, 1, 2, 3 or 4. Even more preferably R⁷ is hydrogen and o is 1 or 2. Most preferably o is 0.

A keto group is herein defined as a -[C(R⁷)₂]ₒC(O)R⁶ group, wherein R⁶ is an optionally substituted methyl group or an optionally substituted C₂- C₂₄ alkyl group, R⁷ is independently selected from the group consisting of hydrogen, halogen, methyl and R⁶, and o is 0 - 24, preferably 0 - 10, and more preferably 0, 1, 2, 3, 4, 5 or 6. Preferably, R⁷ is hydrogen. In a preferred embodiment, R⁶ is an optionally substituted C₂- C₂₄ alkyl group. When Su(A)ₓ is derived from an amino sugar, and A is a keto group bonded to the amino sugar N-atom and o is 0 (i.e. when Su(A) comprises an-NC(O)R⁶ substituent), R⁶ is an optionally substituted C₂- C₂₄ alkyl group.

An alkynyl group is preferably a terminal alkynyl group or a (hetero)cycloalkynyl group as defined above. In one embodiment the alkynyl group is a -[C(R⁷)₂]ₒC≡C-R⁷ group, wherein R⁷ and o are as defined above; R⁷ is preferably hydrogen. More preferably, o is 0, 1, 2, 3, 4, 5 or 6 and R⁷ is hydrogen. Most preferably o is 0.

A thiol group is herein defined as a -[C(R⁷)₂]ₒSH group, wherein R⁷ is independently selected from the group consisting of hydrogen, halogen and an (optionally substituted) C₁- C₂₄ alkyl group, and o is 0 - 24. Preferably R⁷ is hydrogen or a C₁, C₂, C₃ or C₄ alkyl group, more preferably R⁷ is hydrogen or -CH₃. Preferably o is 0 - 10, more preferably 0, 1, 2, 3, 4, 5 or 6. More preferably, R⁷ is hydrogen, -CH₃ or a C₂ alkyl group and/or o is 0, 1, 2, 3 or 4. Even more preferably R⁷ is hydrogen and o is 0, 1, 2 or 3, more preferably o is 1 or 2, most preferably o is 0 or 1. Most preferably o is 0. In a particularly preferred embodiment, R⁷ is hydrogen and o is 0. In another particularly preferred embodiment, R⁷ is hydrogen and o is 1. In another particularly preferred embodiment, R⁷ is hydrogen and o is 2. In another particularly preferred embodiment, R⁷ is hydrogen and o is 3.

A precursor of a thiol group is herein defined as a -[C(R⁷)₂]ₒSC(O)CH₃ group, wherein R⁷ and o, as well as their preferred embodiments, are as defined above for a thiol group. In a particularly preferred embodiment, R⁷ is hydrogen and o is 0. In another particularly preferred embodiment, R⁷ is hydrogen and o is 1. In another particularly preferred embodiment, R⁷ is hydrogen and o is 2. In another particularly preferred embodiment, R⁷ is hydrogen and o is 3. Most preferably, said thiol-precursor is -CH₂CH₂CH₂SC(O)CH₃, -CH₂CH₂SC(O)CH₃, -CH₂SC(O)CH₃ or -SC(O)CH₃, preferably -SC(O)CH₃. In step (4) of the process for the preparation of an antibody-conjugate according to the invention, a sugar derivative Su(A)ₓ wherein A is a precursor of a thiol group may be used. During said process, the thiol-precursor is converted to a thiol group.

A halogen is herein defined as F, Cl, Br or I. Preferably, said halogen is Cl, Br or I, more preferably Cl.

A sulfonyloxy group is herein defined as a -[C(R⁷)₂]ₒOS(O)₂R⁸ group, wherein R⁷ and o are as defined above for a thiol group, and R⁸ is selected from the group consisting of C₁ - C₂₄ alkyl groups, C₇ - C₂₄ alkylaryl groups and C₇ - C₂₄ arylalkyl groups. R⁸ is preferably a C₁ - C₄ alkyl group, C₇ - C₁₂ alkylaryl group or a C₇ - C₁₂ arylalkyl group, more preferably -CH₃, -C₂H₅, a C₃ linear or branched alkyl group or a C₇ alkylaryl group. R⁸ may also be a C₁ - C₂₄ aryl group, preferably a phenyl group. R⁸ is most preferably a methyl group, an ethyl group, a phenyl group or a p-tolyl group. Preferably R⁷ is hydrogen or a C₁, C₂, C₃ or C₄ alkyl group, more preferably R⁷ is hydrogen or -CH₃. Preferably o is 0 - 10, more preferably 0, 1, 2, 3, 4, 5 or 6. More preferably, R⁷ is hydrogen, -CH₃ or a C₂ alkyl group and/or o is 0, 1, 2, 3 or 4. Even more preferably R⁷ is hydrogen and o is 1 or 2, most preferably o is 0. R⁸ is preferably a C₁ - C₄ alkyl group, a C₇ - C₁₂ alkylaryl group or a C₇ - C₁₂ arylalkyl group, more preferably -CH₃, -C₂H₅, a C₃ linear or branched alkyl group or a C₇ alkylaryl group. It is also preferred that R⁸ is a phenyl group. Most preferably the sulfonyloxy group is a mesylate group, -OS(O)₂CH₃, a benzenesulfonate group (-OS(O)₂(C₆H₅)) or a tosylate group (-OS(O)₂(C₆H₄CH₃)).

A halogenated acetamido group is herein defined as an -NHC(O)[C(R⁷)₂]ₒX group, wherein R⁷ is independently selected from the group consisting of hydrogen, halogen and an (optionally substituted) C₁- C₂₄ alkyl group, X is F, Cl, Br or I, and o is 0 - 24. Preferably R⁷ is hydrogen or a C₁, C₂, C₃ or C₄ alkyl group, more preferably R⁷ is hydrogen or -CH₃, most preferably hydrogen. Preferably o is 0 to 10, more preferably 1, 2, 3, 4, 5 or 6, even more preferably 1, 2, 3 or 4 and most preferably o is 1. More preferably, R⁷ is hydrogen, -CH₃ or a C₂ alkyl group and/or o is 1, 2, 3 or 4 and most preferably R⁷ is hydrogen and o is 1. Preferably, X is Cl or Br, more preferably X is Cl. Most preferably, R⁷ is hydrogen, X is Cl and o is 1.

A mercaptoacetamido group is herein defined as an -NHC(O)[C(R⁷)₂]ₒSH group, wherein R⁷ is independently selected from the group consisting of hydrogen, halogen and an (optionally substituted) C₁ - C₂₄ alkyl group and o is 0 - 24. Preferably R⁷ is hydrogen or a C₁, C₂, C₃ or C₄ alkyl group, more preferably R⁷ is hydrogen or -CH₃, most preferably hydrogen. Preferably o is 0 to 10, more preferably 1, 2, 3, 4, 5 or 6, even more preferably 1, 2, 3 or 4 and most preferably o is 2, 3 or 4. More preferably, R⁷ is hydrogen, -CH₃ or a C₂ alkyl group and/or o is 1, 2, 3 or 4. More preferably, R⁷ is hydrogen and o is 1, 2, 3 or 4. Most preferably, R⁷ is hydrogen and o is 1, 2 or 3, preferably 1. Preferred examples include a mercaptoethanoylamido group, a mercaptopropanoylamido group, a mercaptobutanoylamido group and a mercaptopentanoylamido group, preferably a mercaptopropanoylamido group.

A sulfonated hydroxyacetamido group is herein defined as a -NHC(O)[C(R⁷)₂] ₒOS(O)₂R⁸ group, wherein R⁷ is independently selected from the group consisting of hydrogen, halogen and an (optionally substituted) C₁ - C₂₄ alkyl group, R⁸ is selected from the group consisting of C₁ - C₂₄ alkyl groups, C₆ - C₂₄ aryl groups, C₇ - C₂₄ alkylaryl groups and C₇ - C₂₄ arylalkyl groups, and o is 0 - 24. R⁸ is preferably a C₁ - C4 alkyl group, a C₆ - C₁₂ aryl group, a C₇ - C₁₂ alkylaryl group or a C₇ - C₁₂ arylalkyl group, more preferably -CH₃, -C₂H₅, a C₃ linear or branched alkyl group, a C₆ - C₉ aryl group or a C₇ alkylaryl group. Most preferably the sulfonyloxy group is a mesylate group -OS(O)₂CH₃, a benzenesulfonate group -OS(O)₂(C₆H₅) or a tosylate group -OS(O)₂(C₆H₄CH₃). Preferably R⁷ is hydrogen or a C₁, C₂, C₃ or C₄ alkyl group, more preferably R⁷ is hydrogen or -CH₃, most preferably hydrogen. Preferably o is 0 to 10, more preferably 1, 2, 3, 4, 5 or 6, even more preferably 1, 2, 3 or 4 and most preferably o is 1. More preferably, R⁷ is hydrogen, -CH₃ or a C₂ alkyl group and/or o is 1, 2, 3 or 4. Even more preferably R⁷ is hydrogen and o is 1, 2 or 3. Yet even more preferably, R⁷ is H, o is 1 and R⁸ is a mesylate group, a benzenesulfonate group or a tosylate group. Most preferably, R⁷ is hydrogen, R⁸ is -CH₃ and o is 1.

The sugar derivative Su(A)ₓ comprises one or more functional groups A. When Su(A)ₓ comprises two or more functional groups A, each functional group A is independently selected, i.e. one Su(A)ₓ may comprise different functional groups A, e.g. an azido group and a keto group, etc. In a preferred embodiment, x is 1 or 2, more preferably x is 1. In another preferred embodiment, functional group A is an azido group or a keto group, more preferably an azido group. In another preferred embodiment, functional group A is a thiol group or a halogen, more preferably a halogen. In a further preferred embodiment, x is 1 and A is an azido group, a keto group, a thiol group or a halogen.

Sugar derivative Su(A)ₓ is derived from a sugar or a sugar derivative Su, e.g. an amino sugar or an otherwise derivatized sugar. Examples of sugars and sugar derivatives include galactose (Gal), mannose (Man), glucose (Glc), N-acetylneuraminic acid or sialic acid (Sial) and fucose (Fuc).

An amino sugar is a sugar wherein a hydroxyl (OH) group is replaced by an amine group and examples include glucosamine (GlcNH₂) and galactosamine (GalNH₂). Examples of an otherwise derivatized sugar include N-acetylneuraminic acid (sialic acid, Sia or NeuNAc) or fucose (Fuc).

Sugar derivative Su(A)ₓ is preferably derived from galactose (Gal), mannose (Man), N-acetylglucosamine (GlcNAc), glucose (Glc), N-acetylgalactosamine (GalNAc), fucose (Fuc) and N-acetylneuraminic acid (sialic acid Sia or NeuNAc), preferably from the group consisting of GlcNAc, Glc, Gal and GalNAc. More preferably Su(A)ₓ is derived from Gal or GalNAc, and most preferably Su(A)ₓ is derived from GalNAc.

The one or more functional groups A in Su(A)ₓ may be linked to the sugar or sugar derivative Su in several ways. The one or more functional groups A may be bonded to C2, C3, C4 and/or C6 of the sugar or sugar derivative, instead of a hydroxyl (OH) group. It should be noted that, since fucose lacks an OH-group on C6, if A is bonded to C6 of Fuc, then A takes the place of an H-atom.

In a preferred embodiment, the one or more functional groups A in Su(A)ₓ are present on C2 and/or C6 of the sugar or sugar derivative Su. When a functional group A is present instead of an OH-group on C2 of a sugar or sugar derivative, A is preferably selected from the group consisting of an azido group, a halogenated acetamido group, a mercaptoacetamido group and a sulfonylated hydroxyacetamido group. However, when A is present on C2 of a 2-aminosugar derivative, e.g. GalNAc or GlcNAc, A is preferably selected from the group consisting of an azido group, halogen, a thiol group or a derivative thereof and a sulfonyloxy group, more preferably from the group consisting of an azido group, halogen, a thiol group and a sulfonyloxy group.

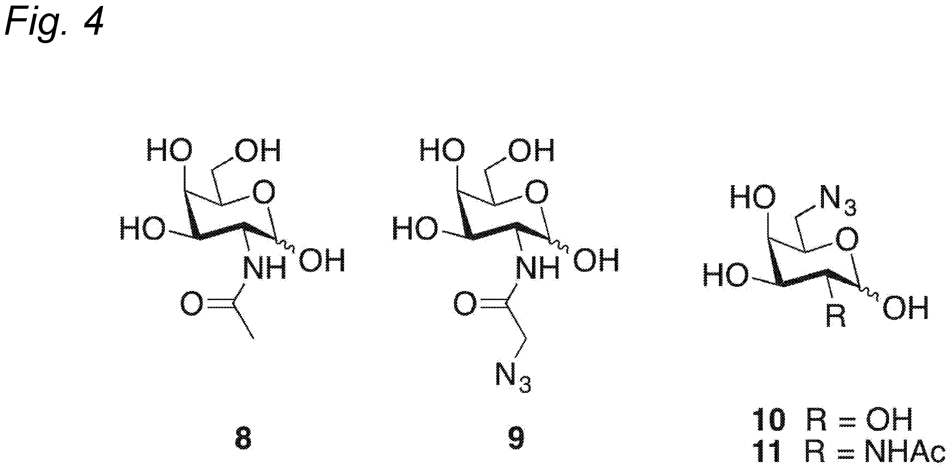

When A is an azido group, it is preferred that A is bonded to C2, C3, C4 or C6. As was described above, the one or more azide substituents in Su(A)ₓ may be bonded to C2, C3, C4 or C6 of the sugar or sugar derivative S, instead of a hydroxyl (OH) group or, in case of 6-azidofucose (6-AzFuc), instead of a hydrogen atom. Alternatively or additionally, the N-acetyl substituent of an amino sugar derivative may be substituted by an azidoacetyl substituent. In a preferred embodiment Su(A)ₓ is selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz), more preferably from the group consisting of GalNAz, 6-AzGal, 4-AzGalNAc, GlcNAz and 6-AzGlcNAc. Examples of Su(A)ₓ-P wherein A is an azido group are graphically depicted in Figure 4 (compounds **9-11**) and shown below.

When A is a keto group, it is preferred that A is bonded to C2 instead of the OH-group of Su. Alternatively A may be bonded to the N-atom of an amino sugar derivative, preferably a 2-amino sugar derivative. The sugar derivative then comprises an -NC(O)R⁶ substituent. R⁶ is preferably a C₂ - C₂₄ alkyl group, optionally substituted. More preferably, R⁶ is an ethyl group. In a preferred embodiment Su(A)ₓ is selected from the group consisting of 2-deoxy-(2-oxopropyl)galactose (2-ketoGal), 2-N-propionylgalactosamine (2-N-propionylGalNAc), 2-N-(4-oxopentanoyl)galactosamine (2-N-LevGal) and 2-N-butyrylgalactosamine (2-N-butyrylGalNAc), more preferably 2-ketoGalNAc and 2-N-propionylGalNAc. Examples of Su(A)ₓ-P wherein A is a keto group are shown below.

When A is an alkynyl group, preferably a terminal alkynyl group or a (hetero)cycloalkynyl group, it is preferred that said alkynyl group is present on a 2-amino sugar derivative. An example of Su(A)x wherein A is an alkynyl group is 2-(but-3-yonic acid amido)-2-deoxy-galactose. An example of Su(A)ₓ-P wherein A is an alkynyl group is shown below.

When A is a thiol group, it is preferred that said thiol group is present on the 6-position of a sugar derivative or on a 2-amino sugar derivative. An example of Su(A)ₓ wherein A is a thiol group is 2-(mercaptoacetamido)-2-deoxy-galactose. Another example of Su(A)ₓ wherein A is a thiol group is 6-mercapto-6-deoxy-galactose.

When A is a halogen, it is preferred that said halogen is present on the 6-position of a sugar derivative or on a 2-amino sugar derivative. An example of Su(A)ₓ wherein A is a halogen is 2-(chloroacetamido)-2-deoxy-galactose. Another example of Su(A)ₓ wherein A is a halogen is 6-iodo-6-deoxy-galactose. Another example of Su(A)ₓ wherein A is a halogen is 6-(chloroacetamido)-6-deoxy-galactose.

When A is a sulfonyloxy group, it is preferred that said sulfonyloxy group is present on the 6-position of a sugar derivative or on a 2-amino sugar derivative. An example of Su(A)ₓ wherein A is a sulfonyloxy group is 2-(methylsulfonyloxyacetamido)-2-deoxy-galactose (2-GalNAcOMs). Another example of SuAₓ wherein A is a sulfonyloxy group is 2-(benzenesulfonyloxyacetamido)-2-deoxy-galactose (2-GalNAcOMs). Another example of Su(A)ₓ wherein A is a sulfonyloxy group is 6-(methylsulfonyl)-galactose.

When A is a halogenated acetamido group, a mercaptoacetamido group or a sulfonylated hydroxyacetamido group it is preferred that said groups are present on the 6-position of a sugar derivative.

P is herein defined as a nucleotide. P is preferably selected from the group consisting of a nucleoside monophosphate and a nucleoside diphosphate, more preferably from the group consisting of uridine diphosphate (UDP), guanosine diphosphate (GDP), thymidine diphosphate (TDP), cytidine diphosphate (CDP) and cytidine monophosphate (CMP), more preferably from the group consisting of uridine diphosphate (UDP), guanosine diphosphate (GDP), cytidine diphosphate and (CDP). Most preferably, P is UDP.

Several compounds of the formula Su(A)ₓ-P, wherein a nucleoside monophosphate or a nucleoside diphosphate P is linked to a sugar derivative Su(A)ₓ, are known in the art. For example Wang et al., Chem. Eur. J. 2010, 16, 13343-13345, Piller et al., ACS Chem. Biol. 2012, 7, 753, Piller et al., Bioorg. Med. Chem. Lett. 2005, 15, 5459-5462 and WO 2009/102820 disclose a number of compounds Su(A)ₓ-P and their syntheses.

Several examples (**9 - 11**) and (**12 - 27**) of azido-, keto-, alkynyl-, halogen, thiol, thiolated acetamido- and halogenated acetamido-substitued sugars and sugar derivatives are shown below, all of which may be converted into their corresponding UDP sugars Su(A)ₓ-UDP (**9b - 11b**) and (**12b - 27b**).

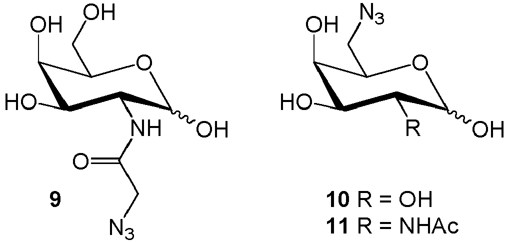

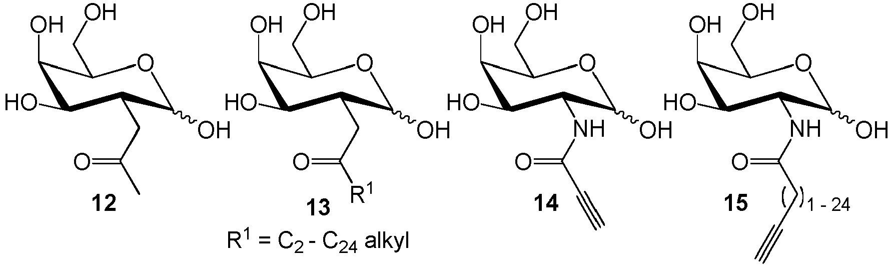

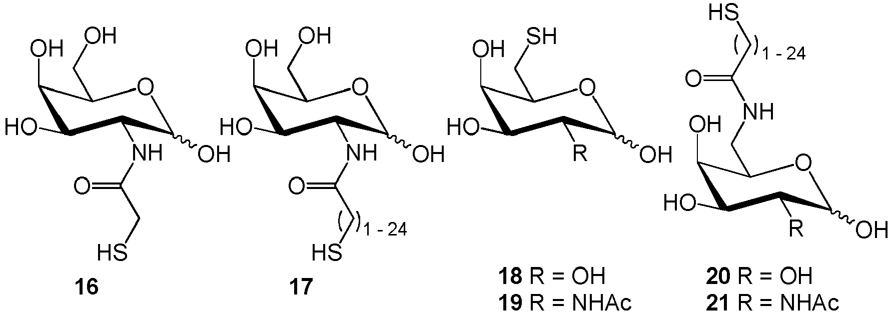

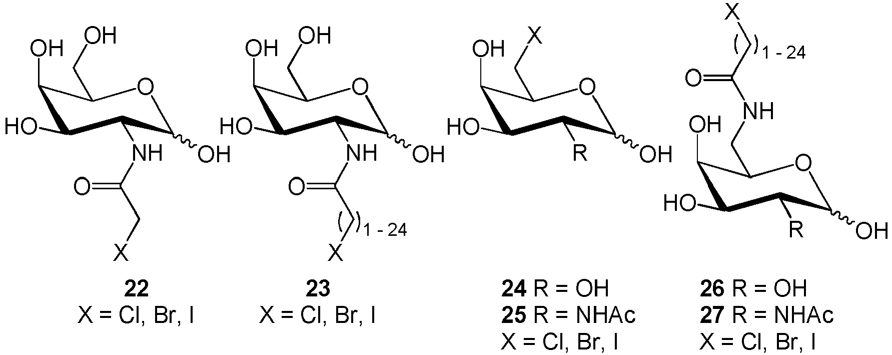

Preferably, Su(A)ₓ-P is selected from the group consisting of GalNAz-UDP(**9b**), 6-AzGal-UDP (**10b**), 6-AzGalNAc-UDP (**11b**), 4-AzGalNAz-UDP, 6-AzGalNAz-UDP, 6-AzGlc-UDP, 6-AzGlcNAz-UDP, 2-ketoGal-UDP (**12b**), 2-N-propionylGalNAc-UDP **(13b,** wherein R¹ is ethyl) and 2-(but-3-yonic acid amido)-2-deoxy-galactose-UDP (**15b**, with n=1). More preferably, Su(A)ₓ-P is GalNAz-UDP (**9b**) or 6-AzGalNAc-UDP (**11b**).

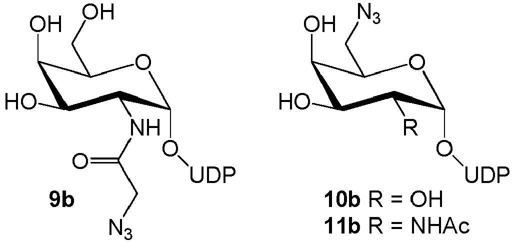

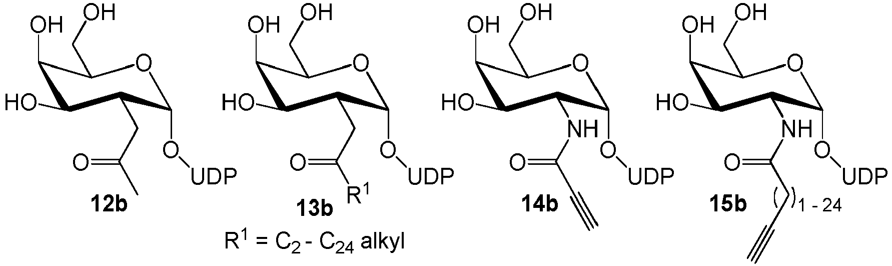

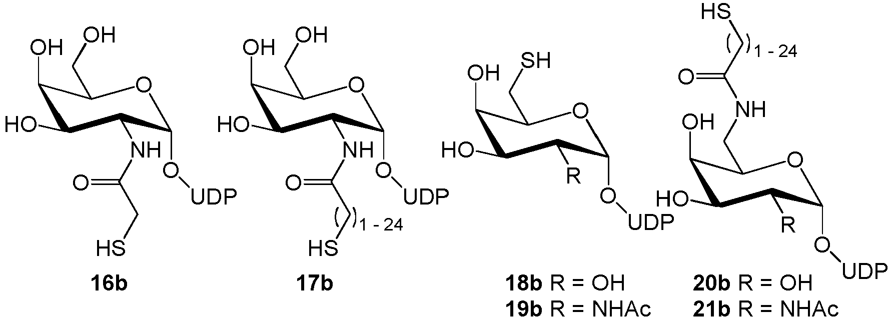

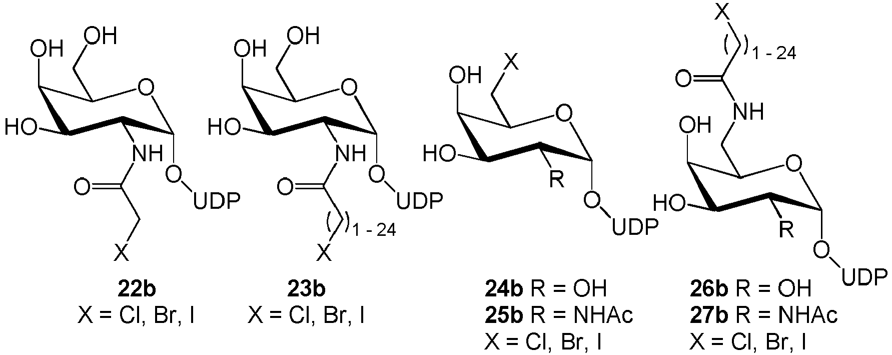

The syntheses of GalNAz-UDP (**9b**) and 6-AzGalNAc-UDP (**11b**) are disclosed in Piller et al., Bioorg. Med. Chem. Lett. 2005, 15, 5459-5462 and Wang et al., Chem. Eur. J. 2010, 16, 13343-13345.

The synthesis of 2-ketoGal-UDP (**12b**) is disclosed in Qasba et al., J. Am. Chem. Soc. 2003, 125, 16162, in particular in the Supporting Information.

The synthesis of 2-(but-3-yonic acid amido)-2-deoxy-galactose-UDP (**15b**) is disclosed in WO 2009/102820.

Further examples of Su(A)ₓ-P include 6-A-6-deoxygalactose-UDP (6-A-Gal-UDP), such as 6-chloro-6-deoxygalactose-UDP (6-ClGal-UDP, (**24b**) with X is Cl), 6-thio-6-deoxygalactose-UDP (6-HSGal-UDP, (**18b**)) or 2-A-2-deoxygalactose-UDP (2-A-Gal-UDP), such as 2-chloro-2-deoxygalactose-UDP (2-ClGal-UDP), 2-thio-2-deoxygalactose-UDP (2-HSGal-UDP). Alternatively, A may be indirectly substituted to the sugar derivative as part of an acetamido group that in turn is substituting a hydroxyl group. Examples include 6-A-acetamido-6-deoxygalactose-UDP (6-GalNAcA-UDP), such as 6-chloroacetamido-6-deoxygalactose-UDP (6-GalNAcCl-UDP, (**26b**) with X is Cl), 6-thioacetamido-6-deoxygalactose-UDP (6-GalNAcSH-UDP, (**20b**)) or 2-A-acetamido-2-deoxygalactose-UDP (2-GalNAcA-UDP), such as 2-chloroacetamido-2-deoxygalactose-UDP (2-GalNAcCl-UDP, (**22b**) with X is Cl), 2-thioacetamido-2-deoxygalactose-UDP (2-GalNAcSH-UDP, (**16b**)) or acetylated 2-thioacetamido-2-deoxygalactose-UDP (2-GalNAcSAc-UDP). Alternatively, A may be indirectly substituted to the sugar derivative as part of another functional group that in turn is substituting a hydroxyl group or is attached to a hydroxyl group. Examples of such other functional group include an (hetero)alkyl chain or a (hetero)aryl chain.

Preferably, Su(A)ₓ-P is selected from the group consisting of GalNAz-UDP (**9b**), 6-AzGalNAc-UDP (**11b**), 6-GalNAcCl-UDP ((**26b**) with X is Cl), 6-GalNAcSH-UDP (**20b**), 2-GalNAcCl-UDP ((**22b**) with X is Cl), 2-GalNAcSH-UDP (**16b**), 6-ClGal-UDP ((**24b**) with X is Cl), 2-ClGal-UDP, 2-HSGal-UDP and 6-HSGal-UDP (**18b**).

More preferably, Su(A)ₓ-P is selected from the group consisting of GalNAz-UDP (**9b**), 6-AzGalNAc-UDP (**11b**), 6-GalNAcCl-UDP ((**26b**) with X is Cl), 6-GalNAcSH-UDP (**20b**), 2-GalNAcCl-UDP ((**22b**) with X is Cl), 2-GalNAcSH-UDP (**16b**), 6-ClGal-UDP ((**24b**) with X is Cl) and 2-ClGal-UDP.

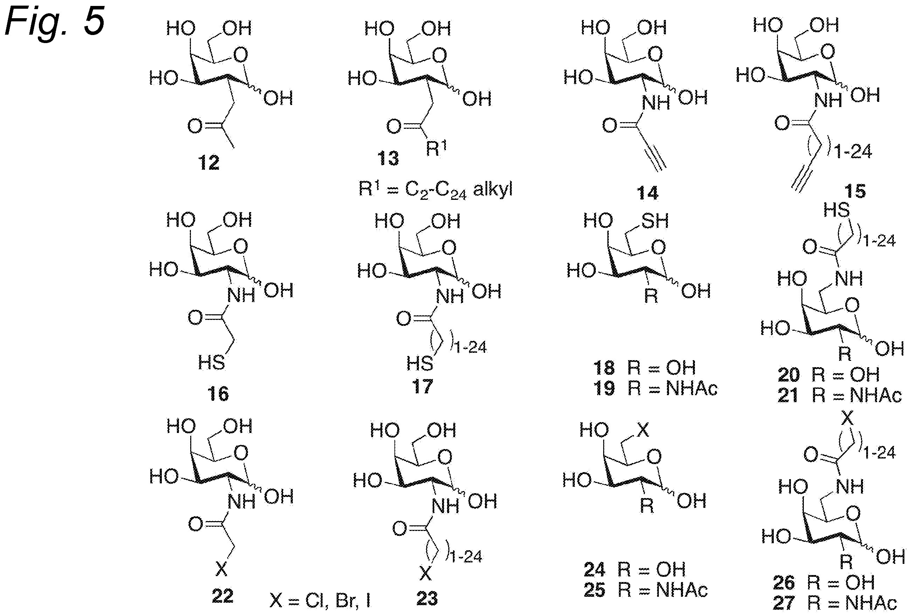

Additional examples of sugars and sugar derivatives are shown in Figures 4 and 5. Figure 4 shows the structures of azido-modified galactose derivatives (**9-11**), for which the corresponding UDP sugar may be used for transfer onto a GlcNAc-terminated sugar under the action of a galactosyl transferase (or a mutant thereof). Figure 5 shows the structures of other galactose derivatives (**12-27**), for which the corresponding UDP sugar may be used for transfer onto a GlcNAc-terminated sugar under the action of a galactosyl transferase (or a mutant thereof).

Several of the sugar derivative nucleotides Su(A)ₓ-P that may be employed in the process for the preparation of a modified antibody according to the invention are a substrate for a wild type galactosyltransferase. For these sugar derivative nucleotides Su(A)ₓ-P, the process according to the invention may be performed in the presence of a wild type galactosyltransferase, preferably a wild type *β*(1,4)-galactosyltransferase, more preferably a wild type *β*(1,4)-galactosyltransferase I, as a catalyst. More preferably, the wild-type a *β*(1,4)-galactosyltransferase is a wild-type human GalT, more preferably a wild-type human GalT selected from the group consisting of a wild-type human *β*4-Gal-T1, a wild-type human *β*(1,4)-Gal-T2, a wild-type human *β*(1,4)-Gal-T3 and a wild-type human *β*(1,4)-Gal-T4.

When a wild type galactosyltransferase is used as a catalyst, it is preferred that Su(A)ₓ-P is selected from the group consisting of Su(A)ₓ-P wherein x is 1 and wherein A is present on C2 or C6, more preferably C6, of the sugar derivative, and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group. A may be directly substituted to the sugar derivative instead of an hydroxyl group. Examples include 6-A-6-deoxygalactose-UDP (6-A-Gal-UDP), such as 6-azido-6-deoxygalactose-UDP (6-AzGal-UDP, (**10b**)), 6-chloro-6-deoxygalactose-UDP (6-ClGal-UDP, (**24b**) with X is Cl), 6-thio-6-deoxygalactose-UDP (6-HSGal-UDP, (**18b**)) or 2-A-2-deoxygalactose-UDP (2-A-Gal-UDP), such as 2-azido-2-deoxygalactose-UDP (2-AzGal-UDP), 2-chloro-2-deoxygalactose-UDP (2-ClGal-UDP), 2-thio-2-deoxygalactose-UDP (2-HSGal-UDP). Alternatively, A may be indirectly substituted to the sugar derivative as part of an acetamido group that in turn is substituting a hydroxyl group. Examples include 6-A-acetamido-6-deoxygalactose-UDP (6-GalNAcA-UDP), such as 6-azidoacetamido-6-deoxygalactose-UDP (6-GalNAcN₃-UDP), 6-chloroacetamido-6-deoxygalactose-UDP (6-GalNAcCl-UDP, (**26b**) with X is Cl), 6-thioacetamido-6-deoxygalactose-UDP (6-GalNAcSH-UDP, (**20b**)) or 2-A-acetamido-2-deoxygalactose-UDP (2-GalNAcA-UDP), such as 2-azidoacetamido-2-deoxygalactose-UDP (2-GalNAcN₃-UDP, (**9b**)), 2-chloroacetamido-2-deoxygalactose-UDP (2-GalNAcCl-UDP, (**22b**)), 2-thioacetamido-2-deoxygalactose-UDP (2-GalNAcSH-UDP, (**16b**)). Alternatively, A may be indirectly substituted to the sugar derivative as part of another functional group that in turn is substituting a hydroxyl group or is attached to a hydroxyl group. Examples of such other functional group include an (hetero)alkyl chain or a (hetero)aryl chain.

In a particularly preferred embodiment of the process for the preparation of a modified antibody according to the invention, Su(A)ₓ-P is selected from the group consisting of GalNAz-UDP (**9b**), 6-AzGalNAc-UDP (**11b**), 2-GalNAcSH-UDP (**16b**), 2-GalNAcX-UDP (**22b**), 2-GalNAcOS(O)₂R⁸-UDP, 6-GalNAcSH-UDP (**20b**), 6-GalNAcX-UDP (**26b**) and 6-GalNAcOS(O)₂R⁸-UDP, and the catalyst is bovine β(1,4)-Gal-T1 comprising a mutant catalytic domain GalT (Y289L); wherein X is Cl, Br or I; and wherein R⁸ is a methyl group, an ethyl group, a phenyl group or a p-tolyl group.

In a further preferred embodiment 2-GalNAcX-UDP is 2-GalNAcCl-UDP or 2-GalNAcBr-UDP, more preferably 2-GalNAcCl-UDP, and 6-GalNAcX-UDP is 6-GalNAcCl-UDP or 6-GalNAcBr-UDP, more preferably 6-GalNAcCl-UDP. In another preferred embodiment, R⁸ in 2-GalNAcOS(O)₂R⁸-UDP is methyl, phenyl or p-tolyl, most preferably methyl, and R⁸ in 6-GalNAcOS(O)₂R⁸-UDP is methyl, phenyl or p-tolyl, most preferably R⁸ is methyl.
In another particularly preferred embodiment of the process for the preparation of a modified antibody according to the invention, Su(A)ₓ-P is selected from the group consisting of 6-AzGalNAc-UDP (**11b**), 6-HSGal-UDP (**18b**), 6-XGal-UDP (**24b**), 6-R⁸S(O)₂OGal-UDP, and the catalyst is a wild-type human GalT; wherein X is Cl, Br or I; and wherein R⁸ is a methyl group, an ethyl group, a phenyl group or a p-tolyl group. X is more preferably Cl or Br, most preferably Cl. R⁸ is more preferably methyl, phenyl or p-tolyl, most preferably methyl. The human GalT is preferably a human *β*4-Gal-T1, a human *β*(1,4)-Gal-T2, a human *β*(1,4)-Gal-T3 and a human *β*(1,4)-Gal-T4.

As was described above, in the process for the modification of a antibody according to the invention, Su(A)ₓ-P may be any sugar derivative nucleotide that is a substrate for a suitable galactosyltransferase catalyst.

In a preferred embodiment of the process for the preparation of an glycoprotein-conjugate, Su(A)ₓ comprises 1 or 2 functional groups A, i.e. preferably x is 1 or 2. More preferably, x is 1. In another preferred embodiment, Su is galactose (Gal). In a further preferred embodiment, x is 1 or 2 and Su is Gal, and most preferably, x is 1 and Su is Gal. In these preferred embodiments it is further preferred that the linker-conjugate comprises 1 or 2, and most preferably 1, molecules of interest.

In a preferred embodiment, Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably form the group consisting of GalNAz, 6-AzGalNAc, 6-GalNAcCl, 6-GalNAcSH, 2-GalNAcCl, 2-GalNAcSH, 6-ClGal-and 2-ClGal. In these preferred embodiments it is further preferred that the linker-conjugate comprises 1 or 2, and most preferably 1, molecules of interest.

In a further preferred embodiment, x is 1 and Su(A)ₓ is selected from the group consisting of GalNAz, 6-AzGalNAc, 6-GalNAcCl, 6-GalNAcSH, 2-GalNAcCl, 2-GalNAcSH, 6-ClGal, 2-ClGal, 2-HSGal and 6-HSGal, more preferably from the group consisting of GalNAz, 6-AzGalNAc, 6-GalNAcCl, 6-GalNAcSH, 2-GalNAcCl, 2-GalNAcSH, 6-ClGal-and 2-ClGal. In these preferred embodiments it is further preferred that the linker-conjugate comprises 1 or 2, and most preferably 1, molecules of interest.

In a preferred embodiment wherein A is an azide group, Su(A)ₓ is preferably selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). In a further preferred embodiment Su(A)ₓ is selected from the group consisting of GalNAz, 6-AzGal, 4-AzGalNAc, GlcNAz and 6-AzGlcNAc. More preferably, x is 1 and Su(A)ₓ is selected from the group consisting of 2-azidoacetamidogalactose (GalNAz), 6-azido-6-deoxygalactose (6-AzGal), 6-azido-6-deoxy-2-acetamidogalactose (6-AzGalNAc), 4-azido-4-deoxy-2-acetamidogalactose (4-AzGalNAc), 6-azido-6-deoxy-2-azidoacetamidogalactose (6-AzGalNAz), 2-azidoacetamidoglucose (GlcNAz), 6-azido-6-deoxyglucose (6-AzGlc), 6-azido-6-deoxy-2-acetamidoglucose (6-AzGlcNAc), 4-azido-4-deoxy-2-acetamidoglucose (4-AzGlcNAc) and 6-azido-6-deoxy-2-azidoacetamidoglucose (6-AzGlcNAz). More preferably, x is 1 and Su(A)ₓ is selected from the group consisting of GalNAz, 6-AzGal, 4-AzGalNAc, GlcNAz and 6-AzGlcNAc. In these preferred embodiments it is further preferred that the linker-conjugate comprises 1 or 2, and most preferably 1, molecules of interest.

In a particularly preferred embodiment of the modified antibody according to the invention, Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In an even more preferred embodiment, x is 1 or 2 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In a most preferred embodiment, x is 1 and Su(A)ₓ is selected from the group consisting of GalNAz, 6-AzGalNAc, 2-GalNAcSH, 2-GalNAcX, 2-GalNAcOS(O)₂R⁸, 6-GalNAcSH, 6-GalNAcX and 6-GalNAcOS(O)₂R⁸. In these preferred embodiments it is further preferred that the linker-conjugate comprises 1 or 2, and most preferably 1, molecules of interest.

The sugar derivative Su(A)ₓ in the proximal N-linked Su(A)ₓGlcNAc-substituent attached to an N-glycosylation site of the IgG antibody may for example be bonded to C4 of the GlcNAc-moiety via a *β*(1,4)-glycosidic bond or to C3 of said GlcNAc-moiety via an *α*(1,3)-glycosidic bond. The proximal N-linked GlcNAc-residue of the Su(A)ₓGlcNAc-substituent is bonded via C1 to the protein or antibody via an N-glycosidic bond, preferably to the amide nitrogen atom in the side chain of an asparagine amino acid of the protein or antibody. The proximal N-linked GlcNAc-residue in said Su(A)ₓGlcNAc-substituent is optionally fucosylated. Whether the sugar derivative Su(A)ₓ in the Su(A)ₓGlcNAc-moiety attached to the antibody is bonded to C4 of said GlcNAc-residue via a *β*(1,4)-glycosidic bond or to C3 of said GlcNAc-moiety via an *α*(1,3)-glycosidic bond depends on the catalyst that was used in step (4) and/or step (5b) of the process according to the invention. When said step is performed in the presence of a *β*(1,4)-galactosyltransferase then binding occurs via C1 of Su(A)ₓ and C4 of the proximal GlcNAc-residue via a *β*(1,4)-glycosidic bond. When the process is performed in the presence of a *α*(1,3)-galactosyltransferase then binding occurs via C1 of Su(A)ₓ and C3 of the proximal GlcNAc-residue via an *α*(1,3)-glycosidic bond.

When A is an azido functional group, the IgG antibody comprising a Su(A)ₓGlcNAc-residue that is obtained via step (4) or (5b) of the process according to the invention is referred to as an azido-modified antibody. When A is a keto functional group, the IgG antibody comprising a Su(A)ₓGlcNAc-residue is referred to as a keto-modified antibody. When A is an alkynyl functional group, IgG antibody comprising a Su(A)ₓGlcNAc-residue is referred to as an alkyne-modified antibody. When A is a thiol group, the IgG antibody comprising a Su(A)ₓGlcNAc-residue is referred to as a thiol-modified antibody. When A is an halogen group, the IgG antibody comprising a Su(A)ₓGlcNAc-residue is referred to as a halogen-modified antibody. When A is an sulfonyloxy group, the IgG antibody comprising a Su(A)ₓGlcNAc-residue is referred to as a sulfonyloxy-modified antibody. When A is a mercaptoacetamido group, the IgG antibody comprising a Su(A)ₓGlcNAc-residue is referred to as a thiolated acetamido-modified antibody. When A is a halogenated acetamido group, the IgG antibody comprising a Su(A)ₓGlcNAc-residue is referred to as a halogenated acetamido-modified antibody. When A is a sulfonylated hydroxyacetamido group, the IgG antibody comprising a Su(A)ₓGlcNAc-residue is referred to as mercaptoacetamido-modified antibody.

Step (4) of the process according to the invention, the attachment of a monosaccharide derivative Su(A)ₓ to a proximal N-linked GlcNAc-residue, is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer.

The process is preferably performed at a temperature in the range of about 4 to about 50°C, more preferably in the range of about 10 to about 45°C, even more preferably in the range of about 20 to about 40°C, and most preferably in the range of about 30 to about 37°C.

The process is preferably performed a pH in the range of about 5 to about 9, preferably in the range of about 5.5 to about 8.5, more preferably in the range of about 6 to about 8. Most preferably, the process is performed at a pH in the range of about 7 to about 8.

### Step (5)

Step (5) of the process according to the invention is an optional step. As was described above, the process comprises one of steps (3), (5) and (7) are both absent from the process.

Step (5) of the process for the preparation of an antibody-conjugate comprises the steps of:
(5a) repeating step (2), in order to trim an oligosaccharide (N-glycan) that is attached to a different glycosylation site than the oligosaccharide that was trimmed during the first time that step (2) was performed, in order to obtain a proximal N-linked GlcNAc-residue, and
(5b) repeating step (4), in order to attach a monosaccharide derivative Su(A)ₓ to the proximal N-linked GlcNAc-residue that was obtained in step (5a), in order to obtain a proximal N-linked Su(A)ₓGlcNAc-substituent.

The description of the details of step (2), and the preferred embodiments thereof, also hold for step (5a), and the description of the details of step (4), and the preferred embodiments thereof, also hold for step (5b).

In step (5a), also an endo-β-N-acetylglucosaminidase is preferred as the enzyme, but in this step the endo-β-N-acetylglucosaminidase is preferably selected from the group consisting of Endo F1, Endo F2, Endo F3, Endo H and Endo M, Endo A and any combination thereof.

A preferred embodiment of a process for the preparation of an antibody-conjugate comprising step (5) is described in more detail below.

### Step (6)

Step (6) of the process for the preparation of an antibody-conjugate according to the invention comprises reacting the proximal N-linked GlcNAc-Su(A)ₓ substituent that was obtained in step (4) or (5b) with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, provided that A is not a thiol group precursor.

In step (6), an antibody-conjugate according to the invention is obtained. An antibody-conjugate is herein defined as an antibody that is conjugated to a molecule of interest D via a linker L. The antibody-conjugate according to the invention may be conjugated to one or to more than one molecule of interest D via said linker L.

A molecule of interest may for example be a reporter molecule, a diagnostic agent, an active substance, an enzyme, an amino acid (including an unnatural amino acid), a (non-catalytic) protein, a peptide, a polypeptide, an oligonucleotide, a glycan, a (poly)ethylene glycol diamine (e.g. 1,8-diamino-3,6-dioxaoctane or equivalents comprising longer ethylene glycol chains), a polyethylene glycol chain, a polyethylene oxide chain, a polypropylene glycol chain, a polypropylene oxide chain, 1,x-diaminoalkane (wherein x is the number of carbon atoms in the alkane), an azide or a (hetero)cycloalkynyl moiety, preferably a bivalent or bifunctional (hetero)cycloalkynyl moiety. In a preferred embodiment, the molecule of interest is selected from the group consisting of an amino acid (in particular lysine), an active substance, a reporter molecule, an azide and a (hetero)cycloalkynyl moiety.

An active substance is a pharmacological and/or biological substance, i.e. a substance that is biologically and/or pharmaceutically active, for example a drug or a prodrug, a diagnostic agent, an amino acid, a protein, a peptide, a polypeptide, a glycan, a lipid, a vitamin, a steroid, a nucleotide, a nucleoside, a polynucleotide, RNA or DNA. Examples of suitable peptide tags include a cell-penetrating peptides like human lactoferrin or polyarginine. An example of a suitable glycan is oligomannose.

In a preferred embodiment, the active substance is selected from the group consisting of drugs and prodrugs. More preferably, the active substance is selected from the group consisting of pharmaceutically active compounds, in particular low to medium molecular weight compounds (e.g. about 200 to about 1500 Da, preferably about 300 to about 1000 Da), such as for example cytotoxins, antiviral agents, antibacterial agents, peptides and oligonucleotides. Examples of cytotoxins include colchicine, vinca alkaloids, camptothecins, doxorubicin, daunorubicin, taxanes, calicheamycins, tubulysins, irinotecans, an inhibitory peptide, amanitin, deBouganin, duocarmycins, maytansines, auristatins or pyrrolobenzodiazepines (PBDs), preferred examples include camptothecins, doxorubicin, daunorubicin, taxanes, calicheamycins, duocarmycins, maytansines, auristatins or pyrrolobenzodiazepines (PBDs).

A reporter molecule is a molecule whose presence is readily detected, for example a diagnostic agent, a dye, a fluorophore, a radioactive isotope label, a contrast agent, a magnetic resonance imaging agent or a mass label. Examples of a fluorophore include all kinds of Alexa Fluor (e.g. Alexa Fluor 555), cyanine dyes (e.g. Cy3 or Cy5), coumarin derivatives, fluorescein, rhodamine, allophycocyanin and chromomycin.

Examples of radioactive isotope label include ^{99m}Tc, ¹¹¹In, ¹⁸F, ¹⁴C, ⁶⁴Cu, ¹³¹I or ¹²³I, which may or may not be connected via a chelating moiety such as DTPA, DOTA, NOTA or HYNIC.

In the antibody-conjugate according to the invention, the molecule of interest D is conjugated to the antibody via a linker L. Linkers or linking units are well known in the art, and are described in more detail below.

In a preferred embodiment, step (6) comprises reacting an IgG antibody comprising at least two N-linked glycosylation sites on the combination of a single heavy chain and single light chain, wherein one or more Su(A)ₓGlcNAc-substituents are attached to an N-linked glycosylation site, with a linker-conjugate, wherein said linker-conjugate comprises a functional group B and one or more molecules of interest D, wherein said functional group B is a functional group that is capable of reacting with a functional group A of a Su(A)ₓGlcNAc-substituent on said antibody, and wherein Su(A)ₓ is a sugar derivative comprising x functional groups A wherein x is 1, 2, 3 or 4 and A is independently selected from the group consisting of an azido group, a keto group, an alkynyl group, a thiol group, a halogen, a sulfonyloxy group, a halogenated acetamido group, a mercaptoacetamido group and a sulfonylated hydroxyacetamido group.

The linker-conjugate preferably is of the formula B-L(D)ᵣ, wherein D is a molecule of interest as defined above, and B and L are as defined below, and r is 1 - 20. Preferably r is 1 - 10, more preferably r is 1 - 8, even more preferably r is 1, 2, 3, 4, 5 or 6, even more preferably r is 1, 2, 3 or 4, even more preferably r is 1 or 2, and most preferably r is 1.

Complementary functional groups B for the functional group A on the modified antibody (A is an azido group, a keto group, an alkynyl group, a thiol group, a halogen, a sulfonyloxygroup, a halogenated acetamido group, a mercaptoacetamido group or a sulfonylated hydroxyacetamido group) are known in the art.

When A is an azido group, linking of the azide-modified antibody and the linker-conjugate preferably takes place via a cycloaddition reaction. Functional group B is then preferably selected from the group consisting of alkynyl groups, preferably terminal alkynyl groups, and (hetero)cycloalkynyl groups.

When A is a keto group, linking of the keto-modified antibody with the linker-conjugate preferably takes place via selective conjugation with hydroxylamine derivatives or hydrazines, resulting in respectively oximes or hydrazones. Functional group B is then preferably a primary amino group, e.g. an -NH₂ group, an aminooxy group, e.g. -O-NH₂, or a hydrazinyl group, e.g. -N(H)NH₂. The linker-conjugate is then preferably H₂N-L(D)ᵣ, H₂N-O-L(D)ᵣ or H₂N-N(H)-L(D)ᵣ respectively, wherein L, D and r are as defined above.

When A is an alkynyl group, linking of the alkyne-modified antibody with the linker-conjugate preferably takes place via a cycloaddition reaction, preferably a 1,3-dipolar cycoaddition. Functional group B is then preferably a 1,3-dipole, such as an azide, a nitrone or a nitrile oxide. The linker-conjugate is then preferably N₃-L(D)ᵣ, wherein L, D and r are as defined above.

When A is a thiol group, linking of the thiol-modified antibody with the linker-conjugate preferably takes place via a Michael-type addition reaction. Functional group B is then preferably an N-maleimidyl group or a halogenated acetamido group. The linker-conjugate is then preferably X-CH₂C(O)NHL(D)ᵣ or X-CH₂C(O)N[L(D)ᵣ]₂ wherein X is F, Cl, Br or I, or a maleimide-linker-conjugate (139) as illustrated below.

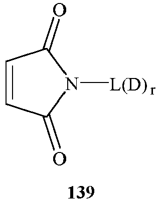

When A is a halogen-modified antibody, a halogenated acetamide-modified antibody, a sulfonyloxy-modified antibody or a mercaptoacetamide-modified antibody, linking of the modified antibody with the linker-conjugate preferably takes place via reaction with a thiol to form a thioether. Functional group B comprises then preferably a thiol group, and a preferred linker-conjugate is HS-L(D)ᵣ. However, functional group B may also comprise an alcohol group or an amine group. When A is a halogen, a halogenated acetamido group, a sulfonyloxy group or a mercaptoacetamido group, linking of the modified antibody with the linker-conjugate preferably takes place via reaction with a thiol to form a thioether. Functional group B comprises then preferably a thiol group, and a preferred linker-conjugate is HS-L(D)ᵣ. However, functional group B may also comprise an alcohol group or an amine group. In other words, when the modified antibody is a halogen-modified antibody, a halogenated acetamide-modified antibody, a sulfonyloxy-modified antibody or a mercaptoacetamide-modified antibody, linking of the modified antibody with the linker-conjugate preferably takes place via reaction with a thiol to form a thioether. Functional group B comprises then preferably a thiol group, and a preferred linker-conjugate is HS-L(D)ᵣ. However, functional group B may also comprise an alcohol group or an amine group.

A preferred embodiment of step (6) comprises reacting the modified antibody with a linker-conjugate, wherein:
(a) when A is an azido group, the linker-conjugate comprises a (hetero)cycloalkynyl group or an alkynyl group, and one or more molecules of interest; or
(b) when A is a keto group, the linker-conjugate comprises a primary amine group, an aminooxy group or a hydrazinyl group, and one or more molecules of interest; or
(c) when A is an alkynyl group, the linker-conjugate comprises an azido group, a nitrone or a nitrile oxide, and one or more molecules of interest.
(d) when A is a thiol group or a mercaptoacetamide group, the linker-conjugate comprises an N-maleimide group or a halogenated acetamido group, and one or more molecules of interest; or
(e) when A is a halogen, a halogenated acetamido group, a sulfonyloxy group or a sulfonylated hydroxyacetamido group, the linker-conjugate comprises a thiol group, and one or more molecules of interest.

When said modified antibody is a halogen-modified antibody and functional group B comprises a thiol group, said thiol group may be an aliphatic or an aromatic thiol group. In a preferred embodiment said thiol group is an aromatic thiol group.

In a preferred embodiment, the modified antibody is a thiol-modified antibody and functional group B comprises an N-maleimide group or a halogenated acetamido group.

In a preferred embodiment of step (6) of the process for the preparation of an antibody-conjugate according to the invention, linker-conjugate B-L(D)ᵣ is selected from the group consisting of linker-conjugates of formula (140a), (140b), (141), (142), (143) or (144): 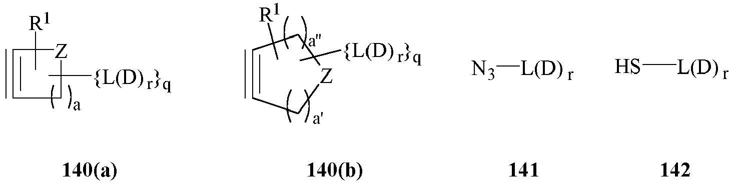 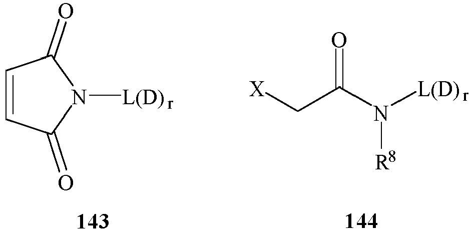 wherein:
L is a linker;
D is a molecule of interest;
r is 1 - 20;
R¹ is independently selected from the group consisting of hydrogen, halogen, - OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
q is 0 or 1, with the proviso that if q is 0 then Z is N-L(D)ᵣ;
a is 1, 2, 3, 4, 5, 6, 7, 8, 9 or 10;
a' is 0, 1, 2, 3, 4, 5, 6, 7 or 8;
a" is 0,1, 2, 3, 4, 5, 6, 7 or 8;
a' + a" < 10;
X is F, Cl, Br or I; and
R⁸ is R¹ or -L(D)ᵣ, preferably hydrogen, -L(D)ᵣ or a C₁ - C₂₄ alkyl group, more preferably hydrogen, -L(D)ᵣ or a C₁ - C₆ alkyl group, even more preferably hydrogen, -L(D)ᵣ or a C₁, C₂, C₃ or C₄ alkyl group, most preferably hydrogen, methyl, ethyl, linear or branched C3 or C4 alkyl.

In another preferred embodiment of step (6) of the process for the preparation of an antibody-conjugate according to the invention, linker-conjugate B-L(D)ᵣ is selected from the group consisting of linker-conjugates of formula **(140a), (141), (142), (143)** or **(144),** as defined above.

In a preferred embodiment, the modified antibody according to the invention is an azide-modified antibody, an alkyne-modified antibody, a halogen-modified antibody or a thiol-modified antibody.

A suitable linker-conjugate for the preparation of a antibody-conjugate according to the invention is a linker-conjugate comprising a functional group B and a molecule of interest. Linkers L, also referred to as linking units, are well known in the art. In a linker-conjugate as described herein, L is linked to a molecule of interest D as well as to a functional group B, as was described above. Numerous methods for linking said functional group B and said molecule of interest D to L are known in the art. As will be clear to a person skilled in the art, the choice of a suitable method for linking a functional group B to one end and a molecule of interest D to another end of a linker depends on the exact nature of the functional group B, the linker L and the molecule of interest D.

A linker may have the general structure F¹-L(F²)ᵣ, wherein F¹ represents either a functional group B or a functional group that is able to react with a functional group F on the functional group B as described above, e.g. a (hetero)cycloalkynyl group, a terminal alkynyl group, a primary amine, an aminooxy group, a hydrazyl group, an azido group, an N-maleimidyl group, an acetamido group or a thiol group. F² represents a functional group that is able to react with a functional group F on the molecule of interest.

Since more than one molecule of interest may be bonded to a linker, more than one functional group F² may be present on L. As was described above, r is 1 to 20, preferably 1 to 10, more preferably 1 to 8, even more preferably 1, 2, 3, 4, 5 or 6, even more preferably 1, 2, 3 or 4 and most preferably, r is 1 or 2.

L may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S and NR⁵, wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups. Most preferably, the heteroatom is O.

F, F¹ and F² may for example be independently selected from the group consisting of hydrogen, halogen, R⁵, C₄ - C₁₀ (hetero)cycloalkyne groups, -CH=C(R⁵)₂, -C≡CR⁵, -[C(R⁵)₂C(R⁵)₂O]_{q}-R⁵, wherein q is in the range of 1 to 200, -CN, -N₃, - NCX, -XCN, -XR⁵, -N(R⁵)₂, -⁺N(R⁵)₃, -C(X)N(R⁵)₂, -C(R⁵)₂XR⁵, -C(X)R⁵, -C(X)XR⁵, - S(O)R⁵, -S(O)₂R⁵, -S(O)OR⁵, -S(O)₂OR⁵, -S(O)N(R⁵)₂, -S(O)₂N(R⁵)₂, -OS(O)R⁵, -OS( O)₂R⁵, -OS(O)OR⁵, -OS(O)₂OR⁵, -P(O)(R⁵)(OR⁵), -P(O)(OR⁵)₂, -OP()O(OR⁵)₂, -Si(R⁵) ₃, -XC(X)R⁵, -XC(X)XR⁵, -XC(X)N(R⁵)₂, -N(R⁵)C(X)R⁵, -N(R⁵)C(X)XR⁵ and -N(R⁵)C(X)N(R⁵)₂, wherein X is oxygen or sulphur and wherein R⁵ is as defined above.

Examples of suitable linking units include (poly)ethylene glycol diamines (e.g. 1,8-diamino-3,6-dioxaoctane or equivalents comprising longer ethylene glycol chains), polyethylene glycol or polyethylene oxide chains, polypropylene glycol or polypropylene oxide chains and 1,x-diaminoalkanes wherein x is the number of carbon atoms in the alkane.

Another class of suitable linkers comprises cleavable linkers. Cleavable linkers are well known in the art. For example Shabat et al., Soft Matter 2012, 6, 1073 discloses cleavable linkers comprising self-immolative moieties that are released upon a biological trigger, *e.g.* an enzymatic cleavage or an oxidation event. Some examples of suitable cleavable linkers are peptide-linkers that are cleaved upon specific recognition by a protease, e.g. cathepsin, plasmin or metalloproteases, or glycoside-based linkers that are cleaved upon specific recognition by a glycosidase, e.g. glucoronidase, or nitroaromatics that are reduced in oxygen-poor, hypoxic areas.

As was described above, when the modified glycoprotein is an azide-modified glycoprotein, it is preferred that the linker-conjugate is a (hetero)cycloalkyne linker-conjugate. In a further preferred embodiment, the (hetero)cycloalkyne linker-conjugate is of formula **(140a):** 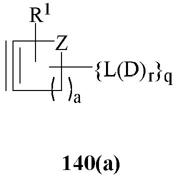 wherein:
L is a linker;
D is a molecule of interest;
r is 1 - 20;
R¹ is independently selected from the group consisting of hydrogen, halogen, - OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
q is 0 or 1, with the proviso that if q is 0 then Z is N-L(D)ᵣ; and
a is 1, 2, 3, 4, 5, 6, 7, 8, 9 or 10.

In a further preferred embodiment, a is 5, i.e. said (hetero)cycloalkynyl group is preferably a (hetero)cyclooctyne group.

In another preferred embodiment, Z is C(R²)₂ or NR². When Z is C(R²)₂ it is preferred that R² is hydrogen. When Z is NR², it is preferred that R² is L(D)ᵣ. In yet another preferred embodiment, r is 1 to 10, more preferably, r is 1, 2, 3, 4, 5 or 6, even more preferably r is 1, 2, 3 or 4, even more preferably r is 1 or 2, and most preferably is 1. In another preferred embodiment, q is 1 or 2, more preferably q is 1. Even more preferably, r is 1 and q is 1, and most preferably, a is 5 and r is 1 and q is 1.

In another further preferred embodiment, when the modified glycoprotein is an azide-modified glycoprotein, the linker-conjugate is a (hetero)cycloalkyne linker-conjugate of formula **(140b):** 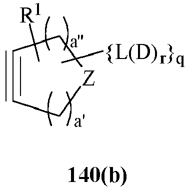 wherein:
L is a linker;
D is a molecule of interest;
r is 1 - 20;
R¹ is independently selected from the group consisting of hydrogen, halogen, - OR⁵, -NO₂, -CN, -S(O)₂R⁵, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹ may be linked together to form an annelated cycloalkyl or an annelated (hetero)arene substituent, and wherein R⁵ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
Z is C(R¹)₂, O, S or NR², wherein R² is R¹ or L(D)ᵣ, and wherein L, D and r are as defined above;
q is 0 or 1, with the proviso that if q is 0 then Z is N-L(D)ᵣ;
a' is 0, 1, 2, 3, 4, 5, 6, 7 or 8;
a" is 0,1, 2, 3, 4, 5, 6, 7 or 8; and
a' + a" < 10.

In a further preferred embodiment, a' + a" is 4, 5, 6 or 7, more preferably a' + a" is 4, 5 or 6 and most preferably a' + a" is 5, i.e. said (hetero)cycloalkynyl group is preferably a (hetero)cyclooctyne group.

In another preferred embodiment, Z is C(R²)₂ or NR². When Z is C(R²)₂ it is preferred that R² is hydrogen. When Z is NR², it is preferred that R² is L(D)ᵣ. In yet another preferred embodiment, r is 1 to 10, more preferably, r is 1, 2, 3, 4, 5 or 6, even more preferably r is 1, 2, 3 or 4, even more preferably r is 1 or 2, and most preferably is 1. In another preferred embodiment, q is 1 or 2, more preferably q is 1. Even more preferably, r is 1 and q is 1, and most preferably, a' + a" is 5 and r is 1 and q is 1.

The L(D)ᵣ substituent may be present on a C-atom in said (hetero)cycloalkynyl group, or, in case of a heterocycloalkynyl group, on the heteroatom of said heterocycloalkynyl group. When the (hetero)cycloalkynyl group comprises substituents, e.g. an annelated cycloalkyl, the L(D)ᵣ substituent may also be present on said substituents.

The methods to connect a linker L to a (hetero)cycloalkynyl group on the one end and to a molecule of interest on the other end, in order to obtain a linker-conjugate, depend on the exact the nature of the linker, the (hetero)cycloalkynyl group and the molecule of interest. Suitable methods are known in the art.

Preferably, the linker-conjugate comprises a (hetero)cyclooctyne group, more preferably a strained (hetero)cyclooctyne group. Suitable (hetero)cycloalkynyl moieties are known in the art. For example DIFO, DIFO2 and DIFO 3 are disclosed in US 2009/0068738. DIBO is disclosed in WO 2009/067663. BARAC is disclosed in J. Am. Chem. Soc. 2010, 132, 3688 - 3690 and US 2011/0207147.

Preferred examples of linker-conjugates comprising a (hetero)cyclooctyne group are shown below.

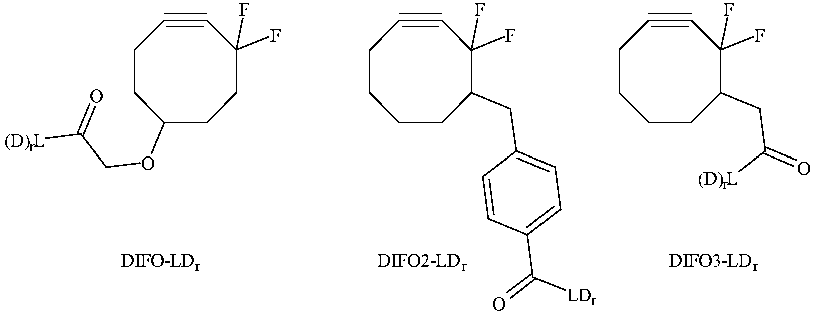

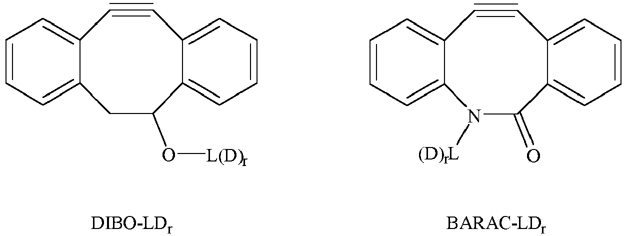

Other cyclooctyne moieties that are known in the art are DIBAC (also known as ADIBO or DBCO) and BCN. DIBAC is disclosed in Chem. Commun. 2010, 46, 97 - 99. BCN is disclosed in WO 2011/136645.

In a preferred embodiment, said linker-conjugate has the Formula **(145):** 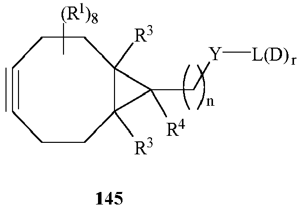 wherein:
R¹, L, D and r are as defined above;
Y is O, S or NR², wherein R² is as defined above;
R³ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
R⁴ is selected from the group consisting of hydrogen, Y-L(D)ᵣ, -(CH₂)ₙ-Y-L(D)ᵣ, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted; and
n is 1, 2, 3, 4, 5, 6, 7, 8, 9 or 10.

In a further preferred embodiment, R¹ is hydrogen. In another preferred embodiment, R³ is hydrogen. In another preferred embodiment, n is 1 or 2. In another preferred embodiment, R⁴ is hydrogen, Y-L(D)ᵣ or -(CH₂)ₙ-Y-L(D)ᵣ. In another preferred embodiment, R² is hydrogen or L(D)ᵣ. In a further preferred embodiment, the linker-conjugate has the Formula **(146):** 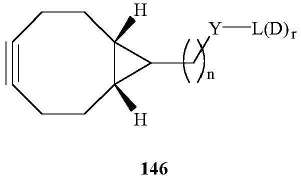 wherein Y, L, D, n and r are as defined above.

In another preferred embodiment, said linker-conjugate has the Formula **(147):** 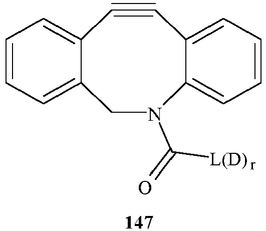 wherein L, D and r are as defined above.

As described above, in a preferred embodiment the modified antibody is a thiol-modified antibody and functional group B comprises an N-maleimide group or a halogenated acetamido group.

### Step (7)

Step (7) of the process according to the invention is an optional step. As was described above, the process comprises one of steps (3), (5) and (7).

Step (7) of the process for the preparation of an antibody-conjugate comprises the steps of:
7(a) repeating step (2), in order to trim an oligosaccharide (N-glycan) that is attached to a different glycosylation site than the oligosaccharide that was trimmed during the first time that step (2) was performed, in order to obtain a proximal N-linked GlcNAc-residue, and
7(b) repeating step (4), in order to attach a monosaccharide derivative Su(A)ₓ to the proximal N-linked GlcNAc-residue that was obtained in step (7a), in order to obtain a proximal N-linked Su(A)ₓGlcNAc-substituent,
7(c) repeating step (6), in order to attach the proximal N-linked Su(A)ₓGlcNAc-substituent that was obtained in step (7b) to a linker-conjugate.

The description of the details of step (2), and the preferred embodiments thereof, also hold for step (7a), the description of the details of step (4), and the preferred embodiments thereof, also hold for step (7b) and the description of the details of step (6), and the preferred embodiments thereof, also hold for step (7c).

In step (7a), also an endo-β-N-acetylglucosaminidase is preferred as the enzyme, but in this step the endo-β-N-acetylglucosaminidase is preferably selected from the group consisting of Endo F1, Endo F2, Endo F3, Endo H and Endo M, Endo A, and any combination thereof.

A preferred embodiment of a process for the preparation of an antibody-conjugate comprising step (7) is described in more detail below.

### Preferred embodiments of the process for the preparation of an antibody-conjugate according to the invention

Several particularly preferred embodiments of the process for the preparation of an antibody-conjugate according to the invention for the preparation of an antibody are described in more detail below.

### First preferred embodiment of the process for the preparation of an antibody-conjugate according to the invention

In a first preferred embodiment of the process for the preparation of an antibody-conjugate according to the invention, said process comprises the steps (1), (2), (4) and (6) as defined above. In this preferred embodiment, the process does not comprise the steps (3), (5) and (7).

In a first preferred embodiment of the process for the preparation of an antibody-conjugate according to the invention, the process thus comprises the steps of:
(1) providing an IgG antibody comprising at least two N-linked glycosylation sites on the combination of a single heavy chain and single light chain; and
(2) trimming an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
wherein the proximal N-linked GlcNAc-residue in steps (2), (4) and (6) is optionally fucosylated.

It is particularly preferred that the enzyme in step (2) is an endoglycosidase, in particular an endo-β-N-acetylglucosaminidase.

The details of steps (1), (2), (4) and (6) of the process according to the invention, and their preferred embodiments, which are described in detail above, also apply to the first preferred embodiment that is described below.

Preferably, in step (2) of this first preferred embodiment of the process according to the invention, the oligosaccharides present on substantially all N-linked glycosylation sites of the IgG antibody comprising at least two N-linked glycosylation sites on the combination of a single heavy chain and a single light chain are trimmed, and converted into proximal N-linked GlcNAc-moieties. Preferably step 1 of this first preferred embodiment of the process is performed in the presence of two or more suitable enzymes, more preferably in the presence of two suitable enzymes, and most preferably in the presence of one suitable enzyme. Even more preferably, step 1 is performed in the presence of Endo F, or in the presence of a combination of Endo S and Endo F.

During step (4) of the first preferred embodiment, substantially all proximal N-linked GlcNAc-moieties are converted to Su(A)xGlcNAc-substituents, by reaction with Su(A)x-P, and during step (6) of the first preferred embodiment, substantially all Su(A)xGlcNAc-substituents are linked to a molecule of interest via a linker L.

An antibody-conjugate obtainable by the process according to the first preferred embodiment of the process according to the invention typically comprises only one type of molecule of interest, conjugated to the antibody via one type of linker, via one type of sugar derivative Su.

The here described first preferred embodiment of the process for the preparation of an antibody-conjugate is particularly preferred for the development of ADCs with improved therapeutic index.

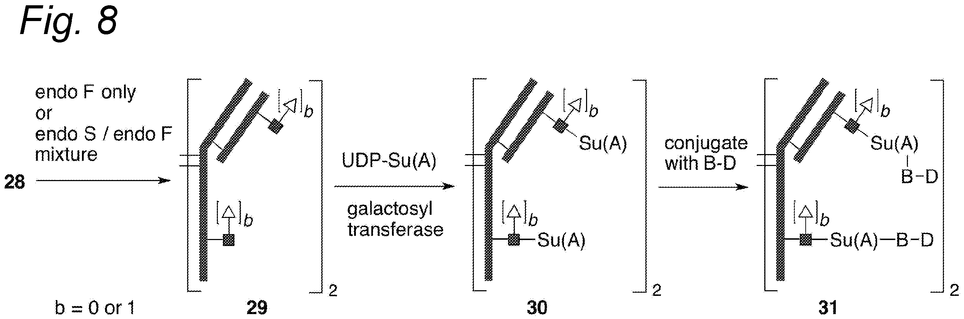

An example of the first preferred embodiment of the process is shown in Figure 8. Figure 8 shows the chemoenzymatic conversion of an IgG with two glycosylation sites (one at N297 and one at another site) into an IgG with two functional groups D upon trimming of both glycans **(28 → 29),** then galactosyl transfer of a modified galactose Su(A) **(29 → 30),** then conjugation with excess B-D, leading to **31.**

Other examples of the first preferred embodiment of the process are shown in Figure 11 and 12. Figure 11 shows the structure of an IgG **38** with two glycosylation sites (none at N297), both glycans of which may be trimmed in a single procedure with for example endoglycosidase F3. Figure 12 shows the structure of an IgG **39** with three glycosylation sites (one at N297 and two at other sites). In this case, either all glycans may be trimmed in a single procedure with for example endoglycosidase F3. Alternatively, the glycan at N297 may be trimmed first with an endoglycosidase selective for that position, e.g. endo S, followed by trimming of the other two glycosylation sites with for example endoglycosidase F3.

### Second preferred embodiment of the process for the preparation of an antibody-conjugate

In a second preferred embodiment of the process according to the invention, said process comprises the steps (1), (2), (4), (6) and (7) as defined above. In this preferred embodiment, the process does not comprise steps (3) and (5).

The details of steps (1), (2), (4), (6) and (7) of the process according to the invention, and their preferred embodiments, which are described in detail above, also apply to the second preferred embodiment that is described below.

In this second preferred embodiment of the process for the preparation of an antibody-conjugate according to the invention, the process thus comprises the steps of:
(1) providing an IgG antibody comprising at least two N-linked glycosylation sites on the combination of a single heavy chain and single light chain; and
(2) trimming an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
(7a) repeating step (2) in order to trim an oligosaccharide that is attached to a different glycosylation site; and
(7b) repeating step (4) in order to attach a monosaccharide derivative Su(A)ₓ to the proximal N-linked GlcNAc-residue that was obtained in step (7a), in order to obtain a proximal N-linked Su(A)ₓGlcNAc-substituent; and
(7c) repeating step (6) in order to attach the proximal N-linked Su(A)ₓGlcNAc-substituent that was obtained in step (7b) to a linker-conjugate; and
wherein the proximal N-linked GlcNAc-residue in steps (2), (4) and (6) is optionally fucosylated.

It is particularly preferred that the enzyme in step (2) is an endoglycosidase, in particular an endo-β-N-acetylglucosaminidase.

In step (2) of the second preferred embodiment, the native N-glycosylation site at N297 is trimmed, preferably by the action of Endo S or Endo S49. Subsequently, in step (4) and (6), the N-linked proximal N-linked GlcNAc-moiety that is obtained in step (2) is conjugated to a molecule of interest. Then, in step (7a), a second N-glycosylation site is trimmed by the action of a different enzyme, e.g. Endo F, and said site is subsequently conjugated to a molecule of interest via steps (7b) and (7c). In cases where more than two N-glycosilation sites are present on the combination of a single heavy chain and a single light chain, typically these additional sites are also trimmed and conjugated to a molecule of interest in steps (7b) and (7c).

In this second preferred embodiment, in step (4) and step (7b) the same sugar derivative Su(A)ₓ may be attached in both steps, but also a different Su(A)ₓ may be attached. Similarly, in steps (6) and (7c), the same or a different molecule of interest may be conjugated to the antibody.

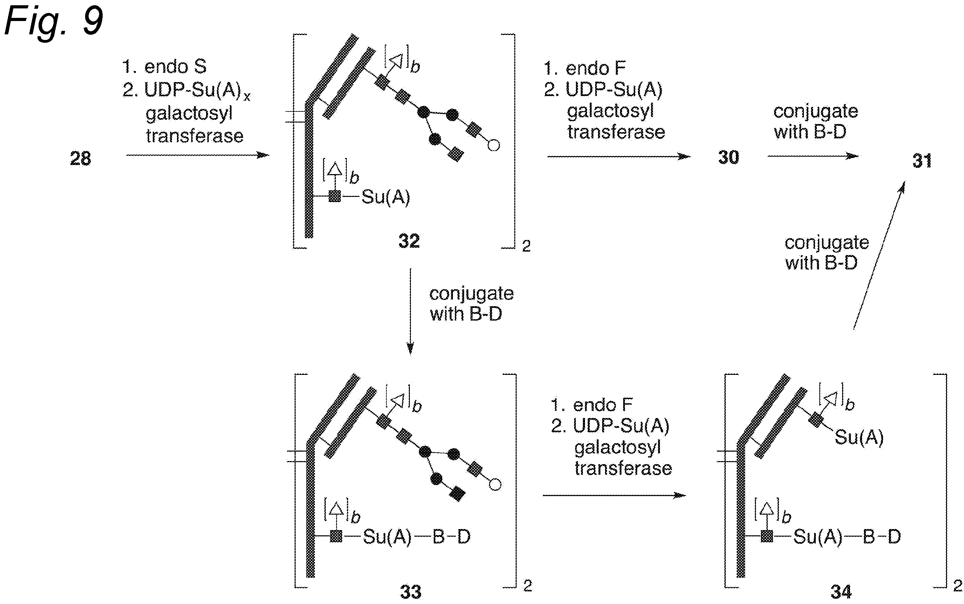

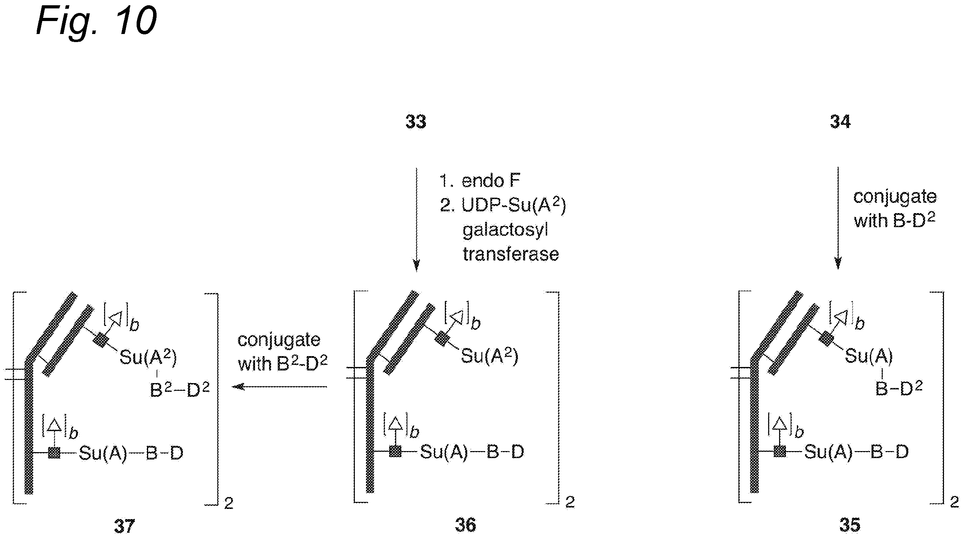

An example of this embodiment of the process according to the invention is shown in Figures 9 and 10. Figure 9 shows the transformation of **28** into **33** by selective trimming of the native glycosylation site with endo S, followed by introduction of modified sugar Su(A) **(28 → 32)** and conjugation with B-D **(32 → 33).** Figure 10 shows subsequent trimming of the remaining glycan in **33** with endo F, followed by introduction of modified sugar Su(A²) **(33 → 36)** and conjugation with B²-D² **(36 → 37).**

The here described second preferred embodiment of the process for the preparation of an antibody-conjugate is particularly preferred when an antibody-conjugate comprising two different types of molecule of interest is desired, and/or two different linkers and/or sugar derivatives is desired.

### Third preferred embodiment of the process for the preparation of an antibody-conjugate

In a third preferred embodiment of the process according to the invention, said process comprises the steps (1), (2), (4), (5) and (6) as defined above. In this preferred embodiment, the process does not comprise steps (3) and (7).

The details of steps (1), (2), (4), (5) and (6) of the process according to the invention, and their preferred embodiments, which are described in detail above, also apply to the third preferred embodiment that is described below.

In this third preferred embodiment of the process for the preparation of an antibody-conjugate according to the invention, the process thus comprises the steps of:
(1) providing an IgG antibody comprising at least two N-linked glycosylation sites on the combination of a single heavy chain and single light chain; and
(2) trimming an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
5(a) repeating step (2), in order to trim an oligosaccharide that is attached to a different glycosylation site; and
5(b) repeating step (4) in order to attach a monosaccharide derivative Su(A)ₓ to the proximal N-linked GlcNAc-residue that was obtained in step (5a); and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
wherein the proximal N-linked GlcNAc-residue in steps (2), (4) and (6) is optionally fucosylated.

It is particularly preferred that the enzyme in step (2) is an endoglycosidase, in particular an endo-β-N-acetylglucosaminidase.

In this third preferred embodiment the native N-glycosylation site on N297 (if present) is trimmed in step (2), preferably by the action of e.g. Endo S or Endo S49. After attaching a sugar derivative Su(A)ₓ to this first glycosylation site, a second (and third, etc, if present) site is trimmed, preferably by the action of a different endoglycosidase, e.g. Endo F. The conjugation step (6) is then performed for all sites in a single step.

Since the conjugation step for all N-linked glycosylation sites is performed in step (6), the antibody-conjugate according to this third preferred embodiment of the process according to the invention comprises one type of molecule of interest.

Examples of the second and third preferred embodiment of the process according to the invention are shown in Figure 9. Figure 9 shows two step-wise approaches for the same overall transformation of 28 into 30. Both routes commence by selective trimming of the native glycosylation site with endo S, followed by introduction of modified sugar Su(A) **(28 → 32).** Next, one route involves endo F trimming, followed by introduction of the second Su(A), then global conjugation as in Figure 8 ((**29 → 30**). The second route comprises a single conjugation with B-D **(32 → 33),** prior to endo F trimming and Su(A) introduction **(33 → 34)** and again conjugation with B-D to give the same product **31.**

### Fourth preferred embodiment of the process for the preparation of an antibody-conjugate

In a fourth preferred embodiment of the process according to the invention, said process comprises the steps (1), (2), (3), (4) and (6) as defined above. In this preferred embodiment, the process does not comprise steps (5) and (7).

The details of steps (1), (2), (3), (4) and (6) of the process according to the invention, and their preferred embodiments, which are described in detail above, also apply to the fourth preferred embodiment that is described below.

In this fourth preferred embodiment of the process for the preparation of an antibody-conjugate according to the invention, the process comprises the steps of:
(1) providing an IgG antibody comprising at least two N-linked glycosylation sites on the combination of a single heavy chain and single light chain; and
(2) trimming an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate; and
(3) repeating step (2) in order to trim an oligosaccharide that is attached to a different glycosylation site; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
wherein the proximal N-linked GlcNAc-residue in steps (2), (4) and (6) is optionally fucosylated.

It is particularly preferred that the enzyme in step (2) is an endoglycosidase, in particular an endo-β-N-acetylglucosaminidase.

In the fourth preferred embodiment of the process according to the invention, the native glycosylation site on N297 is trimmed first in step (2), by the action of Endo S or Endo S49. A second glycosylation (and third, etc.) site is then trimmed, preferably by the action of a different enzyme, e.g. Endo F. The attaching of Su(A)ₓ in step (4) and subsequent conjugation in step (6) is performed together for all glycosylation sites.

### Antibody-conjugate

The present invention also relates to an antibody-conjugate obtainable by the process for the preparation of an antibody-conjugate according to the invention. Said process and preferred embodiments thereof are described in detail above. An antibody-conjugate is herein defined as an antibody that is conjugated to a molecule of interest D via a linker L. A molecule of interest D, and preferred embodiments thereof, are described in more detail above.

The invention further relates to an antibody-conjugate, wherein the antibody-conjugate is an IgG antibody comprising at least two N-linked glycosylation sites on the combination of a single heavy chain and a single light chain, wherein said IgG antibody is conjugated to a molecule of interest D at each glycosylation site via a linker L.

In a preferred embodiment, the antibody-conjugate is obtainable by the process according to the invention, wherein in step (6) an azide-modified antibody is reacted with a the linker-conjugate comprising a (hetero)cycloalkynyl group or an alkynyl group, and one or more molecules of interest.

In another preferred embodiment, the antibody-conjugate is obtainable by the process according to the invention, wherein in step (6) a keto-modified antibody, is reacted with a linker-conjugate comprising a primary amine group, an aminooxy group or a hydrazinyl group, and one or more molecules of interest.

In another preferred embodiment, the antibody-conjugate is obtainable by the process according to the invention, wherein in step (6) an alkyne-modified antibody is reacted with a linker-conjugate comprising an azido group, and one or more molecules of interest.

In another preferred embodiment, the antibody-conjugate is obtainable by the process according to the invention, wherein in step (6) a thiol-modified antibody or a mercaptoacetamide-modified antibody is reacted with a linker-conjugate comprising an N-maleimide group or a halogenated acetamido group, and one or more molecules of interest.

In another preferred embodiment, the antibody-conjugate is obtainable by the process according to the invention, wherein in step (6) a halogen-modified antibody, a halogenated acetamido-modified antibody, a sulfonyloxy-modified antibody or a sulfonylated hydroxyacetamido-modified antibody is reacted with a linker-conjugate comprising a thiol group, and one or more molecules of interest.

In a further preferred embodiment, the antibody-conjugate is obtainable by the process according to the invention, wherein in step (6) a thiol-modified antibody is reacted with a linker-conjugate comprising a functional group B, and functional group B comprises an N-maleimide group or a halogenated acetamido group.

In a further preferred embodiment, the antibody-conjugate is obtainable by the process according to the invention, wherein in step (6) an azide-modified antibody is reacted with a linker-conjugate according to formulas **(140a),** (**140b**), **(145), (146)** or **(147).** In another preferred embodiment, the antibody-conjugate is obtainable by the process according to the invention, wherein in step (6) an azide-modified antibody is reacted with a linker-conjugate according to formulas **(140a), (145), (146)** or **(147).**

Since the antibody-conjugate is based on an IgG antibody comprising at least two N-linked glycosylation sites on the combination of a single heavy chain and a single light chain, when the antibody is a whole antibody, the antibody conjugate is conjugated to 4 or more molecules of interest. In a preferred embodiment the antibody-conjugate comprises 4, 6, 8 or 10 molecules of interest, preferably 4 or 6 molecules of interest and most preferably 4 molecules of interest.

The process for the preparation of an antibody according to the invention makes it possible to introduce different types of molecules of interest into an antibody-conjugate. In a preferred embodiment, the antibody-conjugate comprises two or more different types of a molecule of interest, more preferably two different types of molecules of interest.

In another preferred embodiment, the molecule of interest is selected from the group consisting of a reporter molecule, an active substance, an enzyme, an amino acid, a protein, a peptide, a polypeptide, an oligonucleotide, a glycan, an azide or a (hetero)cycloalkynyl moiety.

In a further preferred embodiment, the molecule of interest D is selected from the group consisting of pharmaceutically active substances, and more preferably D is selected from the group consisting of drugs and prodrugs.

More preferably, the active substance is selected from the group consisting of pharmaceutically active compounds, in particular low to medium molecular weight compounds (e.g. about 200 to about 1500 Da, preferably about 300 to about 1000 Da), such as for example cytotoxins, antiviral agents, antibacterials agents, peptides and oligonucleotides. Examples of cytotoxins include colchicine, vinca alkaloids, camptothecins, doxorubicin, daunorubicin, taxanes, calicheamycins, tubulysins, irinotecans, an inhibitory peptide, amanitin, deBouganin, duocarmycins, maytansines, auristatins or pyrrolobenzodiazepines (PBDs), preferred examples include camptothecins, doxorubicin, daunorubicin, taxanes, calicheamycins, duocarmycins, maytansines, auristatins or pyrrolobenzodiazepines (PBDs). In a preferred embodiment, the cytotoxin is selected from the group consisting of camptothecins, doxorubicin, daunorubicin, taxanes, calicheamycins, duocarmycins, maytansines, auristatins and pyrrolobenzodiazepines (PBDs). In another preferred embodiment, the cytotoxin is selected from the group consisting of colchicine, vinca alkaloids, tubulysins, irinotecans, an inhibitory peptide, amanitin and deBouganin.

In a preferred embodiment, the drug is selected from the group of toxins or radiopharmaceuticals.

Toxins are described in more detail above.

The invention particularly relates to an antibody-conjugate according to the invention, wherein the molecule of interest is a drug or a prodrug, preferably a toxin, and wherein the antibody is conjugated to 4 or 6 molecules of interest, more preferably to 4 molecules of interest.

In another preferred embodiment, the antibody is conjugated to 4 or 6 molecules of interest, more preferably to 4 molecules of interest, wherein the antibody comprises two different types of molecules of interest, e.g. two different toxins.

In one embodiment, the antibody-conjugate according to the invention is an antibody-conjugate, wherein the conjugation sites are present in the C_{H}2 and/or C_{H}3 region of the antibody. In another embodiment, the antibody-conjugate according to the invention is an antibody-conjugate, wherein the conjugation sites are present in the C_{H}2 and/or C_{H}3 region of the antibody, and wherein the antibody is a Fc-fragment.

In another embodiment, the antibody-conjugate according to the invention is an antibody-conjugate, wherein the conjugation sites are present in the C_{H}1, V_{H}, C_{L} and/or V_{L} region of the antibody. In yet another embodiment, the antibody-conjugate is an antibody-conjugate, wherein the conjugation sites are present in the C_{H}1, V_{H}, C_{L} and/or V_{L} region of the antibody, and wherein the antibody is a Fab-fragment.

The invention therefore also relates to an antibody-conjugate according to the invention, wherein the antibody-conjugate is an antibody-drug conjugate (ADC). An antibody-drug conjugate is herein defined as an antibody that is conjugated to a molecule of interest (D) via a linker (L), wherein D is selected from the group consisting of pharmaceutically active substances, and more preferably D is selected from the group consisting of drugs and prodrugs.

More preferably, the active substance is selected from the group consisting of pharmaceutically active compounds, in particular low to medium molecular weight compounds (e.g. about 200 to about 1500 Da, preferably about 300 to about 1000 Da), such as for example cytotoxins, antiviral agents, antibacterials agents, peptides and oligonucleotides. Examples of cytotoxins include colchicine, vinca alkaloids, camptothecins, doxorubicin, daunorubicin, taxanes, calicheamycins, tubulysins, irinotecans, an inhibitory peptide, amanitin, deBouganin, duocarmycins, maytansines, auristatins or pyrrolobenzodiazepines (PBDs), preferred examples include camptothecins, doxorubicin, daunorubicin, taxanes, calicheamycins, duocarmycins, maytansines, auristatins or pyrrolobenzodiazepines (PBDs).

The invention thus also relates to an antibody-drug-conjugate obtainable by the process for the preparation of an antibody-conjugate according to the invention. The preferred embodiments described above for a antibody-conjugate also hold when the antibody-conjugate is an antibody-drug-conjugate.

The invention further relates to an antibody-drug conjugate, wherein the molecule of interest is a drug or prodrug. More preferably, said molecule of interest is a toxin.

In a preferred embodiment, the antibody-drug conjugate according to the invention has a drug-antibody ratio (DAR) of 4.

The modified antibody, the antibody-conjugate and the processes for the preparation thereof according to the invention have several advantages over the processes, modified antibodies and antibody-conjugates known in the art.

Specific advantages of the process for the preparation of ADCs with two different toxins according to the invention include the potential for distal multiple labeling of an antibody (no interference of labels) and the possibility to perform two-dimensional efficacy optimization with respect to conjugation site. Thirdly, full optimization is possible with respect to toxin, not only with respect to stoichiometry, but also with respect to efficacy. For example, two drugs with a different mode of action can be attached for more effective cell-killing, with a better chance of effect (and potentially synergistic effect). In general, combination therapy of drugs is highly popular for this reason.

In a particularly preferred embodiment of the antibody-conjugate according to the invention, the molecule of interest is selected from the group of pharmaceutically active substances. In a further preferred embodiment the active substance is selected from the group of drugs and prodrugs. Even more preferably, the molecule of interest is selected from the group consisting of low to medium molecular weight compounds. More preferably, the molecule of interest is selected from the group consisting of cytotoxins, antiviral agents, antibacterial agents, peptides and oligonucleotides, and most preferably the molecule of interest is a cytotoxin. In a further preferred embodiment, the molecule of interest is selected from the group consisting of colchicine, vinca alkaloids, camptothecins, doxorubicin, daunorubicin, taxanes, calicheamycins, tubulysins, irinotecans, an inhibitory peptide, amanitin, deBouganin, duocarmycins, maytansines, auristatins and pyrrolobenzodiazepines (PBDs). In a preferred embodiment, the cytotoxin is selected from the group consisting of camptothecins, doxorubicin, daunorubicin, taxanes, calicheamycins, duocarmycins, maytansines, auristatins and pyrrolobenzodiazepines (PBDs). In another preferred embodiment, the cytotoxin is selected from the group consisting of colchicine, vinca alkaloids, tubulysins, irinotecans, an inhibitory peptide, amanitin and deBouganin.

When the molecule of interest in the antibody-conjugate according to the invention is an active substance, the antibody-conjugate may also be referred to as "antibody-drug conjugate" (ADC).

The invention further relates to an antibody-conjugate according to the invention, wherein the molecule of interest is an active substance, for use as a medicament.

The invention also relates to the use of an antibody-conjugate according to the invention, wherein the molecule of interest is an active substance, for use in the treatment of cancer.

The invention further relates to an antibody-conjugate according to the invention, wherein the molecule of interest is an active substance, for use in the treatment of breast cancer, more preferably for use in the treatment of HER2-positive breast cancer.

As described above, the antibody-conjugates according to the invention have several advantages over antibody-conjugates known in the prior art. One of the advantages of the modified antibodies, the antibody-conjugates and the process for their preparation according to the invention is that these antibodies and antibody-conjugates are homogeneous, both in site-specificity and stoichiometry. The modified antibodies and antibody-conjugates according to the invention are obtained with a DAR very near to the theoretical value, and with a very low standard deviation. This also means that the antibody-conjugates according to the invention result in a more consistent product for preclinical testing.

The properties of an antibody conjugate according to the invention may be modulated by designing, expressing, and processing into antibody-drug conjugates the monoclonal antibodies with different glycosylation profiles. The properties that may be modulated are e.g. anti-tumor activity, the maximum tolerated dose, pharmacokinetics such as plasma clearance, therapeutic index, both in terms of efficacy and toxicity, attenuation of the drug, stability of the attachment of the drug and release of the drug after reaching the target. In particular, there is a correlation between location of drug and the *in vivo* efficacy of ADC.

### Glycoengineered antibody and process for the preparation thereof

The invention further relates to a process for the preparation of a glycoengineered antibody, comprising the steps of:
(i) providing an IgG antibody comprising at least two N-linked glycosylation sites on the combination of a single heavy chain and single light chain; and
(ii) trimming an oligosaccharide that is attached to a glycosylation site, by the action of a suitable enzyme, in order to obtain a proximal N-linked GlcNAc-residue at said glycosylation site, wherein a suitable enzyme is defined as an enzyme wherefore the oligosaccharide that is to be trimmed is a substrate; and
(iii) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(iv) optionally repeating steps (ii) and (iii) for a different N-linked glycosylation site;
wherein the protein-proximal N-linked GlcNAc-residue is optionally fucosylated; and wherein a glycoengineered antibody is defined as an antibody comprising two or more protein-proximal N-linked GlcNAc-Su(A)ₓ substituents, wherein the GlcNAc-residue in said substituent is optionally fucosylated.

It is particularly preferred that the enzyme in step (2) is an endoglycosidase, in particular an endo-β-N-acetylglucosaminidase.

The invention also relates to a glycoengineered antibody obtainable by the process as described above.

### Examples

### Synthesis

### Example 1. Synthesis of 41

BCN-PEG₂-alcohol **40** (3.6 g, 11.1 mmol) was dissolved in DCM (150 mL) and Et₃N (4.61 mL, 33.3 mmol) and disuccinimidyl carbonate (4.3 g, 16.7 mmol) were added. After 2 h the reaction was quenched with H₂O (100 mL) and the organic layer was washed with water (2 × 150 mL), dried over Na₂SO₄, filtrated and concentrated in vacuo. Flash column chromatography (EtOAc:MeOH 99:1-94:6) afforded activated carbonate **41** (4.63 g, 8.6 mmol, 78%).

### Example 2. Synthesis of BCN-vc-PABA-MMAF (42)

To a solution of H-Val-Cit-PAB-MMAF.TFA (17.9 mg, 14.3 µmol) in DMF (2 mL) was added **41** (17.9 mg, 14.3 µmol) **(36)** as a solution in DMF (0.78 mL) and triethylamine (6.0 µL). The product (7 mg, 5 µmol, 35%) was obtained after purification *via* reversed phase HPLC (C18, gradient H₂O/MeCN 1% AcOH). LRMS (HPLC, ESI+) calcd for C₇₄H₁₁₄N₁₁O₁₈ (M+H⁺) 1445.79, found 1445.44.

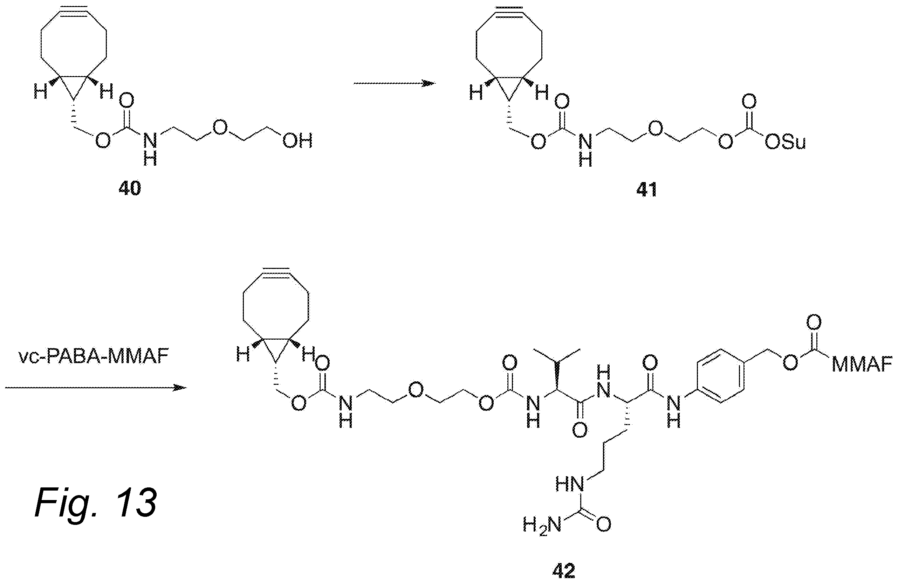

The synthetic route to compound **42** is graphically depicted in Figure 13.

### Example 3. Synthesis of BCN-vc-PABA-β-ala-maytansin (43)

To a suspension of H-Val-Cit-PABA-β-alaninoyl-maytansin (commercially available from Concortis) (27 mg, 0.022 mmol) in MeCN (2 mL) was added triethylamine (9.2 µL, 6.7 mg, 0.066 mmol) and a solution of **41** (9.2 mg, 0.022 mmol) in MeCN (1 mL). After 23 h, the mixture was poured out in a mixture of EtOAc (20 mL) and water (20 mL). After separation, the organic phase was dried (Na₂SO₄) and concentrated. After purification *via* column chromatography (EtOAc → MeOH/EtOAc 1/4) 22 mg (0.015 mmol, 70%) of the desired product **43** was obtained. LRMS (ESI+) calcd for C₇₀H₉₇ClN₁₀O₂₀ (M+H⁺) 1433.66, found 1434.64.

### Antibody glycosylation mutants

Both native trastuzumab and mutant antibodies were transiently expressed in CHO K1 cells by Evitria (Zurich, Switzerland), purified using protein A sepharose and analyzed by mass spectrometry.

A specific L196N mutant of trastuzumab was derived from literature (Qu et al., J. Immunol. Meth. 1998, 213, 131).

### General protocol for mass spectral analysis of IgG

A solution of 50 µg (modified) IgG, 1 M Tris-HCl pH 8.0, 1 mM EDTA and 30 mM DTT with a total volume of approximately 70 µL was incubated for 20 minutes at 37 °C to reduce the disulfide bridges allowing to analyze both light and heavy chain. If present, azide-functionalities are reduced to amines under these conditions. Reduced samples were washed trice with milliQ using an Amicon Ultra-0.5, Ultracel-10 Membrane (Millipore) and concentrated to 10 µM (modified) IgG. The reduced IgG was analyzed by electrospray ionization time-of-flight (ESI-TOF) on a JEOL AccuTOF. Deconvoluted spectra were obtained using Magtran software.

### General Protocol for trimming of IgG glycans using Endo S

Trimming of IgG glycans was performed using endo S from *Streptococcus pyogenes* (commercially available from Genovis, Sweden). The IgG (10 mg/mL) was incubated with endo S (40 U/mL final concentration) in 25 mM Tris pH 8.0 for 16 hours at 37 °C.

### General Protocol for trimming of IgG glycans using Endo F2 or Endo F3

Trimming of IgG glycans was performed using endoF2 from *Elizabethkingia miricola* (commercially available from QA Bio) or endoF3 from *Elizabethkingia meningosepticum* (commercially available from QA Bio). The IgG (10 mg/mL) was incubated with endo F2 (100 mU/mg IgG) or EndoF3 (25 mU/mg IgG) in 100 mM sodium citrate pH 4.5 for 16 hours at 37 °C. The deglycosylated IgG was concentrated and washed with 25 mM Tris-HCl pH 8.0 using an Amicon Ultra-0.5, Ultracel-10 Membrane (Millipore).

### Example 4. Trimming of native trastuzumab

Trastuzumab with base MS peaks at 50444, 50591 and 50753 and 50914 Da, corresponding to G0, G0F, G1F and G2F isoforms of glycosylation, was subjected to the trimming protocol with endo S above. After deconvolution of peaks, the mass spectrum showed one peak of the light chain and two peaks of the heavy chain. The two peaks of heavy chain belonged to one major product (49496 Da, 90% of total heavy chain), resulting from core GlcNAc(Fuc)-substituted trastuzumab, and a minor product (49351 Da, ±10% of total heavy chain), resulting from core GlcNAc-substituted trastuzumab.

### Example 5. Combined endoS/endoF3 trimming of trastuzumab 196 mutant

The trastuzumab-HC-L196N mutant contains two glycosylation sites on the heavy chain (N196 and N297), which is confirmed by the various heavy chain variants ranging from 52300 to 52600 Da (100% of total heavy chain). When trastuzumab-HC-L196N was subjected to the EndoS-based trimming protocol described above, only the N297-glycosylation site was trimmed (all heavy chain products between 51000 and 52100 Da with major peaks of 51265, 51556 and 51847 Da corresponding to the G2FS(0-2) (isoforms with 0, 1 and 2 sialic acids attached), the mass spectral profile is depicted in Figure 17. This product was subsequently incubated with endoglycosidase F3 (Endo F3, 25 mU/mg IgG) from *Elizabethkingia meningosepticum* (commercially available from QA-Bio) in 100 mM sodium citrate pH 4.5 for 16 hrs, which led to complete deglycosylation (all heavy chain products between 49750 and 50050 Da with major peak of 49844 Da resulting from L196N heavy chain with two core GlcNAc(Fuc) moieties).

### Example 6. Combined endo S/endo F2 trimming of cetuximab

Cetuximab contains a second N-glycosylation site at N88, besides the native glycan at N297. The glycan at N88 is located in the Fab region and has a different constitution from the glycan at N297. Treatment of cetuximab similar to trastuzumab led to the formation of a range of heavy chain products with masses of 51600 Da-52300 Da (major peaks at 51663 Da and 51808 Da), indicating that only one glycan was trimmed by Endo S, at N297. Subsequent incubation with Endoglycosidase F2 (EndoF2, 100 mU/mg IgG) from *Elizabethkingia miricola* (commercially available from QA-Bio) in 50 mM sodium citrate pH 4.5 for 16 hrs resulted in complete deglycosylation (major heavy chain product of 49913, ±80% of total heavy chain, and minor heavy chain product of 50041 Da, ±20% of total heavy chain). When cetuximab was directly incubated with EndoF2 the deglycosylated heavy chain products (49913 and 50041 Da) were also observed, showing that EndoF2 is able to trim both glycosylation sites.

### Example 7. Multiple glycosyltransfer with GalT(Y289L) + UDP-GalNAz to trimmed trastuzumab 196 mutant

Trimmed trastuzumab-HC-L196N (10 mg/mL) obtained as described above was incubated with GalNAz-UDP (1.3 mM) and β1,4-Gal-T1-Y289L (0.2 mg/mL) in 10 mM MnCl₂ and 25 mM Tris-HCl pH 8.0 for 16 hours at 30 °C, which led to complete conversion into trastuzumab-HC-L196N(GalNaz)₄ (all heavy chain products between 50200 and 50600 Da with major peak of 50280 Da resulting from L196N heavy chain containing two GalNaz moieties of which the azides have been reduced during sample preparation).

### Example 8. Multiple glycosyltransfer with GalT(Y289L) + UDP-GalNAz to trimmed cetuximab

Trimmed cetuximab (10 mg/mL), obtained by sequential deglycosylation using EndoS and EndoF3 as described above, was incubated with GalNaz-UDP (2 mM) and β1,4-Gal-T1-Y289L (0.2 mg/mL) in 10 mM MnCl₂ and 25 mM Tris-HCl pH 8.0 for 16 hours at 30 °C, which led to complete conversion into Cetuximab(GalNaz)₄ (major heavy chain product of 50352, ±80% of total heavy chain, and minor heavy chain product of 50480 Da, ±20% of total heavy chain).

### Example 9. Conjugation of trastuzumab-HC-L196N-(GalNAz)₄ to BCN-vc-PABA-May 43

BCN-vc-PABA-maytansin derivative **43** (4 eq) in DMF was added to protein A purified trastuzumab-HC-L196N(GalNAz)₄ (100 µM) in PBS and incubated overnight at room temperature. To obtain complete conversion, this step was repeated 5 times resulting in trastuzumab-HC-L196N(-vc-PABA-May)₄ (heavy chain products between 53100 and 53500 Da with major peak of 53200 Da corresponding to heavy chain conjugated to two BCN-vc-PABA-May moieties, ±95% of total heavy chain, and a minor peak at 52434 Da due to fragmentation of the PABA linker during mass spectrometry, ±5% of total heavy chain).

### Example 10. Conjugation of cetuximab-(GalNAz)₄ to BCN-vc-PABA-MMAF 42

BCN-vc-PABA-MMAF **42** (4 eq) in DMF was added to cetuximab(GalNAz)₄ (100 µM) in PBS and incubated overnight at room temperature, which led to complete conversion into cetuximab(vc-PABA-MMAF)₄ (major heavy chain product of 53293, ±75% of total heavy chain, and minor heavy chain product of 53422 Da, ±15% of total heavy chain, which both correspond to the desired product, and a minor heavy chain product of 52517 Da, ±10% of total heavy chain, which corresponds to the desired product with fragmented PABA linker during mass spectrometry analysis).

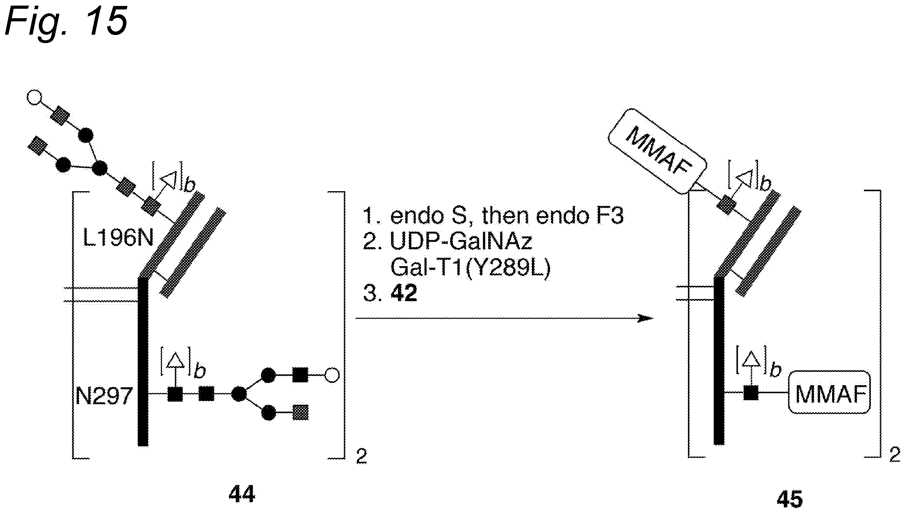

The overall process of Example 8 and Example 10 is schematically depicted in Figure 15.

### Example 11. Galactosidase trimming of trastuzumab, then GalT(Y289L) + UDP-GalNAz, then conjugation to BCN-vc-PABA-May

Trastuzumab (10 mg/mL) was incubated with β1,4-galactosidase (3 mU/mg IgG) from *Streptococcus pneumoniae* (commercially available from Calbiochem) in 50 mM sodium phosphate pH 6.0 for 16 hrs at 37 °C, which led to complete conversion into the trastuzumab with G0 and G0F glycan structures (major product of 50592 Da corresponding to the heavy chain with G0F glycan and no peaks corresponding to the G1, GIF, G2 and G2F glycoforms). Trastuzumab-GO(F) (10 mg/mL) was subsequently incubated with *UDP-GalNAz* (625 µM) and β1,4-Gal-T1-Y289L (0.1 mg/mL) in 10 mM MnCl₂ and 25 mM Tris-HCl pH 8.0 for 16 hours at 30 °C, which led to complete conversion into trastuzumab-G0(F)-(GalNAz)₄ (all heavy chain products between 50800 and 51200 Da with major peak of 51027 Da resulting from the G0F heavy chain containing two GalNaz moieties of which the azides have been reduced during sample preparation).

BCN-vc-PABA-May **43** (4 eq) in DMF was added to trastuzumab-G0(F)-(GalNAz)₄ (100 µM) in PBS and incubated overnight at room temperature. To obtain complete conversion, this step was repeated 5 times resulting in trastuzumab-GO(F)-(-vc-PABA-maytansin)₄ (one major peak of 53946 Da corresponding to heavy chain containing two BCN-vc-PABA-maytansin moieties, ±90% of total heavy chain).

### Example 12. Consecutive endoS-GalNAz-conjugation, then endoF3-GalNAz-conjugation of trastuzumab(L196N) mutant

Trastuzumab(L196N) mutant **46** (7.5 mg, 14 mg/mL) was trimmed with Endo S (3 µL, 20 U/ µL) according to the general protocol. AccuTOF analysis showed complete conversion to the desired products (mass 51556 and 51846, together with some small impurities). The trimmed mutant was incubated with *UDP-GalNAz* (120 µL, 10 mM) and β(1,4)-Gal-T1(Y289L) (38 µL, 2 mg/mL) in 10 mM MnCl₂ and 25 mM Tris-HCl pH 8.0 for 16 hours at 30 °C. AccuTOF analysis showed complete conversion to the desired products (mass 51776 and 52067, expected mass 51775 and 52065), as depicted in Figure 18. After ProtA purification trast-(GalNAz)₂ (7 mg, 23 mg/ml in PBS) was isolated and BCN-vc-PABA-maytansin **(43,** 4.4 µL, 40 mM) was added. The mixture was rotated end-over-end and extra BCN-vc-PABA-maytansin **(43,** 4.4 µL, 40 mM) was added three times. After two days, complete conversion was achieved and ProtA purification gave trast-(vc-PABA-maytansin)₂ **47** (3.4 mg, 42 mg/ml). Next trast-(vc-PABA-maytansin)₂ **47** (1 mg, 42 mg/ml) was spin-filtered three times against sodium citrate (50 mM, pH 4.5) and diluted to 50 µL with the same buffer. Trimming of the other glycosylation site was performed with Endo F3 (5 µL, 0.33 U/mL, 20 mM tris-HCl) overnight at 37 °C. The trimmed trast-(vc-PABA-maytansin)₂ was incubated with *UDP-GalNAz* (10 µL, 10 mM) and β(1,4)-Gal-T1(Y289L) (5 µL, 2 mg/mL) in 10 mM MnCl₂ and 25 mM Tris-HCl pH 8.0 for 16 hours at 30 °C. AccuTOF analysis showed complete conversion to the desired product (mass 51741 Da, expected mass 51742 Da). After ProtA purification trast-(vc-PABA-maytansin)₂-(GalNAz)₂ (0.54 mg, 10 mg/mL in PBS) was isolated and BCN-vc-PABA-MMAF **(42,** 0.7 µL, 40 mM) was added. The mixture was rotated end-over-end overnight and analysis by AccuTOF showed 85% conversion to trast-(vc-PABA-maytansin)₂-(vc-PABA-MMAF)₂ (mass 53213, expected mass 53215), 15% were impurities.

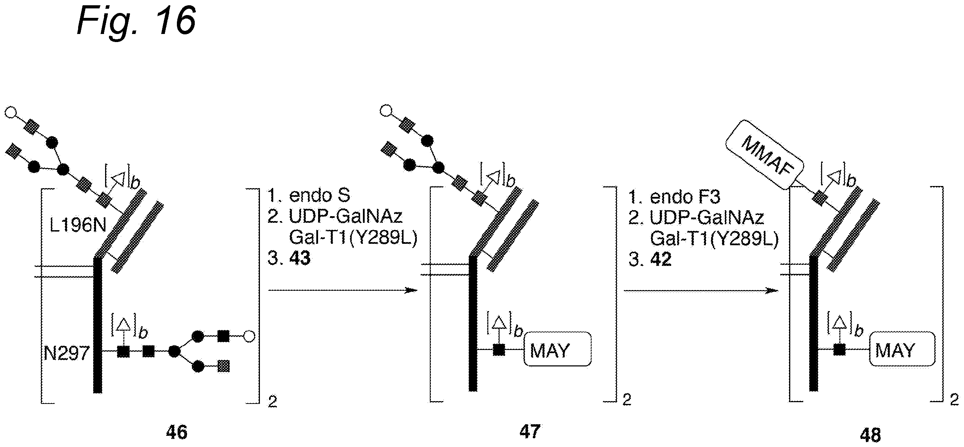

The above process for conversion of trastuzumab(L196N) mutant to trast-(vc-PABA-maytansin)₂-(vc-PABA-MMAF)₂ is schematically depicted in Figure 16.

The corresponding MS profiles are depicted in Figure 19 (trastuzumab derivative **47),** Figure 20 (trastuzumab derivative **47** + trimming with endo F3), Figure 21 (trastuzumab derivative **47** + trimming with endo F3 + transfer of UDP-GalNAz) and Figure 22 (trast-(vc-PABA-maytansin)₂-(vc-PABA-MMAF)₂ **48**).

### Example 13: In vitro efficacy

SK-Br-3 (Her2+), SK-OV-3 (Her2+) and MDA-MB-231 (Her2-) cells were plated in 96-wells plates (5000 cells/well) in RPMI 1640 GlutaMAX (Invitrogen) supplemented with 10% fetal calf serum (FCS) (Invitrogen, 200 µL/well) and incubated overnight at 37 °C and 5% CO₂. A three-fold dilution series (ranging from ±0.002 to 100 nM) of the sterile-filtered compounds was prepared in RPMI 1640 GlutaMAX supplemented with 10% FCS. After removal of the culture medium, the concentration series were added in quadruplo and incubated for three days at 37 °C and 5% CO₂. The culture medium was replaced by 0.01 mg/mL resazurin (Sigma Aldrich) in RPMI 1640 GlutaMAX supplemented with 10% FCS. After 4 to 6 hours at 37°C and 5% CO₂ fluorescence was detected with a fluorescence plate reader (Tecan Infinite 200) at 540 nm excitation and 590 nm emission. The relative fluorescent units (RFU) were normalized to cell viability percentage by setting wells without cells at 0% viability and wells with lowest dose of compound at 100% viability. For each conditions the average cell viability percentage ± sem is shown.

The in vitro cytotoxicity of trastuzumab-(vc-PABA-maytansin)₂, trastuzumab-HC-L196N(-vc-PABA-May)₄, trastuzumab-G0(F)-(-vc-PABA-maytansin)₄ were compared to T-DM1 as a positive control and trastuzumab and rituximab-(vc-PABA-maytansin)₂ as negative controls (Figures 23-25). All trastuzumab-based ADCs affect the viability of the Her2-positive cell lines SK-Br-3 and SK-OV-3, but not of the Her2 negative cell line MDA-MB-231, which demonstrates that these ADCs specifically target Her2-positive cells. In the Her2 negative cell line MDA-MB-231, only T-DM1 shows a slight decrease in cell viability at the highest concentration (100 nM).

### Example 14: Cloning and expression of GalT mutants Y289N, Y289F, Y289M, Y289V, Y289A, Y289G and Y289I.

The *GalT* mutant genes were amplified from a construct containing the sequence encoding the catalytic domain of GalT consisting of 130-402 aa residues, by the overlap extension PCR method. The wild type enzyme is represented by SEQ ID NO: 17. For Y289N mutant (represented by SEQ ID NO: 18), the first DNA fragment was amplified with a pair of primers: Oligo38_GalT_External_Fw (CAG CGA CAT ATG TCG CTG ACC GCA TGC CCT GAG GAG TCC represented by SEQ ID NO: 1) and Oligo 19_GalT_Y289N_Rw (GAC ACC TCC AAA **GTT** CTG CAC GTA AGG TAG GCT AAA represented by SEQ ID NO: 2). The *NdeI* restriction site is underlined, while the mutation site is highlighted in bold. The second fragment was amplified with a pair of primers: Oligo29_GalT_External_Rw (CTG ATG GAT GGA TCC CTA GCT CGG CGT CCC GAT GTC CAC represented by SEQ ID NO: 3) and Oligo18_GalT_Y289N_Fw (CCT TAC GTG CAG **AAC** TTT GGA GGT GTC TCT GCT CTA represented by SEQ ID NO: 4). The *BamHI* restriction site is underlined, while the mutation site is highlighted in bold. The two fragments generated in the first round of PCR were fused in the second round using Oligo38_GalT_External_Fw and Oligo29_GalT_External_Rw primers. After digestion with *NdeI* and *BamHI.* This fragment was ligated into the pET16b vector cleaved with the same restriction enzymes. The newly constructed expression vector contained the gene encoding Y289N mutant and the sequence encoding for the His-tag from pET16b vector, which was confirmed by DNA sequencing results. For the construction of Y289F (represented by SEQ ID NO: 19), Y289M (represented by SEQ ID NO: 20), Y289I (represented by SEQ ID NO: 21), Y289V (represented by SEQ ID NO: 22), Y289A (represented by SEQ ID NO: 23) and Y289G (represented by SEQ ID NO: 24) mutants the same procedure was used, with the mutation sites changed to **TTT, ATG, ATT, GTG, GCG** or **GGC** triplets encoding for phenylalanine, methionine, isoleucine, valine, alanine or glycine, respectively. More specifically, for the construction of Y289F the first DNA fragment was amplified with a pair of primers defined herein as SEQ ID NO: 1 and SEQ ID NO: 5 and the second fragment was amplified with a pair of primers defined herein as SEQ ID NO: 3 and SEQ ID NO: 6 (be referred to Table 1 for the related sequences). Furthermore, for the construction of Y289M the first DNA fragment was amplified with a pair of primers defined herein as SEQ ID NO: 1 and SEQ ID NO: 7 and the second fragment was amplified with a pair of primers defined herein as SEQ ID NO: 3 and SEQ ID NO: 8. For the construction of Y289I the first DNA fragment was amplified with a pair of primers defined herein as SEQ ID NO: 1 and SEQ ID NO: 9 and the second fragment was amplified with a pair of primers defined herein as SEQ ID NO: 3 and SEQ ID NO: 10. For the construction of Y289V the first DNA fragment was amplified with a pair of primers defined herein as SEQ ID NO: 1 and SEQ ID NO: 11 and the second fragment was amplified with a pair of primers defined herein as SEQ ID NO: 3 and SEQ ID NO: 12. for the construction of Y289A the first DNA fragment was amplified with a pair of primers defined herein as SEQ ID NO: 1 and SEQ ID NO: 13 and the second fragment was amplified with a pair of primers defined herein as SEQ ID NO: 3 and SEQ ID NO: 14. For the construction of Y289G the first DNA fragment was amplified with a pair of primers defined herein as SEQ ID NO: 1 and SEQ ID NO: 15 and the second fragment was amplified with a pair of primers defined herein as SEQ ID NO: 3 and SEQ ID NO: 16 (be referred to Table 1 for the related sequences).

*GalT* mutants were expressed, isolated and refolded from inclusion bodies according to the reported procedure by Qasba et al. (Prot. Expr. Pur. 2003, 30, 219-229). After refolding, the precipitate was removed and the soluble and folded protein was isolated using a Ni-NTA column (HisTrap excel 1 mL column, GE Healthcare). After elution with 25 mM Tris-HCl pH 8.0, 300 mM NaCl and 200 mM imidazole, the protein was dialyzed against 25 mM Tris-HCl pH 8.0 and concentrated to 2 mg/mL using a spinfilter (Amicon Ultra-15 Centrifugal Filter Unit with Ultracel-10 membrane, Merck Millipore).

**Table 1 Sequence identification of the primers used**

| **SEQ ID NO** | **Nucleotide sequence** |
|---|---|
| SEQ ID NO: 1 | CAG CGA CAT ATG TCG CTG ACC GCA TGC CCT GAG GAG TCC |
| SEQ ID NO: 2 | GAC ACC TCC AAA **GTT** CTG CAC GTA AGG TAG GCT AAA |
| SEQ ID NO: 3 | CTG ATG GAT GGA TCC CTA GCT CGG CGT CCC GAT GTC CAC |
| SEQ ID NO: 4 | CCT TAC GTG CAG **AAC** TTT GGA GGT GTC TCT GCT CTA |
| SEQ ID NO: 5 | GAC ACC TCC AAA **AAA** CTG CAC GTA AGG TAG GCT AAA |
| SEQ ID NO: 6 | CCT TAC GTG CAG **TTT** TTT GGA GGT GTC TCT GCT CTA |
| SEQ ID NO: 7 | GAC ACC TCC AAA **CAT** CTG CAC GTA AGG TAG GCT AAA |
| SEQ ID NO: 8 | CCT TAC GTG CAG **ATG** TTT GGA GGT GTC TCT GCT CTA |
| SEQ ID NO: 9 | GAC ACC TCC AAA **AAT** CTG CAC GTA AGG TAG GCT AAA |
| SEQ ID NO: 10 | CCT TAC GTG CAG **ATT** TTT GGA GGT GTC TCT GCT CTA |
| SEQ ID NO: 11 | GAC ACC TCC AAA **CAC** CTG CAC GTA AGG TAG GCT AAA |
| SEQ ID NO: 12 | CCT TAC GTG CAG **GTG** TTT GGA GGT GTC TCT GCT CTA |
| SEQ ID NO: 13 | GAC ACC TCC AAA **CGC** CTG CAC GTA AGG TAG GCT AAA |
| SEQ ID NO: 14 | CCT TAC GTG CAG **GCG** TTT GGA GGT GTC TCT GCT CTA |
| SEQ ID NO: 15 | GAC ACC TCC AAA **GCC** CTG CAC GTA AGG TAG GCT AAA |
| SEQ ID NO: 16 | CCT TAC GTG CAG **GGC** TTT GGA GGT GTC TCT GCT CTA |

## Claims

1. Process for the preparation of an antibody-conjugate, comprising the steps of:
(1) providing an IgG antibody comprising at least two N-linked glycosylation sites on the combination of a single heavy chain and single light chain; and
(2) trimming an oligosaccharide that is attached to a glycosylation site, by the action of an endoglycosidase, in order to obtain a proximal N-linked GlcNAc-residue at said glycosylation site; and
(3) optionally repeating step (2) in order to trim an oligosaccharide that is attached to a different glycosylation site; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(5) optionally:
(5a) repeating step (2), in order to trim an oligosaccharide that is attached to a different glycosylation site; and
(5b) repeating step (4); and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
(7) optionally:
(7a) repeating step (2) in order to trim an oligosaccharide that is attached to a different glycosylation site; and
(7b) repeating step (4); and
(7c) repeating step (6); and
wherein the proximal N-linked GlcNAc-residue in steps (2), (4) and (6) is optionally fucosylated; wherein the process comprises one of steps (3), (5) and (7).

2. Process according to claim 1, the process comprising the steps of:
(1) providing an IgG antibody comprising at least two N-linked glycosylation sites on the combination of a single heavy chain and single light chain; and
(2) trimming an oligosaccharide that is attached to a glycosylation site, by the action of an endoglycosidase, in order to obtain a proximal N-linked GlcNAc-residue at said glycosylation site; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
wherein the proximal N-linked GlcNAc-residue in steps (2), (4) and (6) is optionally fucosylated.

3. Process according to claim 1, the process comprising the steps of:
(1) providing an IgG antibody comprising at least two N-linked glycosylation sites on the combination of a single heavy chain and single light chain; and
(2) trimming an oligosaccharide that is attached to a glycosylation site, by the action of an endoglycosidase, in order to obtain a proximal N-linked GlcNAc-residue at said glycosylation site; and
(4) attaching a monosaccharide derivative Su(A)ₓ to said proximal N-linked GlcNAc-residue, in the presence of a galactosyltransferase or a galactosyltransferase comprising a mutant catalytic domain, wherein Su(A)ₓ is defined as a monosaccharide derivative comprising x functional groups A wherein x is 1, 2, 3 or 4 and wherein A is selected from the group consisting of an azido group, a keto group, an alkynyl group, a thiol group or a precursor thereof, a halogen, a sulfonyloxy group, a halogenated acetamido group, a mercaptoacetamido group and a sulfonylated hydroxyacetamido group, in order to obtain a proximal N-linked GlcNAc-Su(A)ₓ substituent at said N-glycosylation site; and
(6) reacting said proximal N-linked GlcNAc-Su(A)ₓ substituent with a linker-conjugate, wherein said linker-conjugate comprises a functional group B and a molecule of interest D, wherein said functional group B is a functional group that is capable of reacting with a functional group A of said GlcNAc-Su(A)ₓ substituent, and wherein Su(A)ₓ is defined as above, with the proviso that A is not a thiol group precursor; and
(7a) repeating step (2) in order to trim an oligosaccharide that is attached to a different glycosylation site; and
(7b) repeating step (4); and
(7c) repeating step (6); and
wherein the proximal N-linked GlcNAc-residue in steps (2), (4) and (6) is optionally fucosylated.

4. Process according to any one of the preceding claims, wherein the IgG antibody comprising at least two N-linked glycosylation sites on the combination of a single heavy chain and single light chain comprises at least one native N-linked glycosylation site, and/or wherein the IgG antibody comprising at least two N-linked glycosylation sites on the combination of a single heavy chain and single light chain comprises at least one mutant N-linked glycosylation site as compared to its wild type counterpart.

5. Process according to any one of the preceding claims, wherein the amino acid sequence of the IgG heavy chain is altered in order to remove the native N-glycosylation site present at (or around) position 297.

6. Process according to any one of the preceding claims, wherein the endoglycosidase is an endo-β-N-acetylglucosaminidase selected from the group consisting of Endo S, Endo S49, Endo F1, Endo F2, Endo F3, Endo H, Endo A and Endo M, and any combination thereof, and/or wherein the galactosyltransferase or the galactosyltransferase comprising a mutant catalytic domain is selected from the group consisting of bovine β-4-Gal-T1, human β-4-Gal-T1, human β-4-Gal-T2, human β-4-Gal-T4 and human β-3-Gal-T5.

7. Process according to any one of the preceding claims, wherein Su(A)ₓ is selected from the group consisting of GalNAz-UDP, 6-AzGalNAc-UDP, 6-GalNAcCl-UDP, 6-GalNAcSH-UDP, 6-GalNAcSAc-UDP, 2-GalNAcCl-UDP, 2-GalNAcSH-UDP, 2-GalNAcSAc-UDP, 6-ClGal-UDP, 2-ClGal-UDP, 2-HSGal-UDP and 6-HSGal-UDP.

8. Process according to any one of the preceding claims, wherein:
(a) when A is an azido group, the linker-conjugate comprises a (hetero)cycloalkynyl group or an alkynyl group, and one or more molecules of interest; or
(b) when A is a keto group, the linker-conjugate comprises a primary amino group, an aminooxy group or a hydrazinyl group, and one or more molecules of interest; or
(c) when A is an alkynyl group, the linker-conjugate comprises an azido group a nitrone or a nitrile oxide, and one or more molecules of interest.
(d) when A is a thiol group or a mercaptoacetamido group, the linker-conjugate comprises an N-maleimide group or a halogenated acetamido group or an alkene, and one or more molecules of interest; or
(e) when A is a halogen, a halogenated acetamido group, a sulfonyloxy group or a sulfonylated hydroxyacetamido group, the linker-conjugate comprises a thiol group, and one or more molecules of interest.

9. Antibody-conjugate obtainable by the process according to any one of claims 1 - 8, wherein an antibody conjugate is defined as an antibody that is conjugated to a molecule of interest D via a linker L.

10. Antibody-conjugate according to claim 9, wherein an IgG antibody, comprising at least two N-linked glycosylation sites on the combination of a single heavy chain and a single light chain, is conjugated to a molecule of interest D at each glycosylation site via a linker L.

11. Antibody-conjugate according to claim 9 or 10, wherein the antibody comprises two or more different types of a molecule of interest.

12. Antibody-conjugate according to any one of claims 9 - 11, wherein the molecule of interest is selected from the group consisting of a reporter molecule, an active substance, an enzyme, an amino acid, a protein, a peptide, a polypeptide, an oligonucleotide, a glycan, an azide or a (hetero)cycloalkynyl moiety.

13. Antibody-conjugate according to any one of claims 9 - 12, for use as a medicament.

14. Antibody-conjugate according to any one of claims 9 - 12, for use in the treatment of cancer.

15. Antibody-conjugate according to any one of claims 9 - 12, for use in the treatment of breast cancer, more preferably for use in the treatment of HER2-positive breast cancer.

## Patentansprüche

1. Verfahren zur Herstellung eines Antikörper-Konjugats, das die Schritte umfasst:
(1) Bereitstellen eines IgG-Antikörpers, der mindestens zwei N-gebundene Glykosylierungsstellen an der Kombination einer einzelnen schweren Kette und einer einzelnen leichten Kette umfasst; und
(2) Trimmen eines Oligosaccharids, das an eine Glykosylierungsstelle gebunden ist, durch die Wirkung einer Endoglykosidase, um einen proximalen N-gebundenen GlcNAc-Rest an der Glykosylierungsstelle zu erhalten; und
(3) optional Wiederholen von Schritt (2), um ein Oligosaccharid zu trimmen, das an eine andere Glykosylierungsstelle gebunden ist; und
(4) Anhaften eines Monosaccharidderivats Su(A)ₓ an den proximalen N-gebundenen GlcNAc-Rest in der Gegenwart einer Galactosyltransferase oder einer Galactosyltransferase, die eine mutierte katalytische Domäne umfasst, wobei Su(A)ₓ als ein Monosaccharidderivat definiert ist, das x funktionelle Gruppen A umfasst, wobei x 1, 2, 3 oder 4 ist und wobei A aus der Gruppe ausgewählt ist, die aus einer Azidogruppe, einer Ketogruppe, einer Alkinylgruppe, einer Thiolgruppe oder einem Vorläufer davon, einem Halogen, einer Sulfonyloxygruppe, einer halogenierten Acetamidogruppe, einer Mercaptoacetamidogruppe und einer sulfonylierten Hydroxyacetamidogruppe besteht, um einen proximalen N-gebundenen GlcNAc-Su(A)ₓ-Substituenten an der N-Glycosylierungsstelle zu erhalten; und
(5) optional:
(5a) Wiederholen von Schritt (2), um ein Oligosaccharid zu trimmen, das an eine andere Glykosylierungsstelle gebunden ist; und
(5b) Wiederholen von Schritt (4); und
(6) Umsetzen des proximalen N-gebundenen GlcNAc-Su(A)ₓ-Substituenten mit einem Linker-Konjugat, wobei das Linker-Konjugat eine funktionelle Gruppe B und ein Molekül von Interesse D umfasst, wobei die funktionelle Gruppe B eine funktionelle Gruppe ist, die in der Lage ist, mit einer funktionellen Gruppe A des GlcNAc-Su(A)ₓ-Substituenten zu reagieren, und wobei Su(A)ₓ wie oben definiert ist, mit der Maßgabe, dass A kein Thiolgruppen-Vorläufer ist; und
(7) optional:
(7a) Wiederholen von Schritt (2), um ein Oligosaccharid zu trimmen, das an eine andere Glykosylierungsstelle gebunden ist; und
(7b) Wiederholen von Schritt (4); und
(7c) Wiederholen von Schritt (6); und
wobei der proximale N-gebundene GlcNAc-Rest in den Schritten (2), (4) und (6) optional fucosyliert ist; wobei das Verfahren einen der Schritte (3), (5) und (7) umfasst.

2. Verfahren nach Anspruch 1, wobei das Verfahren die Schritte umfasst:
(1) Bereitstellen eines IgG-Antikörpers, der mindestens zwei N-gebundene Glykosylierungsstellen an der Kombination einer einzelnen schweren Kette und einer einzelnen leichten Kette umfasst; und
(2) Trimmen eines Oligosaccharids, das an eine Glykosylierungsstelle gebunden ist, durch die Wirkung einer Endoglykosidase, um einen proximalen N-gebundenen GlcNAc-Rest an der Glykosylierungsstelle zu erhalten; und
(4) Anhaften eines Monosaccharidderivats Su(A)ₓ an den proximalen N-gebundenen GlcNAc-Rest in der Gegenwart einer Galactosyltransferase oder einer Galactosyltransferase, die eine mutierte katalytische Domäne umfasst, wobei Su(A)ₓ als ein Monosaccharidderivat definiert ist, das x funktionelle Gruppen A umfasst, wobei x 1, 2, 3 oder 4 ist und wobei A aus der Gruppe ausgewählt ist, die aus einer Azidogruppe, einer Ketogruppe, einer Alkinylgruppe, einer Thiolgruppe oder einem Vorläufer davon, einem Halogen, einer Sulfonyloxygruppe, einer halogenierten Acetamidogruppe, einer Mercaptoacetamidogruppe und einer sulfonylierten Hydroxyacetamidogruppe besteht, um einen proximalen N-gebundenen GlcNAc-Su(A)ₓ-Substituenten an der N-Glycosylierungsstelle zu erhalten; und
(6) Umsetzen des proximalen N-gebundenen GlcNAc-Su(A)ₓ-Substituenten mit einem Linker-Konjugat, wobei das Linker-Konjugat eine funktionelle Gruppe B und ein Molekül von Interesse D umfasst, wobei die funktionelle Gruppe B eine funktionelle Gruppe ist, die in der Lage ist, mit einer funktionellen Gruppe A des GlcNAc-Su(A)ₓ-Substituenten zu reagieren, und wobei Su(A)ₓ wie oben definiert ist, mit der Maßgabe, dass A kein Thiolgruppen-Vorläufer ist; und
wobei der proximale N-gebundenen GlcNAc-Rest in den Schritten (2), (4) und (6) optional fucosyliert ist.

3. Verfahren nach Anspruch 1, wobei das Verfahren die Schritte umfasst:
(1) Bereitstellen eines IgG-Antikörpers, der mindestens zwei N-gebundene Glykosylierungsstellen an der Kombination einer einzelnen schweren Kette und einer einzelnen leichten Kette umfasst; und
(2) Trimmen eines Oligosaccharids, das an eine Glykosylierungsstelle gebunden ist, durch die Wirkung einer Endoglykosidase, um einen proximalen N-gebundenen GlcNAc-Rest an der Glykosylierungsstelle zu erhalten; und
(4) Anhaften eines Monosaccharidderivats Su(A)ₓ an den proximalen N-gebundenen GlcNAc-Rest in der Gegenwart einer Galactosyltransferase oder einer Galactosyltransferase, die eine mutierte katalytische Domäne umfasst, wobei Su(A)ₓ als ein Monosaccharidderivat definiert ist, das x funktionelle Gruppen A umfasst, wobei x 1, 2, 3 oder 4 ist und wobei A aus der Gruppe ausgewählt ist, die aus einer Azidogruppe, einer Ketogruppe, einer Alkinylgruppe, einer Thiolgruppe oder einem Vorläufer davon, einem Halogen, einer Sulfonyloxygruppe, einer halogenierten Acetamidogruppe, einer Mercaptoacetamidogruppe und einer sulfonylierten Hydroxyacetamidogruppe besteht, um einen proximalen N-gebundenen GlcNAc-Su(A)ₓ-Substituenten an der N-Glycosylierungsstelle zu erhalten; und
(6) Umsetzen des proximalen N-gebundenen GlcNAc-Su(A)ₓ-Substituenten mit einem Linker-Konjugat, wobei das Linker-Konjugat eine funktionelle Gruppe B und ein Molekül von Interesse D umfasst, wobei die funktionelle Gruppe B eine funktionelle Gruppe ist, die in der Lage ist, mit einer funktionellen Gruppe A des GlcNAc-Su(A)ₓ-Substituenten zu reagieren, und wobei Su(A)ₓ wie oben definiert ist, mit der Maßgabe, dass A kein Thiolgruppen-Vorläufer ist; und
(7a) Wiederholen von Schritt (2), um ein Oligosaccharid zu trimmen, das an eine andere Glykosylierungsstelle gebunden ist; und
(7b) Wiederholen von Schritt (4); und
(7c) Wiederholen von Schritt (6); und
wobei der proximale N-gebundene GlcNAc-Rest in den Schritten (2), (4) und (6) optional fucosyliert ist.

4. Verfahren nach einem der vorangehenden Ansprüche, wobei der IgG-Antikörper, der mindestens zwei N-gebundene Glykosylierungsstellen an der Kombination aus einer einzelnen schweren Kette und einer einzelnen leichten Kette umfasst, mindestens eine native N-gebundene Glykosylierungsstelle umfasst, und/oder wobei der IgG-Antikörper, der mindestens zwei N-gebundene Glykosylierungsstellen an der Kombination einer einzelnen schweren Kette und einer einzelnen leichten Kette umfasst, mindestens eine mutierte N-gebundene Glykosylierungsstelle im Vergleich zu seinem Wildtyp-Gegenstück umfasst.

5. Verfahren nach einem der vorhergehenden Ansprüche, wobei die Aminosäuresequenz der schweren IgG-Kette verändert wird, um die native N-Glykosylierungsstelle an (oder um) Position 297 zu entfernen.

6. Verfahren nach einem der vorhergehenden Ansprüche, wobei die Endoglycosidase eine endo-β-N-Acetylglucosaminidase ist, die aus der Gruppe ausgewählt ist, die aus Endo S, Endo S49, Endo F1, Endo F2, Endo F3, Endo H, Endo A und Endo M und einer Kombination davon besteht, und/oder wobei die Galactosyltransferase oder die Galactosyltransferase, die eine mutierte katalytische Domäne umfasst, ausgewählt ist aus der Gruppe bestehend aus bovinem β-4-Gal-T1, menschlichem β-4-Gal-T1, menschlichem β-4-Gal-T2, menschlichem β-4-Gal-T4 und menschlichem β-3-Gal-T5.

7. Verfahren nach einem der vorhergehenden Ansprüche, wobei Su(A)ₓ ausgewählt ist aus der Gruppe bestehend aus GalNAz-UDP, 6-AzGalNAc-UDP, 6-GalNAcCl-UDP, 6-GalNAcSH-UDP, 6-GalNAcSAc-UDP, 2-GalNAcCl-UDP, 2-GalNAcSH-UDP, 2-GalNAcSAc-UDP, 6-ClGal-UDP, 2-ClGal-UDP, 2-HSGal-UDP und 6-HSGal-UDP.

8. Verfahren nach einem der vorhergehenden Ansprüche, wobei:
(a) wenn A eine Azidogruppe ist, das Linker-Konjugat eine (Hetero)cycloalkinylgruppe oder eine Alkinylgruppe und ein oder mehrere Moleküle von Interesse umfasst; oder
(b) wenn A eine Ketogruppe ist, das Linker-Konjugat eine primäre Aminogruppe, eine Aminooxygruppe oder eine Hydrazinylgruppe und ein oder mehrere Moleküle von Interesse umfasst; oder
(c) wenn A eine Alkinylgruppe ist, das Linker-Konjugat eine Azidogruppe, ein Nitron oder ein Nitriloxid und ein oder mehrere Moleküle von Interesse umfasst.
(d) wenn A eine Thiolgruppe oder eine Mercaptoacetamidogruppe ist, das Linker-Konjugat eine N-Maleimidgruppe oder eine halogenierte Acetamidogruppe oder ein Alken und ein oder mehrere Moleküle von Interesse umfasst; oder
(e) wenn A ein Halogen, eine halogenierte Acetamidogruppe, eine Sulfonyloxygruppe oder eine sulfonylierte Hydroxyacetamidogruppe ist, das Linker-Konjugat eine Thiolgruppe und ein oder mehrere Moleküle von Interesse umfasst.

9. Antikörper-Konjugat, erhältlich durch das Verfahren nach einem der Ansprüche 1 bis 8, wobei ein Antikörper-Konjugat definiert ist als ein Antikörper, der über einen Linker L mit einem Molekül von Interesse D konjugiert ist.

10. Antikörper-Konjugat nach Anspruch 9, wobei ein IgG-Antikörper, der mindestens zwei N-gebundene Glykosylierungsstellen an der Kombination einer einzelnen schweren Kette und einer einzelnen leichten Kette umfasst, an jeder Glykosylierungsstelle über einen Linker L mit einem Molekül von Interesse D konjugiert ist.

11. Antikörper-Konjugat nach Anspruch 9 oder 10, wobei der Antikörper zwei oder mehr verschiedene Typen eines Moleküls von Interesse umfasst.

12. Antikörper-Konjugat nach einem der Ansprüche 9 - 11, wobei das Molekül von Interesse ausgewählt ist aus der Gruppe bestehend aus einem Reportermolekül, einem Wirkstoff, einem Enzym, einer Aminosäure, einem Protein, einem Peptid, einem Polypeptid, einem Oligonukleotid, einem Glycan, einem Azid oder einer (Hetero)cycloalkinyl-Einheit.

13. Antikörper-Konjugat nach einem der Ansprüche 9 - 12 zur Verwendung als ein Medikament.

14. Antikörper-Konjugat nach einem der Ansprüche 9 - 12 zur Verwendung bei der Behandlung von Krebs.

15. Antikörper-Konjugat nach einem der Ansprüche 9 - 12 zur Verwendung bei der Behandlung von Brustkrebs, bevorzugter zur Verwendung bei der Behandlung von HER2-positivem Brustkrebs.

## Revendications

1. - Procédé de préparation d'un conjugué d'anticorps, comprenant les étapes consistant à :
1) fournir un anticorps IgG comprenant au moins deux sites de glycosylation N-liés sur la combinaison d'une seule chaîne lourde et d'une seule chaîne légère ; et
2) couper un oligosaccharide qui est attaché à un site de glycosylation, par l'action d'une endoglycosidase, afin d'obtenir un résidu GlcNAc N-lié proximal audit site de glycosylation ; et
3) facultativement répéter l'étape (2) de façon à couper un oligosaccharide qui est attaché à un site de glycosylation différent ; et
4) attacher un dérivé de monosaccharide Su(A)ₓ audit résidu GlcNAc N-lié proximal, en présence d'une galactosyltransférase ou d'une galactosyltransférase comprenant un domaine catalytique mutant, où Su(A)ₓ est défini comme un dérivé de monosaccharide comprenant x groupes fonctionnels A où x est 1, 2, 3 ou 4 et où A est choisi dans le groupe constitué par un groupe azido, un groupe céto, un groupe alcynyle, un groupe thiol ou un précurseur de celui-ci, un halogène, un groupe sulfonyloxy, un groupe acétamido halogéné, un groupe mercaptoacétamido et un groupe hydroxyacétamido sulfoné, afin d'obtenir un substituant GlcNAc-Su(A)ₓ N-lié proximal audit site de N-glycosylation ; et
5) facultativement :
5a. répéter l'étape (2), afin de couper un oligosaccharide qui est attaché à un site de glycosylation différent ; et
5b. répéter l'étape (4) ; et
6) faire réagir ledit substituant GlcNAc-Su(A)ₓ N-lié proximal avec un conjugué de lieur, ledit conjugué de lieur comprenant un groupe fonctionnel B et une molécule d'intérêt D, ledit groupe fonctionnel B étant un groupe fonctionnel qui est capable de réagir avec un groupe fonctionnel A dudit substituant GlcNAc-Su(A)ₓ , et Su(A)ₓ étant défini comme ci-dessus, à la condition que A ne soit pas un précurseur de groupe thiol ; et
7) facultativement :
7a. répéter l'étape (2) afin de couper un oligosaccharide qui est attaché à un site de glycosylation différent ; et
7b. répéter l'étape (4) ; et
7c. répéter l'étape (6) ; et
dans lequel le résidu GlcNAc N-lié proximal dans les étapes (2), (4) et (6) est facultativement fucosylé ; dans lequel le procédé comprend l'une des étapes (3), (5) et (7).

2. - Procédé selon la revendication 1, le procédé comprenant les étapes consistant à :
1) fournir un anticorps IgG comprenant au moins deux sites de glycosylation N-liés sur la combinaison d'une seule chaîne lourde et d'une seule chaîne légère ; et
2) couper un oligosaccharide qui est attaché à un site de glycosylation, par l'action d'une endoglycosidase, afin d'obtenir un résidu GlcNAc N-lié proximal audit site de glycosylation ; et
4) attacher un dérivé de monosaccharide Su(A)ₓ audit résidu GlcNAc N-lié proximal, en présence d'une galactosyltransférase ou d'une galactosyltransférase comprenant un domaine catalytique mutant, où Su(A)ₓ est défini comme un dérivé de monosaccharide comprenant x groupes fonctionnels A où x est 1, 2, 3 ou 4 et où A est choisi dans le groupe constitué par un groupe azido, un groupe céto, un groupe alcynyle, un groupe thiol ou un précurseur de celui-ci, un halogène, un groupe sulfonyloxy, un groupe acétamido halogéné, un groupe mercaptoacétamido et un groupe hydroxyacétamido sulfoné, afin d'obtenir un substituant GlcNAc-Su(A)ₓ N-lié proximal audit site de N-glycosylation ; et
6) faire réagir ledit substituant GlcNAc-Su(A)ₓ N-lié proximal avec un conjugué de lieur, ledit conjugué de lieur comprenant un groupe fonctionnel B et une molécule d'intérêt D, ledit groupe fonctionnel B étant un groupe fonctionnel qui est capable de réagir avec un groupe fonctionnel A dudit substituant GlcNAc-Su(A)ₓ, et Su(A)ₓ étant défini comme ci-dessus, à la condition que A ne soit pas un précurseur de groupe thiol ; et
dans lequel le résidu GlcNAc N-lié proximal dans les étapes (2), (4) et (6) est facultativement fucosylé.

3. - Procédé selon la revendication 1, le procédé comprenant les étapes consistant à :
1) fournir un anticorps IgG comprenant au moins deux sites de glycosylation N-liés sur la combinaison d'une seule chaîne lourde et d'une seule chaîne légère ; et
2) couper un oligosaccharide qui est attaché à un site de glycosylation, par l'action d'une endoglycosidase, afin d'obtenir un résidu GlcNAc N-lié proximal audit site de glycosylation ; et
4) attacher un dérivé de monosaccharide Su(A)ₓ audit résidu GlcNAc N-lié proximal, en présence d'une galactosyltransférase ou d'une galactosyltransférase comprenant un domaine catalytique mutant, où Su(A)ₓ est défini comme un dérivé de monosaccharide comprenant x groupes fonctionnels A où x est 1, 2, 3 ou 4 et où A est choisi dans le groupe constitué par un groupe azido, un groupe céto, un groupe alcynyle, un groupe thiol ou un précurseur de celui-ci, un halogène, un groupe sulfonyloxy, un groupe acétamido halogéné, un groupe mercaptoacétamido et un groupe hydroxyacétamido sulfoné, afin d'obtenir un substituant GlcNAc-Su(A)ₓ N-lié proximal audit site de N-glycosylation ; et
6) faire réagir ledit substituant GlcNAc-Su(A)ₓ N-lié proximal avec un conjugué de lieur, ledit conjugué de lieur comprenant un groupe fonctionnel B et une molécule d'intérêt D, ledit groupe fonctionnel B étant un groupe fonctionnel qui est capable de réagir avec un groupe fonctionnel A dudit substituant GlcNAc-Su(A)x, et Su(A)ₓ étant défini comme ci-dessus, à la condition que A ne soit pas un précurseur de groupe thiol ; et
7a) répéter l'étape (2) afin de couper un oligosaccharide qui est attaché à un site de glycosylation différent ; et
7b) répéter l'étape (4) ; et
7c) répéter l'étape (6) ; et
dans lequel le résidu GlcNAc N-lié proximal dans les étapes (2), (4) et (6) est facultativement fucosylé.

4. - Procédé selon l'une quelconque des revendications précédentes, dans lequel l'anticorps IgG comprenant au moins deux sites de glycosylation N-liés sur la combinaison d'une seule chaîne lourde et d'une seule chaîne légère comprend au moins un site de glycosylation N-lié natif, et/ou dans lequel l'anticorps IgG comprenant au moins deux sites de glycosylation N-liés sur la combinaison d'une seule chaîne lourde et d'une seule chaîne légère comprend au moins un site de glycosylation N-lié mutant par comparaison avec son homologue de type sauvage.

5. - Procédé selon l'une quelconque des revendications précédentes, dans lequel la séquence d'acides aminés de la chaîne lourde d'IgG est modifiée afin de retirer le site de N-glycosylation natif présent en (ou autour de la) position 297.

6. - Procédé selon l'une quelconque des revendications précédentes, dans lequel l'endoglycosidase est une endo-β-N-acétylglucosaminidase choisie dans le groupe constitué par Endo S, Endo S49, Endo F1, Endo F2, Endo F3, Endo H, Endo A et Endo M, et toute combinaison de celles-ci, et/ou dans lequel la galactosyltransférase ou la galactosyltransférase comprenant un domaine catalytique mutant est choisie dans le groupe constitué par β-4-Gal-T1 bovine, β-4-Gal-T1 humaine, β-4-Gal-T2 humaine, β-4-Gal-T4 humaine et β-3-Gal-T5 humaine.

7. - Procédé selon l'une quelconque des revendications précédentes, dans lequel Su(A)ₓ est choisi dans le groupe constitué par GalNAz-UDP, 6-AzGalNAc-UDP, 6-GalNAcCl-UDP, 6-GalNAcSH-UDP, 6-GalNAcSAc-UDP, 2-GalNAcCl-UDP, 2-GalNAcSH-UDP, 2-GalNAcSAc-UDP, 6-ClGal-UDP, 2-ClGal-UDP, 2-HSGal-UDP et 6-HSGal-UDP.

8. - Procédé selon l'une quelconque des revendications précédentes, dans lequel :
a) lorsque A est un groupe azido, le conjugué de lieur comprend un groupe (hétéro)cycloalcynyle ou un groupe alcynyle, et une ou plusieurs molécules d'intérêt ; ou
b) lorsque A est un groupe céto, le conjugué de lieur comprend un groupe amino primaire, un groupe aminooxy ou un groupe hydrazinyle, et une ou plusieurs molécules d'intérêt ; ou
c) lorsque A est un groupe alcynyle, le conjugué de lieur comprend un groupe azido, une nitrone ou un oxyde de nitrile, et une ou plusieurs molécules d'intérêt.
d) lorsque A est un groupe thiol ou un groupe mercaptoacétamido, le conjugué de lieur comprend un groupe N-maléimide ou un groupe acétamido halogéné ou un alcène, et une ou plusieurs molécules d'intérêt ; ou
e) lorsque A est un halogène, un groupe acétamido halogéné, un groupe sulfonyloxy ou un groupe hydroxyacétamido sulfonylé, le conjugué de lieur comprend un groupe thiol, et une ou plusieurs molécules d'intérêt.

9. - Conjugué d'anticorps pouvant être obtenu par le procédé selon l'une quelconque des revendications 1 à 8, dans lequel un conjugué d'anticorps est défini comme un anticorps qui est conjugué à une molécule d'intérêt D par l'intermédiaire d'un lieur L.

10. - Conjugué d'anticorps selon la revendication 9, dans lequel un anticorps IgG, comprenant au moins deux sites de glycosylation N-liés sur la combinaison d'une seule chaîne lourde et d'une seule chaîne légère, est conjugué à une molécule d'intérêt D à chaque site de glycosylation par l'intermédiaire d'un lieur L.

11. - Conjugué d'anticorps selon l'une des revendications 9 ou 10, dans lequel l'anticorps comprend deux types différents ou plus d'une molécule d'intérêt.

12. - Conjugué d'anticorps selon l'une quelconque des revendications 9 à 11, dans lequel la molécule d'intérêt est choisie dans le groupe constitué par une molécule rapporteur, une substance active, une enzyme, un acide aminé, une protéine, un peptide, un polypeptide, un oligonucléotide, un glycane, un azide ou une fraction (hétéro)cycloalcynyle.

13. - Conjugué d'anticorps selon l'une quelconque des revendications 9 à 12, destiné à être utilisé comme médicament.

14. - Conjugué d'anticorps selon l'une quelconque des revendications 9 à 12, destiné à être utilisé dans le traitement du cancer.

15. - Conjugué d'anticorps selon l'une quelconque des revendications 9 à 12, pour une utilisation dans le traitement du cancer du sein, de façon davantage préférée pour une utilisation dans le traitement du cancer du sein HER2- positif.
